# EUROPEAN PATENT SPECIFICATION

(11) **EP 2 917 218 B1**
(45) Date of publication and mention of the grant of the patent: **04.01.2017**
(21) Application number: 13792833.9
(22) Date of filing: 07.11.2013
(51) Int. Cl.: C07D 471/08, C07D 471/18, C07D 498/08, C07D 498/18, C07D 498/22, A61K 31/395, C07K 5/00, A61P 35/00, C07K 7/06, C07K 7/56

(54) **MACROCYCLIC COMPOUNDS FOR INHIBITION OF INHIBITORS OF APOPTOSIS**
MAKROCYCLISCHE VERBINDUNGEN ZUR HEMMUNG VON APOPTOSEINHIBITOREN
COMPOSÉS MACROCYCLIQUES POUR L'INHIBITION D'INHIBITEURS DE L'APOPTOSE

(30) Priority: 09.11.2012 US 201261724579 P
(43) Date of publication of application: 16.09.2015
(73) Proprietor: Ensemble Therapeutics Corporation, Cambridge MA 02139 (US)
(72) Inventor: BORZILLERI, Robert M., Princeton, New Jersey 08543 (US); ZHANG, Yong, Princeton, New Jersey 08543 (US); MILLER, Michael M., Princeton, New Jersey 08543 (US); SEIGAL, Benjamin A., Cambridge, Massachusetts 02139 (US)
(74) Representative: Potter Clarkson LLP
(86) International application number: PCT/US2013/068834
(87) International publication number: WO 2014/074658

(56) References cited:
- WO-A1-2011/104266
- WO-A1-2013/071035
- WO-A2-2006/020060
- WO-A2-2006/091972
- WO-A2-2010/022249

## Description

### FIELD OF THE INVENTION

The invention relates generally to macrocyclic compounds that modulate the activity of inhibitors of apoptosis (IAPs), pharmaceutical compositions containing said compounds and compounds for use in methods of treating proliferative disorders and disorders of dysregulated apoptosis, such as cancer, utilizing the compounds of the invention.

### BACKGROUND OF THE INVENTION

Apoptosis or programmed cell death is a genetically and biochemically regulated mechanism that plays an important role in development and homeostasis in invertebrates as well as vertebrates.

Aberrancies in apoptosis that lead to premature cell death have been linked to a variety of developmental disorders. Deficiencies in apoptosis that result in the lack of cell death have been linked to cancer and chronic viral infections.

Caspases are cysteine-containing aspartate specific proteases that play a key role in effecting apoptosis. Once activated from their inactive zymogen form by proteolytic processing, caspases digest vital cell proteins from within the cell. Since caspases are such strong proteases, tight control of this family of proteins is necessary to prevent premature cell death. In addition to proteolytic processing, caspases are also regulated by a family of molecules known as Inhibitors of Apoptosis Proteins (IAP). IAPs are naturally occurring intra-cellular proteins that suppress caspase-dependent apoptosis. SMAC, an intracellular protein also known as DIABLO, functions to modulate the activity of IAPs. In normal healthy cells, SMAC and IAPs function together to maintain healthy cells. However, in certain disease states, e.g., cancers and other proliferative disorders, the activities of IAPs are not adequately modulated and therefore, prevent apoptosis and cause or exacerbate abnormal proliferation and survival.

IAP antagonists, also known as SMAC mimetics, are synthetic molecules that mimic the structure and IAP modulating activity of the four N-terminal amino acids of SMAC (AVPI). When administered to a subject suffering proliferative disorders, the compounds antagonize IAP activities causing an increase in apoptosis among abnormally proliferating cells.

IAPs are found in all organisms ranging from Drosophila to human and are known to be overexpressed in many human cancers. IAPs comprise one to three Baculovirus IAP repeat (BIR) domains. The BIR domain is a zinc binding domain of about 70 residues comprising 4 alpha-helices and 3 beta strands, with cysteine and histidine residues that coordinate the zinc ion. The BIR 2 and 3 domains contain a conserved inhibitor of apoptosis binding motif (IBM) capable of binding caspases - and inhibiting their proteloytic activity.

As an example, human X-chromosome linked IAP (XIAP) inhibits the executioner caspases-3, and -7 as well as the Apaf-1-cytochrome C mediated activation of the initiator caspase-9. Caspases-3 and -7 are inhibited by the BIR2 domain of XIAP, while the BIR3 domain of XIAP is responsible for the inhibition of caspase-9 activation. XIAP is expressed ubiquitously in most adult and fetal tissues. Overexpression of XIAP in tumor cells has been demonstrated to confer protection of the tumor cells against a variety of pro-apoptotic stimuli and promotes resistance to chemotherapy. Consistent with this, a strong correlation between XIAP protein levels and survival has been demonstrated for patients with acute myelogenous leukemia.

Other BIR2-3 containing IAP family members, while capable of binding caspases, do not directly inhibit their proteloytic activity. Rather they inhibit apoptosis by affecting signaling activities of key proteins in cell survival pathways. Like XIAP, these IAPs possess a carboxyl-terminal RING finger domain capable of conjugating ubiquitin to specific protein substrates. As an example, cellular IAPs 1 and 2 (cIAP1/2), ubiquitinate RIPK, a signaling intermediate of tumor necrosis death receptor (TNF-DR) activation. Ubiquitinated RIPK is unable to activate caspase-8 in the context of DR activation by TNF family DR ligands. On the contrary, the long ubiquitin chains attached to RIPK provide a scaffold by which cell components of the NFkB cell survival signaling cascade can attach and become activated.

In normal cells undergoing apoptosis, the IAP-mediated inhibition is removed by the mitochondrial protein SMAC (second mitochondrial activator of caspases; also known as DIABLO). SMAC is synthesized as a precursor molecule of 239 amino acids; the N-terminal 55 residues serving as the mitochondria targeting sequence that is removed after import. The mature form of SMAC resides in the inter-membrane space of mitochondria. At the time of apoptosis induction, SMAC is released from mitochondria into the cytosol where, together with cytochrome c, it binds to XIAP, and eliminates its' inhibitory effect on caspases. SMAC also binds cIAP1/2 and inhibits their ability to ubiquinate RIPK. SMAC interacts with essentially all IAPs that have been examined to date and thus appears to be a master regulator of apoptosis in mammals.

Down-regulation of XIAP expression by antisense oligonucleotides has been shown to sensitize tumor cells to death induced by a wide range of pro-apoptotic agents, both in vitro and in vivo. SMAC/DIABLO-derived peptides have also been demonstrated to sensitize a number of different tumor induced select cell lines to undergo apoptosis as single agents, while other cell lines require an additional stimulus such as DR agonists or co-treatment with pro-apoptotic drugs. Because IAP inhibition appears to be a viable mechanism for promoting apoptosis and treating diseases and conditions that are sensitive to apoptosis, there is a continuing need to develop compounds that can inhibit IAP.

WO 2013/071035 was published on 16 May 2013 and refers to macrocyclic compounds for inhibition of inhibitors of apoptosis. WO 2010/022249 was published on 25 February 2010 and refers to macrocyclic compounds for inhibition of tumor necrosis factor alpha. WO 2011/104266 was published on 1 September 2011 and refers to dimeric IAP inhibitors. WO 2006/020060 was published on 23 February 2006 and refers to IAP binding compounds. WO 2006/091972 was published on 31 August 2006 and refers to dimeric IAP inhibitors.

### SUMMARY OF THE INVENTION

The present invention provides compounds, *ex vivo* methods of inducing apoptosis in a cell, and compounds for use in treating various medical conditions.

Also described herein are processes and intermediates for making the compounds of the present invention or stereoisomers, tautomers or pharmaceutically acceptable salts thereof.

The present invention also provides pharmaceutical compositions comprising a pharmaceutically acceptable carrier and one or more of the compounds of the present invention or stereoisomers, tautomers or pharmaceutically acceptable salts thereof.

The compounds of the invention may be used in the treatment and/or prophylaxis of multiple diseases or disorders associated with IAP inhibition, such as cancer and other maladies.

The compounds of the invention may be used in therapy.

The compounds of the invention may be used for the manufacture of a medicament for the treatment and/or prophylaxis of multiple diseases or disorders associated with IAP inhibition.

The compounds of the invention can be used alone, in combination with other compounds of the present invention, or in combination with one or more other agent(s).

Other features and advantages of the invention will be apparent from the following detailed description and claims.

### DETAILED DESCRIPTION OF THE INVENTION

### I. COMPOUNDS OF THE INVENTION

In a first aspect, the invention provides a compound of Formula (I): 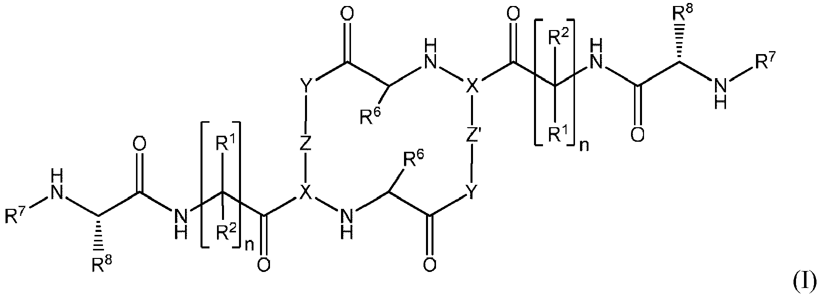 or a pharmaceutically acceptable salt thereof, wherein:
each n is independently 1 or 2;
each R¹ is independently hydrogen, optionally substituted C₁-C₄ alkyl, cycloalkyl, hydroxyalkyl, heterocyclyl or -(C₁-C₄ alkylene)-R⁴, wherein each R⁴ is independently hydrogen, -COOH, aryl, heteroaryl or cycloalkyl, and wherein at least one R¹ is other than hydrogen; and
each R² is hydrogen; or
R¹ and R² are taken together with the carbon atom to which they are commonly bound to form a cycloalkyl;
each R⁶ is independently -(C₁-C₄ alkylene)-R⁹, wherein each R⁹ is independently selected from hydrogen, aryl, heteroaryl and cycloalkyl; wherein any aryl, heteroaryl or cycloalkyl portion of R⁶ is optionally substituted with up to two substituents independently selected from halo, CF₃, OH, C₁-C₄ alkoxy, C₁-C₄ alkenyloxy, phenyl, phenyloxy, and phenylmethyloxy; and wherein one -CH₂- in the -(C₁-C₄ alkylene)- portion of R⁶ is optionally replaced with -O-;
each R⁷ is independently C₁-C₄ alkyl;
each R⁸ is independently C₁-C₄ alkyl;
each X is independently: 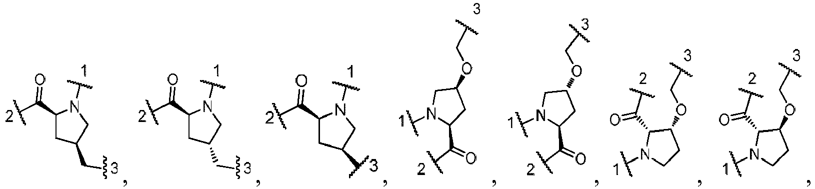 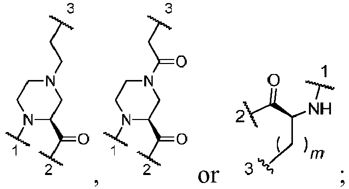
each of Z and Z' are independently: 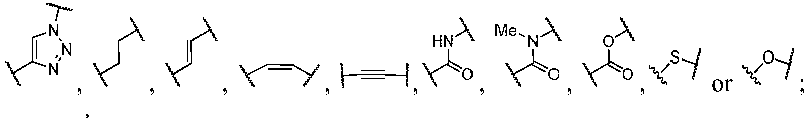
wherein each 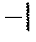 represents a point of attachment to the compound; however, Z and Z' cannot both be 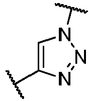 in any given compound;
each Y is independently: 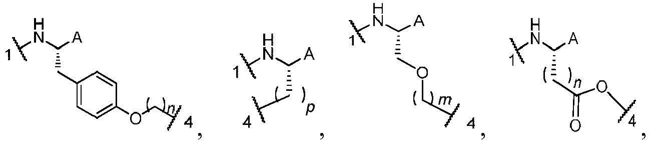 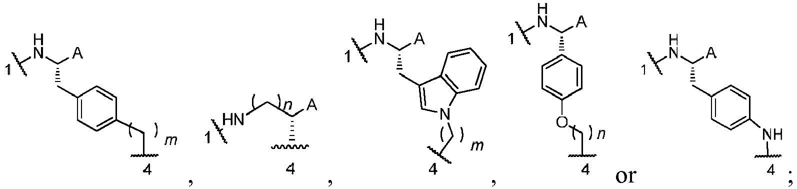 wherein: 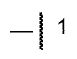 represents a point of attachment to a -C=O portion of the compound; 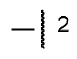 represents a point of attachment to a -NH portion of the compound; 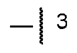 represents a first point of attachment to Z; 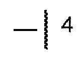 represents a second point of attachment to Z;
m = 0-3; n= 1-3, p = 0-4; and
A is -C(O)R³ or 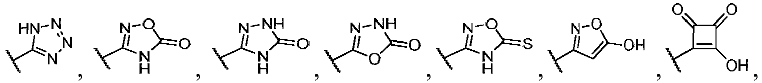 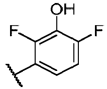 (including the various tautomeric forms);
R³ is OH, NHCN, NHSO₂R¹⁰, NHOR¹¹ or N(R¹²)(R¹³);
R¹⁰ and R¹¹ are hydrogen, optionally substituted: -C₁-C₄ alkyl, cycloalkyl, aryl, heteroaryl, heterocyclyl or heterocycloalkyl;
each of R¹² and R¹³ are independently selected from hydrogen, -C₁-C₄ alkyl, -(C₁-C₄ alkylene)-NH-(C₁-C₄ alkyl), and -(C₁-C₄ alkylene)-O-(C₁-C₄ hydroxyalkyl), or R¹² and R¹³ are taken together with the nitrogen atom to which they are commonly bound to form a saturated heterocyclyl optionally comprising one additional heteroatom selected from N, O and S, and wherein the saturated heterocycle is optionally substituted with methyl.

In a second aspect, the invention provides a compound of Formula (I) within the scope of the first aspect, wherein
each R⁶ is independently -(C₁-C₄ alkylene)-R⁹, wherein each R⁹ is independently selected from hydrogen, aryl and heteroaryl;
each R⁷ is independently selected from hydrogen and methyl;
each R⁸ is independently selected from methyl and ethyl;
each X is independently 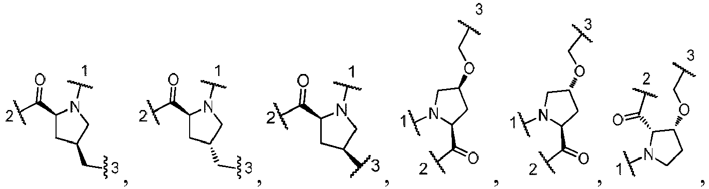 or 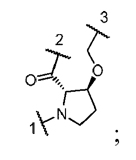 each Y is independently 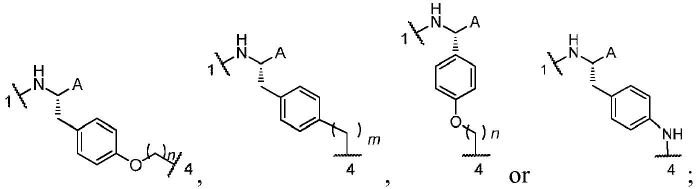 A is -C(O)R³; and
R³ is OH or NHSO₂R¹⁰.

In a third aspect, the invention provides a compound of Formula (I) within the scope of the first or second aspect, wherein:
each R¹ is independently t-butyl;
each R² is independently hydrogen;
each R⁶ is independently naphthalenylmethyl;
each R⁷ is independently methyl;
each R⁸ is independently methyl;
each X is independently 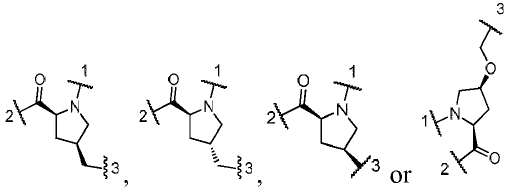
each Y is independently 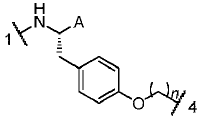
each of Z and Z' are independently 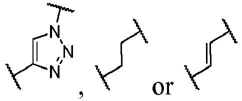 wherein each 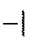 represents a point of attachment to the
compound; however, Z and Z' cannot both be 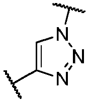 in any given compound;
A is -C(O)R³ or tetrazole;
R³ is OH, or NHSO₂R¹⁰, where R¹⁰ is C₁-C₄ alkyl, preferably methyl, or cycloalkyl, preferably cyclopropyl.

In another aspect, the invention provides a compound selected from the exemplified examples within the scope of the first aspect, or a pharmaceutically acceptable salt, tautomer or stereoisomer thereof.

In another aspect, the invention provides a compound selected from any subset list of compounds within the scope of any of the above aspects.

In another embodiment, the compounds of the invention have BIR3 IC₅₀ values ≤ 0.10 as measured in the BIR3 FP Assay.

In another embodiment, the compounds of the invention have BIR3 IC₅₀ values ≤ 0.075 as measured in the BIR3 FP Assay.

In another embodiment, the compounds of the invention have BIR3 IC₅₀ values ≤ 0.050 as measured in the BIR3 FP Assay.

In another embodiment, the compounds of the invention have BIR3 IC₅₀ values ≤ 0.050 as measured in the BIR3 HTRF Assay.

In another embodiment, the compounds of the invention have BIR3 IC₅₀ values ≤ 0.010 as measured in the BIR3 HTRF Assay.

In another embodiment, the compounds of the invention have BIR3 IC₅₀ values ≤ 0.005 as measured in the BIR3 HTRF Assay.

In another embodiment, the compounds of the invention have BIR3 IC₅₀ values ≤ 0.010 as measured in the BIR3 HTRF Assay.

In another embodiment, the compounds of the invention have BIR2-3 IC₅₀ values ≤ 0.025.

In another embodiment, the compounds of the invention have BIR2-3 IC₅₀ values ≤ 0.010.

In another embodiment, the compounds of the invention have BIR2-3 IC₅₀ values ≤ 0.0050.

In another embodiment, the compounds of the invention have BIR2-3 IC₅₀ values ≤ 0.0010.

### II. OTHER EMBODIMENTS OF THE INVENTION

In another embodiment, the present invention provides a composition comprising one or more compounds of the present invention or a stereoisomer, a tautomer, a pharmaceutically acceptable salt, or a solvate thereof.

In another embodiment, the present invention provides a pharmaceutical composition comprising a pharmaceutically acceptable carrier and at least one of the compounds of the present invention or a stereoisomer, a tautomer, a pharmaceutically acceptable salt, or a solvate thereof.

In another embodiment, the present invention provides a pharmaceutical composition, comprising: a pharmaceutically acceptable carrier and a therapeutically effective amount of at least one of the compounds of the present invention or a stereoisomer, a tautomer, a pharmaceutically acceptable salt, or a solvate thereof.

In another embodiment, the present invention provides a process for making a compound of the present invention or a stereoisomer, a tautomer, a pharmaceutically acceptable salt, or a solvate thereof.

In another embodiment, the present invention provides an intermediate for making a compound of the present invention or a stereoisomer, a tautomer, a pharmaceutically acceptable salt, or a solvate thereof.

In another embodiment, the present invention provides compounds for use in a method for the treatment and/or prophylaxis of various types of cancer, comprising administering to a patient in need of such treatment and/or prophylaxis a therapeutically effective amount of one or more compounds of the present invention, alone, or, optionally, in combination with another compound of the present invention and/or at least one other type of therapeutic agent.

In another embodiment, the present invention provides a compound of the present invention for use in therapy.

In another embodiment, the present invention provides a combined preparation of a compound of the present invention and additional therapeutic agent(s) for simultaneous, separate or sequential use in therapy.

In another embodiment, the present invention provides a combined preparation of a compound of the present invention and additional therapeutic agent(s) for simultaneous, separate or sequential use in the treatment and/or prophylaxis of multiple diseases or disorders associated with the inhibition of apoptosis.

Also described herein, there is a method of treating a patient suffering from or susceptible to a medical condition that is sensitive to apoptosis. A number of medical conditions can be treated. The method comprises administering to the patient a therapeutically effective amount of a composition comprising a compound described herein. For example, the compounds described herein may be used to treat or prevent infections, proliferative diseases (e.g., cancer), and autoimmune diseases.

In another aspect, the invention provides an *ex vivo* method of inhibiting the activity of an IAP in a cell, thus promoting apoptosis. The method comprises exposing the cell to a compound described herein.

### III. THERAPEUTIC APPLICATIONS

The compounds and pharmaceutical compositions of the present invention are useful in treating or preventing any disease or conditions that are sensitive to apoptosis. These include infections (e.g. skin infections, GI infection, urinary tract infections, genito-urinary infections, systemic infections), proliferative diseases (e.g., cancer), and autoimmune diseases (e.g., rheumatoid arthritis, lupus). The compounds and pharmaceutical compositions may be administered to animals, preferably mammals (e.g., domesticated animals, cats, dogs, mice, rats), and more preferably humans. Any method of administration may be used to deliver the compound or pharmaceutical composition to the animal. In certain embodiments, the compound or pharmaceutical composition is administered orally. In other embodiments, the compound or pharmaceutical composition is administered parenterally.

In one embodiment, the compounds of this invention can be used for the treatment of any cancer type that fails to undergo apoptosis in a patient. This includes, but is not limited to: solid tumors, including but not limited to carcinomas; sarcomas including Kaposi's sarcoma; erythroblastoma; glioblastoma; meningioma; astrocytoma; melanoma; and myoblastoma. Treatment or prevention of non-solid tumor cancers, such as leukemia, is also contemplated by this invention.

Types of cancers that may be treated with the compounds of this invention include, but are not limited to, brain cancers, skin cancers, bladder cancers, ovarian cancers, breast cancers, gastric cancers, pancreatic cancers, prostate cancers, colon cancers, blood cancers, lung cancers and bone cancers. Examples of such cancer types include neuroblastoma, intestine carcinoma such as rectum carcinoma, colon carcinoma, familiar adenomatous polyposis carcinoma and hereditary non-polyposis colorectal cancer, esophageal carcinoma, labial carcinoma, larynx carcinoma, hypopharynx carcinoma, tong carcinoma, salivary gland carcinoma, gastric carcinoma, adenocarcinoma, medullary thyroid carcinoma, papillary thyroid carcinoma, renal carcinoma, kidney parenchymal carcinoma, ovarian carcinoma, cervix carcinoma, uterine corpus carcinoma, endometrium carcinoma, chorion carcinoma, pancreatic carcinoma, prostate carcinoma, testis carcinoma, breast carcinoma, urinary carcinoma, melanoma, brain tumors such as glioblastoma, astrocytoma, meningioma, medulloblastoma and peripheral neuroectodermal tumors, Hodgkin lymphoma, non-Hodgkin lymphoma, Burkitt lymphoma, acute lymphatic leukemia (ALL), chronic lymphatic leukemia (CLL), acute myeloid leukemia (AML), chronic myeloid leukemia (CML), adult T-cell leukemia lymphoma, diffuse large B-cell lymphoma (DLBCL), hepatocellular carcinoma, gall bladder carcinoma, bronchial carcinoma, small cell lung carcinoma, non-small cell lung carcinoma, multiple myeloma, basalioma, teratoma, retinoblastoma, choroid melanoma, seminoma, rhabdomyosarcoma, craniopharyngioma, osteosarcoma, chondrosarcoma, myosarcoma, liposarcoma, fibrosarcoma, Ewing sarcoma and plasmocytoma.

In addition to apoptosis defects found in tumors, defects in the ability to eliminate self- reactive cells of the immune system due to apoptosis resistance are considered to play a key role in the pathogenesis of autoimmune diseases. Autoimmune diseases are characterized in that the cells of the immune system produce antibodies against its own organs and molecules or directly attack tissues resulting in the destruction of the latter. A failure of those self-reactive cells to undergo apoptosis leads to the manifestation of the disease. Defects in apoptosis regulation have been identified in autoimmune diseases such as systemic lupus erythematosus or rheumatoid arthritis.

Also described herein, there is a method of treating an autoimmune disease by providing to a patient in need thereof a compound or composition of the present invention. Examples of such autoimmune diseases include, but are not limited to, collagen diseases such as rheumatoid arthritis, systemic lupus erythematosus. Sharp's syndrome, CREST syndrome (calcinosis, Raynaud's syndrome, esophageal dysmotility, telangiectasia), dermatomyositis, vasculitis (Morbus Wegener's) and Sjogren's syndrome, renal diseases such as Goodpasture's syndrome, rapidly-progressing glomerulonephritis and membrano-proliferative glomerulonephritis type II, endocrine diseases such as type-I diabetes, autoimmune polyendocrinopathy-candidiasis-ectodermal dystrophy (APECED), autoimmune parathyroidism, pernicious anemia, gonad insufficiency, idiopathic Morbus Addison's, hyperthyreosis, Hashimoto's thyroiditis and primary myxedema, skin diseases such as pemphigus vulgaris, bullous pemphigoid, herpes gestationis, epidermolysis bullosa and erythema multiforme major, liver diseases such as primary biliary cirrhosis, autoimmune cholangitis, autoimmune hepatitis type-1, autoimmune hepatitis type-2, primary sclerosing cholangitis, neuronal diseases such as multiple sclerosis, myasthenia gravis, myasthenic Lambert-Eaton syndrome, acquired neuromyotomy, Guillain-Barre syndrome (Muller-Fischer syndrome), stiff-man syndrome, cerebellar degeneration, ataxia, opsoclonus, sensoric neuropathy and achalasia, blood diseases such as autoimmune hemolytic anemia, idiopathic thrombocytopenic purpura (Morbus Werlhof), infectious diseases with associated autoimmune reactions such as AIDS, Malaria and Chagas disease.

Compounds of the invention are useful for sensitizing cells to apoptotic signals. Thus, in one embodiment, the compounds of the invention are co-administered with radiation therapy or a second therapeutic agent with cytostatic or antineoplastic activity. Suitable cytostatic chemotherapy compounds include, but are not limited to (i) antimetabolites; (ii) DNA-fragmenting agents, (iii) DNA-crosslinking agents, (iv) intercalating agents (v) protein synthesis inhibitors, (vi) topoisomerase I poisons, such as camptothecin or topotecan; (vii) topoisomerase II poisons, (viii) microtubule-directed agents, (ix) kinase inhibitors (x) miscellaneous investigational agents (xi) hormones and (xii) hormone antagonists. It is contemplated that compounds of the invention may be useful in combination with any known agents falling into the above 12 classes as well as any future agents that are currently in development. In particular, it is contemplated that compounds of the invention may be useful in combination with current Standards of Care as well as any that evolve over the foreseeable future. Specific dosages and dosing regimens would be based on physicians' evolving knowledge and the general skill in the art.

The combination therapy is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single dosage form having a fixed ratio of each therapeutic agent or in multiple, single dosage forms for each of the therapeutic agents. Sequential or substantially simultaneous administration of each therapeutic agent can be effected by any appropriate route including, but not limited to, oral routes, intravenous routes, intramuscular routes, and direct absorption through mucous membrane tissues. The therapeutic agents can be administered by the same route or by different routes. For example, a first therapeutic agent of the combination selected may be administered by intravenous injection while the other therapeutic agents of the combination may be administered orally. Alternatively, for example, all therapeutic agents may be administered orally or all therapeutic agents may be administered by intravenous injection. Combination therapy also can embrace the administration of the therapeutic agents as described above in further combination with other biologically active ingredients and non-drug therapies (*e.g*., surgery or radiation treatment.) Where the combination therapy further comprises a non-drug treatment, the non-drug treatment may be conducted at any suitable time so long as a beneficial effect from the co-action of the combination of the therapeutic agents and non-drug treatment is achieved. For example, in appropriate cases, the beneficial effect is still achieved when the non-drug treatment is temporally removed from the administration of the therapeutic agents, perhaps by days or even weeks.

### IV. PHARMACEUTICAL COMPOSITIONS AND DOSING

The invention also provides pharmaceutically acceptable compositions which comprise a therapeutically effective amount of one or more of the compounds of Formula I, formulated together with one or more pharmaceutically acceptable carriers (additives) and/or diluents, and optionally, one or more additional therapeutic agents described above. As described in detail below, the pharmaceutical compositions of the present invention may be specially formulated for administration in solid or liquid form, including those adapted for the following: (1) oral administration, for example, drenches (aqueous or nonaqueous solutions or suspensions), tablets, e.g., those targeted for buccal, sublingual, and systemic absorption, boluses, powders, granules, pastes for application to the tongue; (2) parenteral administration, for example, by subcutaneous, intramuscular, intravenous or epidural injection as, for example, a sterile solution or suspension, or sustained release formulation; (3) topical application, for example, as a cream, ointment, or a controlled release patch or spray applied to the skin; (4) intravaginally or intrarectally, for example, as a pessary, cream or foam; (5) sublingually; (6) ocularly; (7) transdermally; or (8) nasally.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable carrier" as used herein means a pharmaceutically acceptable material, composition or vehicle, such as a liquid or solid filler, diluent, excipient, manufacturing aid (e.g., lubricant, talc magnesium, calcium or zinc stearate, or steric acid), or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; and (22) other non-toxic compatible substances employed in pharmaceutical formulations.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Formulations of the present invention include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient which can be combined with a carrier material to produce a single dosage form will generally be that amount of the compound which produces a therapeutic effect. Generally, out of one hundred percent, this amount will range from about 0.1 percent to about ninety-nine percent of active ingredient, preferably from about 5 percent to about 70 percent, most preferably from about 10 percent to about 30 percent.

In certain embodiments, a formulation of the present invention comprises an excipient selected from the group consisting of cyclodextrins, celluloses, liposomes, micelle forming agents, e.g., bile acids, and polymeric carriers, e.g., polyesters and polyanhydrides; and a compound of the present invention. In certain embodiments, an aforementioned formulation renders orally bioavailable a compound of the present invention.

Methods of preparing these formulations or compositions include the step of bringing into association a compound of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary or paste.

In solid dosage forms of the invention for oral administration (capsules, tablets, pills, dragees, powders, granules, troches and the like), the active ingredient is mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds and surfactants, such as poloxamer and sodium lauryl sulfate; (7) wetting agents, such as, for example, cetyl alcohol, glycerol monostearate, and non-ionic surfactants; (8) absorbents, such as kaolin and bentonite clay; (9) lubricants, such as talc, calcium stearate, magnesium stearate, solid polyethylene glycols, sodium lauryl sulfate, zinc stearate, sodium stearate, stearic acid, and mixtures thereof; (10) coloring agents; and (11) controlled release agents such as crospovidone or ethyl cellulose. In the case of capsules, tablets and pills, the pharmaceutical compositions may also comprise buffering agents. Solid compositions of a similar type may also be employed as fillers in soft and hard shelled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface active or dispersing agent. Molded tablets may be made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be formulated for rapid release, e.g., freeze-dried. They may be sterilized by, for example, filtration through a bacteria retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions of the invention for rectal or vaginal administration may be presented as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active compound.

Formulations of the present invention which are suitable for vaginal administration also include pessaries, tampons, creams, gels, pastes, foams or spray formulations containing such carriers as are known in the art to be appropriate.

Dosage forms for the topical or transdermal administration of a compound of this invention include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required.

The ointments, pastes, creams and gels may contain, in addition to an active compound of this invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this invention, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of a compound of the present invention to the body. Such dosage forms can be made by dissolving or dispersing the compound in the proper medium. Absorption enhancers can also be used to increase the flux of the compound across the skin. The rate of such flux can be controlled by either providing a rate controlling membrane or dispersing the compound in a polymer matrix or gel.

Ophthalmic formulations, eye ointments, powders, solutions and the like, are also contemplated as being within the scope of this invention.

Pharmaceutical compositions of this invention suitable for parenteral administration comprise one or more compounds of the invention in combination with one or more pharmaceutically acceptable sterile isotonic aqueous or non-aqueous solutions, dispersions, suspensions or emulsions, or sterile powders which may be reconstituted into sterile injectable solutions or dispersions just prior to use, which may contain sugars, alcohols, antioxidants, buffers, bacteriostats, solutes which render the formulation isotonic with the blood of the intended recipient or suspending or thickening agents.

Examples of suitable aqueous and non-aqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms upon the subject compounds may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming microencapsuled matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissue.

When the compounds of the present invention are administered as pharmaceuticals, to humans and animals, they can be given per se or as a pharmaceutical composition containing, for example, 0.1 to 99% (more preferably, 10 to 30%) of active ingredient in combination with a pharmaceutically acceptable carrier.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of this invention may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular compound of the present invention employed, or the ester, salt or amide thereof, the route of administration, the time of administration, the rate of excretion or metabolism of the particular compound being employed, the rate and extent of absorption, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved.

In general, a suitable daily dose of a compound of the invention will be that amount of the compound which is the lowest dose effective to produce a therapeutic effect. Such an effective dose will generally depend upon the factors described above. Generally, oral, intravenous, intracerebroventricular and subcutaneous doses of the compounds of this invention for a patient will range from about 0.01 to about 50 mg per kilogram of body weight per day.

If desired, the effective daily dose of the active compound may be administered as two, three, four, five, six or more sub-doses administered separately at appropriate intervals throughout the day, optionally, in unit dosage forms. In certain aspects of the invention, dosing is one administration per day.

While it is possible for a compound of the present invention to be administered alone, it is preferable to administer the compound as a pharmaceutical formulation (composition).

### V. DEFINITIONS

Throughout the specification and the appended claims, a given chemical formula or name shall encompass all stereo and optical isomers and racemates thereofwhere such isomers exist. Unless otherwise indicated, all chiral (enantiomeric and diastereomeric) and racemic forms are within the scope of the invention. Many geometric isomers of C=C double bonds, C=N double bonds, ring systems, and the like can also be present in the compounds, and all such stable isomers are contemplated in the present invention. Cis- and trans- (or E- and Z-) geometric isomers of the compounds of the present invention are described and may be isolated as a mixture of isomers or as separated isomeric forms. The present compounds can be isolated in optically active or racemic forms. Optically active forms may be prepared by resolution of racemic forms or by synthesis from optically active starting materials. All processes used to prepare compounds of the present invention and intermediates made therein are considered to be part of the present invention. When enantiomeric or diastereomeric products are prepared, they may be separated by conventional methods, for example, by chromatography or fractional crystallization. Depending on the process conditions the end products of the present invention are obtained either in free (neutral) or salt form. Both the free form and the salts of these end products are within the scope of the invention. If so desired, one form of a compound may be converted into another form. A free base or acid may be converted into a salt; a salt may be converted into the free compound or another salt; a mixture of isomeric compounds of the present invention may be separated into the individual isomers. Compounds of the present invention, free form and salts thereof, may exist in multiple tautomeric forms, in which hydrogen atoms are transposed to other parts of the molecules and the chemical bonds between the atoms of the molecules are consequently rearranged. It should be understood that all tautomeric forms, insofar as they may exist, are included within the invention.

As used herein, the term "alkyl" or "alkylene" is intended to include both branched and straight-chain saturated aliphatic hydrocarbon groups having the specified number of carbon atoms. For example, "C₁-C₆ alkyl" denotes alkyl having 1 to 6 carbon atoms. Example alkyl groups include, but are not limited to, methyl (Me), ethyl (Et), propyl (*e.g.*, n-propyl and isopropyl), butyl (*e.g.*, n-butyl, isobutyl, *t*-butyl), and pentyl (*e.g*., n-pentyl, isopentyl, neopentyl).

The term "alkoxy" or "alkyloxy" refers to an -O-alkyl group. "C₁₋₆ alkoxy" (or alkyloxy), is intended to include C₁, C₂, C₃, C₄, C₅, and C₆ alkoxy groups. Example alkoxy groups include, but are not limited to, methoxy, ethoxy, propoxy (e.g., n-propoxy and isopropoxy), and *t*-butoxy. Similarly, "alkylthio" or "thioalkoxy" represents an alkyl group as defined above with the indicated number of carbon atoms attached through a sulphur bridge; for example methyl-S- and ethyl-S-.

The term"aryl", either alone or in combination with another radical, means a carbocyclic aromatic monocyclic group containing 6 carbon atoms which may be further fused to a second 5- or 6-membered carbocyclic group which may be aromatic, saturated or unsaturated. Aryl includes, but is not limited to, phenyl, indanyl, 1-naphthalenyl, 2-naphthalenyl and terahydro naphthalenyl. The fused aryls may be connected to another group either at a suitable position on the cycloalkyl ring or the aromatic ring. For example:

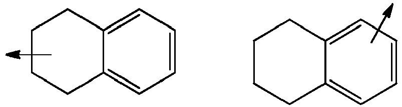

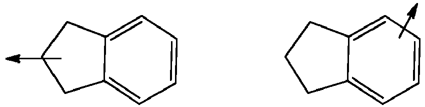

Arrowed lines drawn from the ring system indicate that the bond may be attached to any of the suitable ring atoms.

The term "cycloalkyl" refers to cyclized alkyl groups. C₃₋₆ cycloalkyl is intended to include C₃, C₄, C₅, and C₆ cycloalkyl groups. Example cycloalkyl groups include, but are not limited to, cyclopropyl, cyclobutyl, cyclopentyl, cyclohexyl, and norbornyl. Branched cycloalkyl groups such as 1-methylcyclopropyl and 2-methylcyclopropyl are included in the definition of "cycloalkyl". The term "cycloalkenyl" refers to cyclized alkenyl groups. C₄₋₆ cycloalkenyl is intended to include C₄, C₅, and C₆ cycloalkenyl groups. Example cycloalkenyl groups include, but are not limited to, cyclobutenyl, cyclopentenyl, and cyclohexenyl.

"Halo" or "halogen" includes fluoro, chloro, bromo, and iodo. "Haloalkyl" is intended to include both branched and straight-chain saturated aliphatic hydrocarbon groups having the specified number of carbon atoms, substituted with 1 or more halogens. Examples of haloalkyl include, but are not limited to, fluoromethyl, difluoromethyl, trifluoromethyl, trichloromethyl, pentafluoroethyl, pentachloroethyl, 2,2,2-trifluoroethyl, heptafluoropropyl, and heptachloropropyl. Examples of haloalkyl also include "fluoroalkyl" that is intended to include both branched and straight-chain saturated aliphatic hydrocarbon groups having the specified number of carbon atoms, substituted with 1 or more fluorine atoms.

"Haloalkoxy" or "haloalkyloxy" represents a haloalkyl group as defined above with the indicated number of carbon atoms attached through an oxygen bridge. For example, "C₁₋₆ haloalkoxy", is intended to include C₁, C₂, C₃, C₄, C₅, and C₆ haloalkoxy groups. Examples of haloalkoxy include, but are not limited to, trifluoromethoxy, 2,2,2-trifluoroethoxy, and pentafluorothoxy. Similarly, "haloalkylthio" or "thiohaloalkoxy" represents a haloalkyl group as defined above with the indicated number of carbon atoms attached through a sulphur bridge; for example trifluoromethyl-S-, and pentafluoroethyl-S-.

As used herein, the term "heteroaryl" or "aromatic heterocyclic group" is intended to mean stable monocyclic and polycyclic aromatic hydrocarbons that include at least one heteroatom ring member such as sulfur, oxygen, or nitrogen. Heteroaryl groups include, without limitation, pyridyl, pyrimidinyl, pyrazinyl, pyridazinyl, triazinyl, furyl, quinolyl, isoquinolyl, thienyl, imidazolyl, thiazolyl, indolyl, pyrroyl, oxazolyl, benzofuryl, benzothienyl, benzthiazolyl, isoxazolyl, pyrazolyl, triazolyl, tetrazolyl, indazolyl, 1,2,4-thiadiazolyl, isothiazolyl, purinyl, carbazolyl, benzimidazolyl, indolinyl, benzodioxolanyl, and benzodioxane. Heteroaryl groups are substituted or unsubstituted. The nitrogen atom is substituted or unsubstituted (*i.e*., N or NR wherein R is H or another substituent, if defined). The nitrogen and sulfur heteroatoms may optionally be oxidized (*i.e*., N→O and S(O)ₚ, wherein p is 0, 1 or 2).

As used herein, the term "heterocyclo", "heterocyclic" or "heterocyclyl" is intended to mean a 5,6 or 7 membered non-aromatic ring system containing from 1 to 4 heteroatoms selected from O, N or S. Examples of heterocycles include, but are not limited to, pyrrolidinyl, tetrahydrofuranyl, tetrahydropyranyl, piperidyl, pyrrolinyl, piperazinyl, imidazolinyl, morpholinyl, imidazolidinyl, pyrazolidinyl and pyrazolinyl.

The term "counter ion" is used to represent a negatively charged species such as chloride, bromide, hydroxide, acetate, and sulfate or a positively charged species such as sodium (Na+), potassium (K+), ammonium (RₙNHₘ+ where n=0-4 and m=0-4) and the like.

The term "electron withdrawing group" (EWG) refers to a substituent which polarizes a bond, drawing electron density towards itself and away from other bonded atoms. Examples of EWG include, but are not limited to, CF₃, CF₂CF₃, CN, halogen, haloalkyl, NO₂, sulfone, sulfoxide, ester, sulfonamide, carboxamide, alkoxy, alkoxyether, alkenyl, alkynyl, OH, C(O)alkyl, CO₂H, phenyl, heteroaryl, -O-phenyl, and -O-heteroaryl. Preferred examples of EWG include, but are not limited to, CF₃, CF₂CF₃, CN, halogen, SO₂(C₁₋₄ alkyl), CONH(C₁₋₄ alkyl), CON(C₁₋₄ alkyl)₂, and heteroaryl. More preferred examples of EWG include, but are not limited to, CF₃ and CN.

As used herein, the term "amine protecting group" means any group known in the art of organic synthesis for the protection of amine groups which is stable to an ester reducing agent, a disubstituted hydrazine, R4-M and R7-M, a nucleophile, a hydrazine reducing agent, an activator, a strong base, a hindered amine base and a cyclizing agent. Such amine protecting groups fitting these criteria include those listed in Wuts, P. G. M. and Greene, T.W. Projecting Groups in Organic Synthesis, 4th Edition, Wiley (2007) and The Peptides: Analysis, Synthesis, Biology, Vol. 3, Academic Press, New York (1981). Examples of amine protecting groups include, but are not limited to, the following: (1) acyl types such as formyl, trifluoroacetyl, phthalyl, and p-toluenesulfonyl; (2) aromatic carbamate types such as benzyloxycarbonyl (Cbz) and substituted benzyloxycarbonyls, 1-(p-biphenyl)-1-methylethoxycarbonyl, and 9-fluorenylmethyloxycarbonyl (Fmoc); (3) aliphatic carbamate types such as *tert*-butyloxycarbonyl (Boc), ethoxycarbonyl, diisopropylmethoxycarbonyl, and allyloxycarbonyl; (4) cyclic alkyl carbamate types such as cyclopentyloxycarbonyl and adamantyloxycarbonyl; (5) alkyl types such as triphenylmethyl and benzyl; (6) trialkylsilane such as trimethylsilane; (7) thiol containing types such as phenylthiocarbonyl and dithiasuccinoyl; and (8) alkyl types such as triphenylmethyl, methyl, and benzyl; and substituted alkyl types such as 2,2,2-trichloroethyl, 2-phenylethyl, and *t*-butyl; and trialkylsilane types such as trimethylsilane.

As referred to herein, the term "substituted" means that at least one hydrogen atom is replaced with a non-hydrogen group, provided that normal valencies are maintained and that the substitution results in a stable compound. Ring double bonds, as used herein, are double bonds that are formed between two adjacent ring atoms (*e.g*., C=C, C=N, or N=N).

In cases wherein there are nitrogen atoms (*e.g*., amines) on compounds of the present invention, these may be converted to N-oxides by treatment with an oxidizing agent (*e.g*., mCPBA and/or hydrogen peroxides) to afford other compounds of this invention. Thus, shown and claimed nitrogen atoms are considered to cover both the shown nitrogen and its N-oxide (N→O) derivative.

When any variable occurs more than one time in any constituent or formula for a compound, its definition at each occurrence is independent of its definition at every other occurrence. Thus, for example, if a group is shown to be substituted with 0-3 R, then said group may optionally be substituted with up to three R groups, and at each occurrence R is selected independently from the definition of R. Also, combinations of substituents and/or variables are permissible only if such combinations result in stable compounds.

When a bond to a substituent is shown to cross a bond connecting two atoms in a ring, then such substituent may be bonded to any atom on the ring. When a substituent is listed without indicating the atom in which such substituent is bonded to the rest of the compound of a given formula, then such substituent may be bonded via any atom in such substituent. Combinations of substituents and/or variables are permissible only if such combinations result in stable compounds.

The phrase "pharmaceutically acceptable" is employed herein to refer to those compounds, materials, compositions, and/or dosage forms that are, within the scope of sound medical judgment, suitable for use in contact with the tissues of human beings and animals without excessive toxicity, irritation, allergic response, and/or other problem or complication, commensurate with a reasonable benefit/risk ratio.

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic groups such as amines; and alkali or organic salts of acidic groups such as carboxylic acids. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, and nitric; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, and isethionic, and the like.

The pharmaceutically acceptable salts of the present invention can be synthesized from the parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington: The Science and Practice of Pharmacy, 22nd Edition, Allen, L. V. Jr., Ed.; Pharmaceutical Press, London, UK (2012).

It is also mentioned herein that compounds of formula I may have prodrug forms. Any compound that will be converted *in vivo* to provide the bioactive agent (*i.e*., a compound of formula I) is a prodrug mentioned herein. Various forms of prodrugs are well known in the art. For examples of such prodrug derivatives, see:
a) Bundgaard, H., ed., Design of Prodrugs, Elsevier (1985), and Widder, K. et al., eds., Methods in Enzymology, 112:309-396, Academic Press (1985);
b) Bundgaard, H., Chapter 5, "Design and Application of Prodrugs," A Textbook of Drug Design and Development, pp. 113-191, Krosgaard-Larsen, P. et al., eds., Harwood Academic Publishers (1991);
c) Bundgaard, H., Adv. Drug Deliv. Rev., 8:1-38 (1992);
d) Bundgaard, H. et al., J. Pharm. Sci., 77:285 (1988);
e) Kakeya, N. et al., Chem. Pharm. Bull., 32:692 (1984); and
f) Rautio, J (Editor). Prodrugs and Targeted Delivery (Methods and Principles in Medicinal Chemistry), Vol 47, Wiley-VCH, 2011.

Compounds containing a carboxy group can form physiologically hydrolyzable esters that serve as prodrugs by being hydrolyzed in the body to yield formula I compounds *per se.* Such prodrugs may be administered orally since hydrolysis in many instances occurs principally under the influence of the digestive enzymes. Parenteral administration may be used where the ester *per se* is active, or in those instances where hydrolysis occurs in the blood. Examples of physiologically hydrolyzable esters of compounds of formula I include C₁₋₆alkyl, C₁₋₆alkylbenzyl, 4-methoxybenzyl, indanyl, phthalyl, methoxymethyl, C₁₋₆ alkanoyloxy-C₁₋₆alkyl (*e.g.*, acetoxymethyl, pivaloyloxymethyl or propionyloxymethyl), C₁₋₆alkoxycarbonyloxy-C₁₋₆alkyl (*e.g*., methoxycarbonyl-oxymethyl or ethoxycarbonyloxymethyl, glycyloxymethyl, phenylglycyloxymethyl, (5-methyl-2-oxo-1,3-dioxolen-4-yl)-methyl), and other well known physiologically hydrolyzable esters used, for example, in the penicillin and cephalosporin arts. Such esters may be prepared by conventional techniques known in the art.

Preparation of prodrugs is well known in the art and described in, for example, Medicinal Chemistry: Principles and Practice, King, F.D., ed., The Royal Society of Chemistry, Cambridge, UK (1994); Testa, B. et al., Hydrolysis in Drug and Prodrug Metabolism. Chemistry, Biochemistry and Enzymology, VCHA and Wiley-VCH, Zurich, Switzerland (2003); The Practice of Medicinal Chemistry, Wermuth, C.G., ed., Academic Press, San Diego, CA (1999).

The present invention is intended to include all isotopes of atoms occurring in the present compounds. Isotopes include those atoms having the same atomic number but different mass numbers. By way of general example and without limitation, isotopes of hydrogen include deuterium and tritium. Isotopes of carbon include ¹³C and ¹⁴C. Isotopically-labeled compounds of the invention can generally be prepared by conventional techniques known to those skilled in the art or by processes analogous to those described herein, using an appropriate isotopically-labeled reagent in place of the non-labeled reagent otherwise employed.

The term "solvate" means a physical association of a compound of this invention with one or more solvent molecules, whether organic or inorganic. This physical association includes hydrogen bonding. In certain instances the solvate will be capable of isolation, for example when one or more solvent molecules are incorporated in the crystal lattice of the crystalline solid. The solvent molecules in the solvate may be present in a regular arrangement and/or a non-ordered arrangement. The solvate may comprise either a stoichiometric or nonstoichiometric amount of the solvent molecules. "Solvate" encompasses both solution-phase and isolable solvates. Exemplary solvates include, but are not limited to, hydrates, ethanolates, methanolates, and isopropanolates. Methods of solvation are generally known in the art.

As used herein, the term "patient" refers to organisms to be treated by the compounds of the present invention. Such organisms preferably include, but are not limited to, mammals (*e.g*., murines, simians, equines, bovines, porcines, canines, felines, and the like), and most preferably includes humans.

As used herein, the term "therapeutically effective amount" refers to the amount of a compound (*e.g*., a compound of the present invention) sufficient to effect beneficial or desired results. An effective amount can be administered in one or more administrations, applications or dosages and is not intended to be limited to a particular formulation or administration route. As used herein, the term "treating" includes any effect, *e.g*., lessening, reducing, modulating, ameliorating or eliminating, that results in the improvement of the condition, disease, disorder, and the like, or ameliorating a symptom thereof.

As used herein, the term "pharmaceutical composition" refers to the combination of an active agent with a carrier, inert or active, making the composition especially suitable for diagnostic or therapeutic use *in vivo* or *ex vivo.*

Examples of bases include, but are not limited to, alkali metals (*e.g*., sodium) hydroxides, alkaline earth metals (*e.g*., magnesium), hydroxides, ammonia, and compounds of formula NW₄⁺, wherein W is C₁₋₄ alkyl, and the like.

For therapeutic use, salts of the compounds of the present invention are contemplated as being pharmaceutically acceptable. However, salts of acids and bases that are non-pharmaceutically acceptable may also find use, for example, in the preparation or purification of a pharmaceutically acceptable compound.

### VI. METHODS OF PREPARATION

Macrocyclic compounds **9** and **10** can be prepared through the synthetic sequence depicted in Scheme **1**. In Scheme 1, Y¹ is a derivative of Y (as defined above), wherein the A group has been removed. Reaction of a chlorinated resin **2** with Fmoc-protected amino acid **1** in the presence of a base, such as Hunig's base, provides resin-linked compound **3**. Conversion to resin-linked peptide **4** can occur through standard Fmoc solid phase peptide synthesis protocol (e.g. sequential removal of the Fmoc protecting group under basic conditions such as piperidine, followed by amide formation in the presence of a coupling reagent, such as HATU). Azide-alkyne cycloaddition of the resin-linked peptide **4** with a Fmoc protected alkyne **5** then provides the resin-linked triazole compound **6**, which can be converted to the fully elaborated peptide **7** through Fmoc solid phase peptide synthesis protocol followed by cleavage from the resin. Conversion to the macrocyclic analogs **9** can be accomplished via ruthenium-mediated ring-closing metathesis reaction of **7**, followed by hydrolysis of the methyl ester and Boc deprotection of **8**. Futher elaboration to the saturated alkyl analogs **10** can be achieved by hydrogenation of **9** using, for example Pd on carbon as a catalyst.

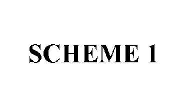

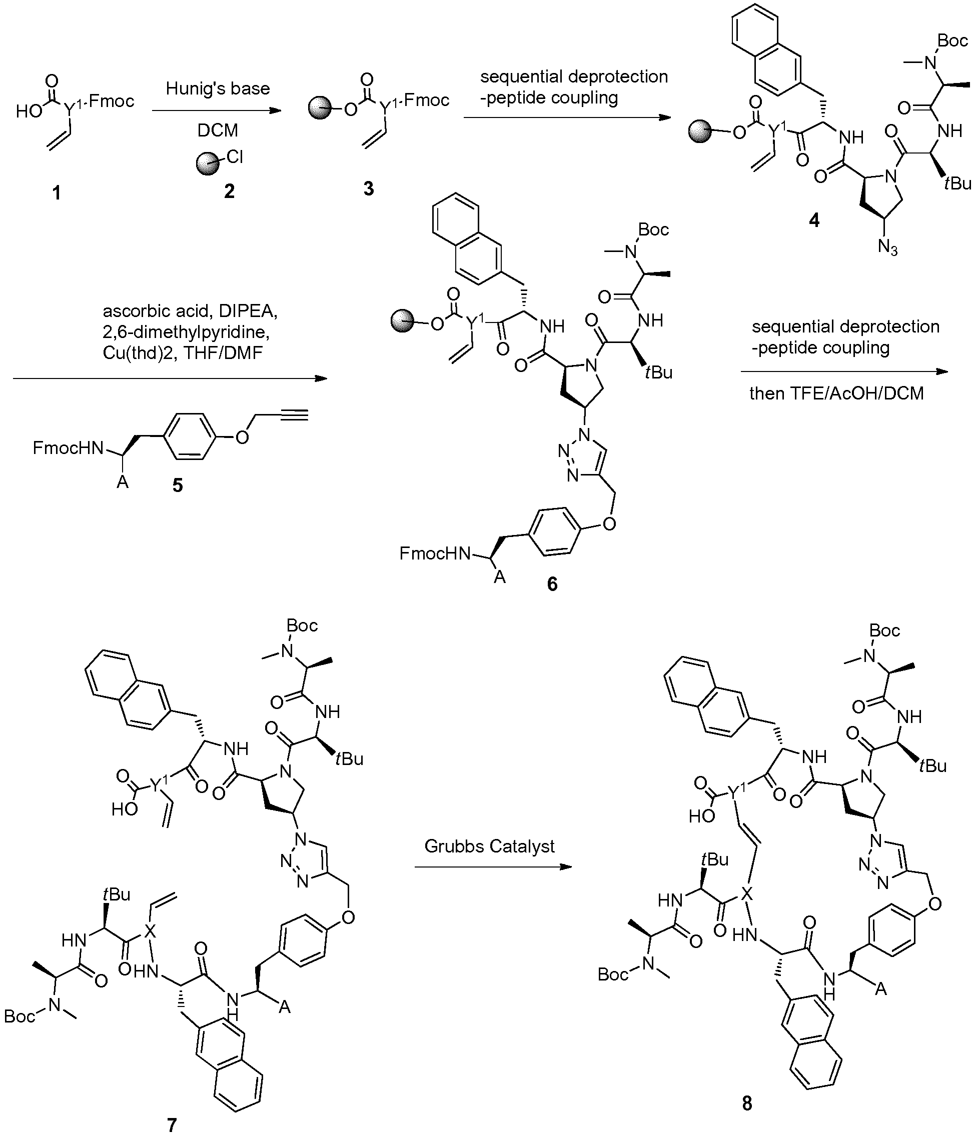

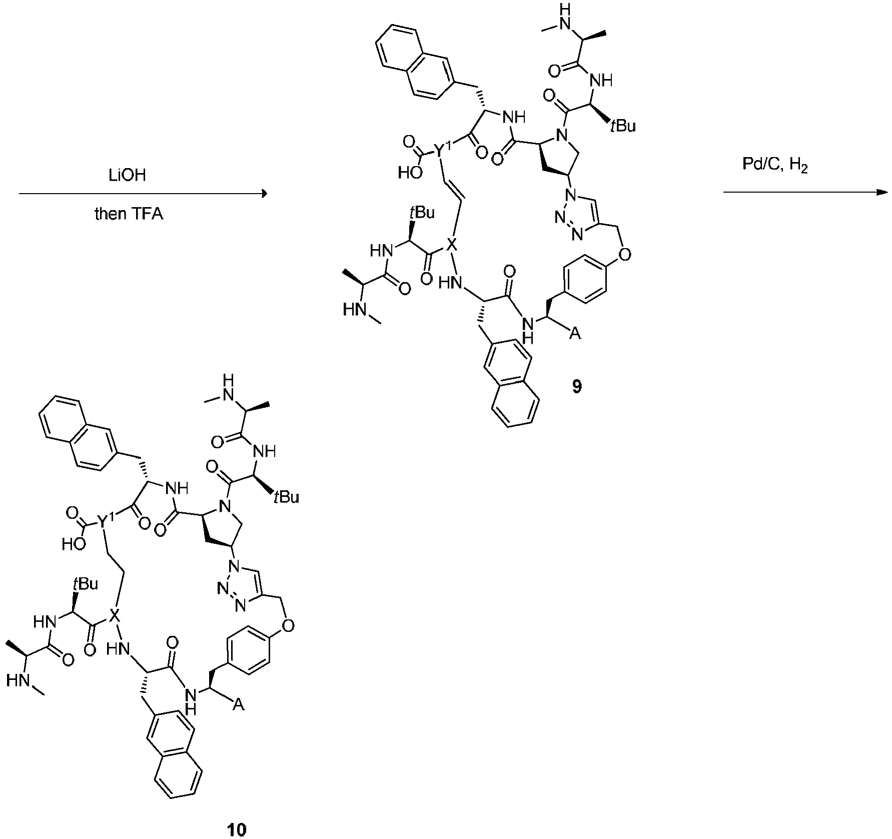

Analogs such as **14** can be prepared according to the synthetic route illustrated in Scheme 2. Treatment of macrocyclic compound **11**, which can be derived by the methods depicted in Scheme 1, with CDI, followed by an appropriately substituted sulfonamide **12** in the presence of a base, such as DBU, can provide acylsulfonamide derivative **13**. Hydrolysis of the methyl ester **13**, followed by removal of the Boc group under acidic condition (e.g. TFA) can then provide macrocylic compounds **14**.

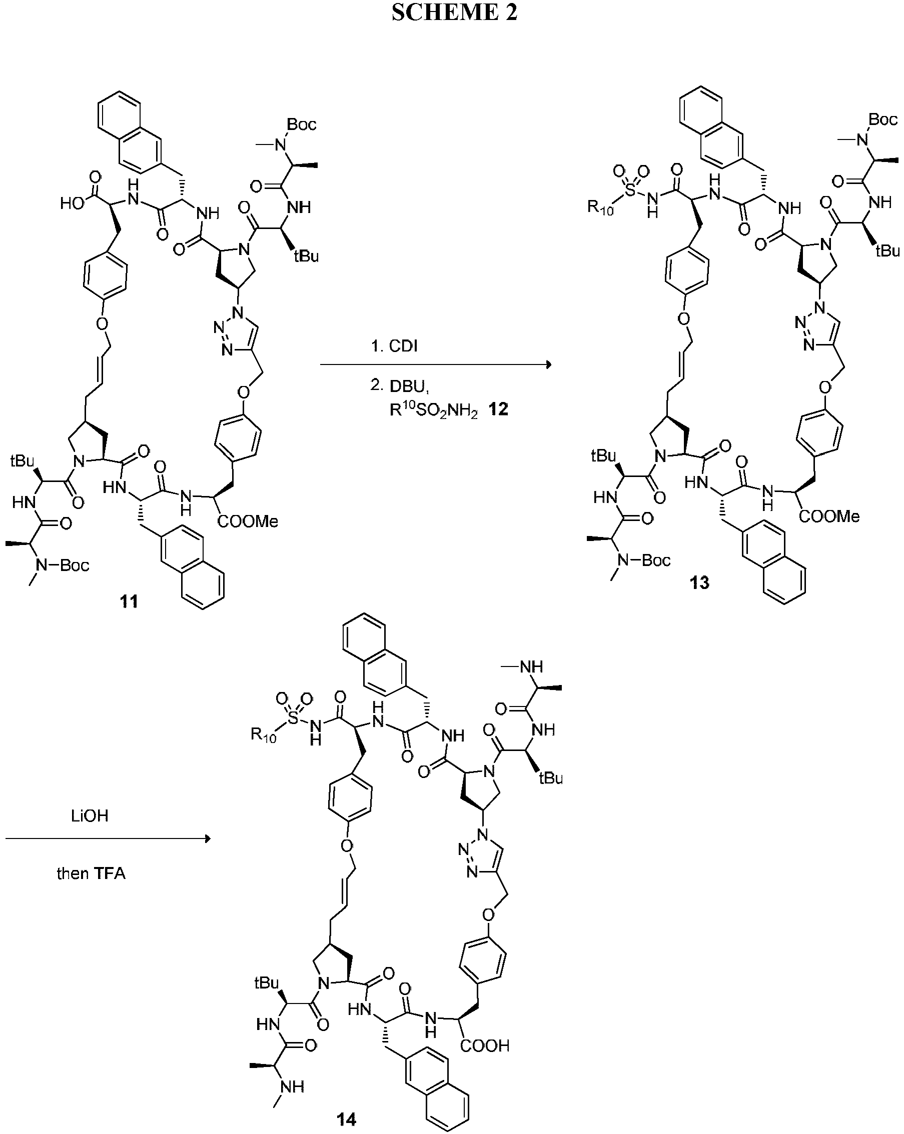

The pentapeptide intermediates **23** can be prepared as shown in Scheme 3. Commercially available (*S*)-2-naphthyl-alanine methylester **15** (Chem-Impex Int'l Inc.) and acid **16** can undergo amide formation in the presence of, for example, EDC to give dipeptide **17.** Following removal of the Boc carbamate of **17**, amide formation with commercially available acid **18** (Chem-Impex Int'l Inc.) can provide tripeptide **19**, which can be converted to the tetrapeptide intermediate **21** after Boc-deprotection and amide formation with commercially available acid **20** (Chem-Impex Int'l Inc.). Hydrolysis of the resulting methyl ester intermediate, followed by amide formation between compound **21** and primary amines **22** can provide pentapeptides **23.**

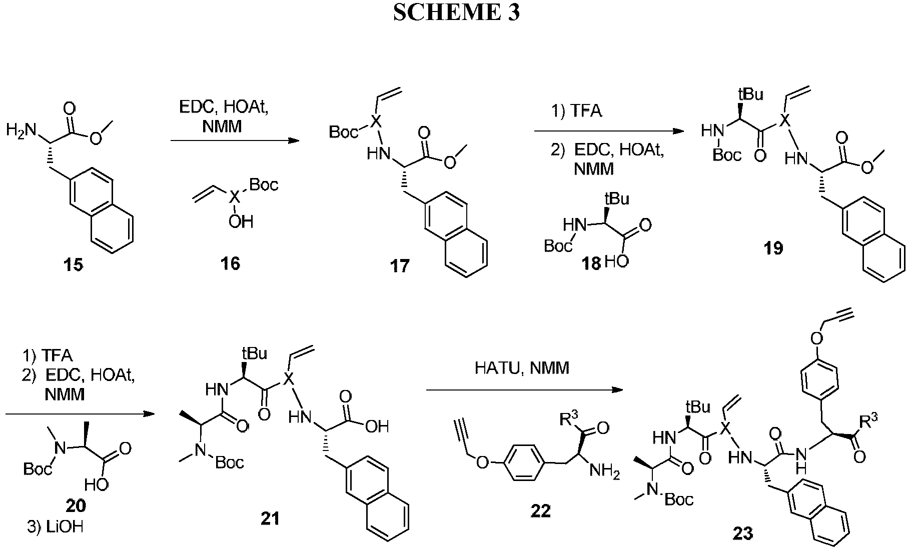

Analogs such as **31** can be prepared in a resin-free fashion according to the synthetic route illustrated in Scheme 4. Peptide coupling partner **29** can be prepared from commercially available (*S*)-2-naphthyl-alanine methylester **15** following chemistry analogous to that used to prepared peptides **23** as previously shown in Scheme 3. Azide-alkyne coupling of **29** with **23** in the presence of a copper catalyst then provides peptide intermediates **30**. Conversion of compounds **30** to the macrocyclic analogs **31** can be accomplished using a ruthenium catalyst, followed by global deprotection of the Boc carbamate and *tert*-butyl ester under acidic condition (e.g., TFA). Reduction of the double bond using Pd/C in the presence of hydrogen can then provide analogs **32**.

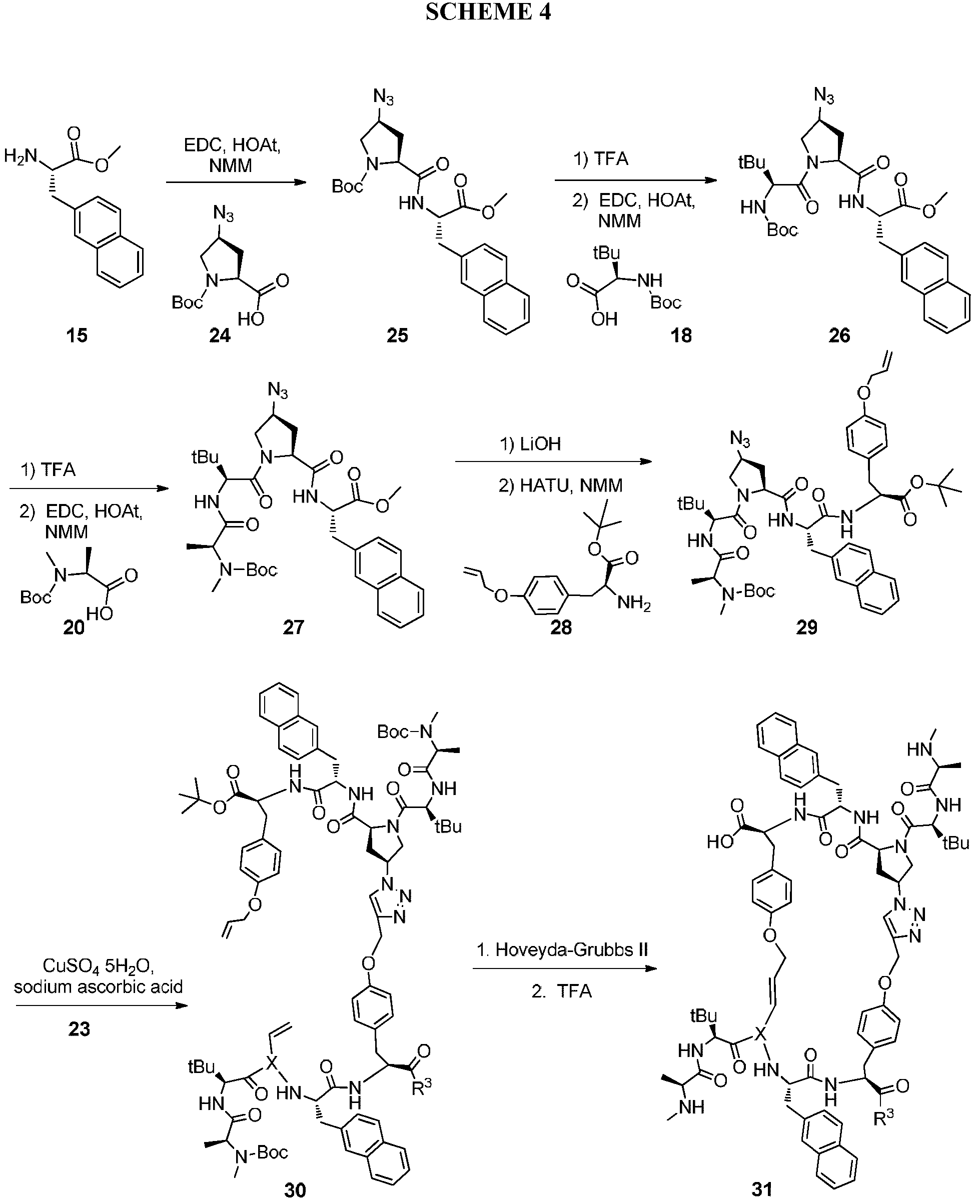

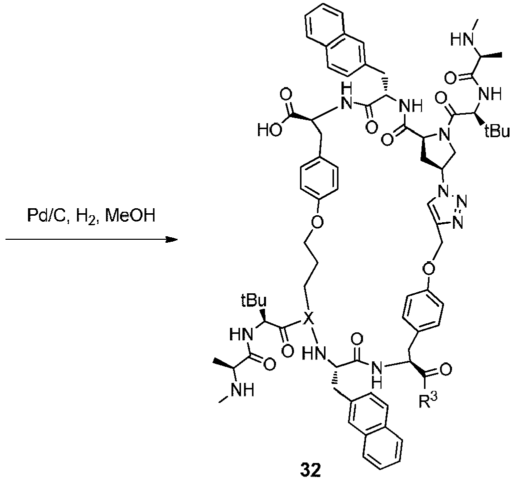

Analogs such as **37** can be prepared as shown in Scheme 5. In Scheme 5, Y¹ and Y² are both derivatives of Y (as defined above), wherein the A group has been removed. In Scheme **5**, resin-linked peptides **4** (prepared according to Scheme 1) can be converted to resin-linked intermediates **34** by reducing the azide and then reacting with Fmoc-protected acid **33** in the presence of HATU. Further elaboration using a standard Fmoc peptide synthesis protocol, followed by cleavage from the resin can then provide peptides **35.** Conversion to the macrocyclic analogs **36** can be accomplished using a ruthenium-based catalyst. Hydrolysis of the methyl ester of **36** and removal of the Boc carbamate then provides analogs **37**.

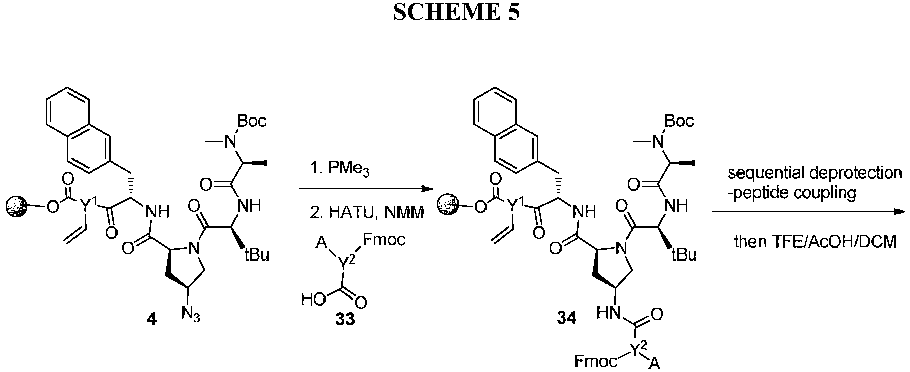

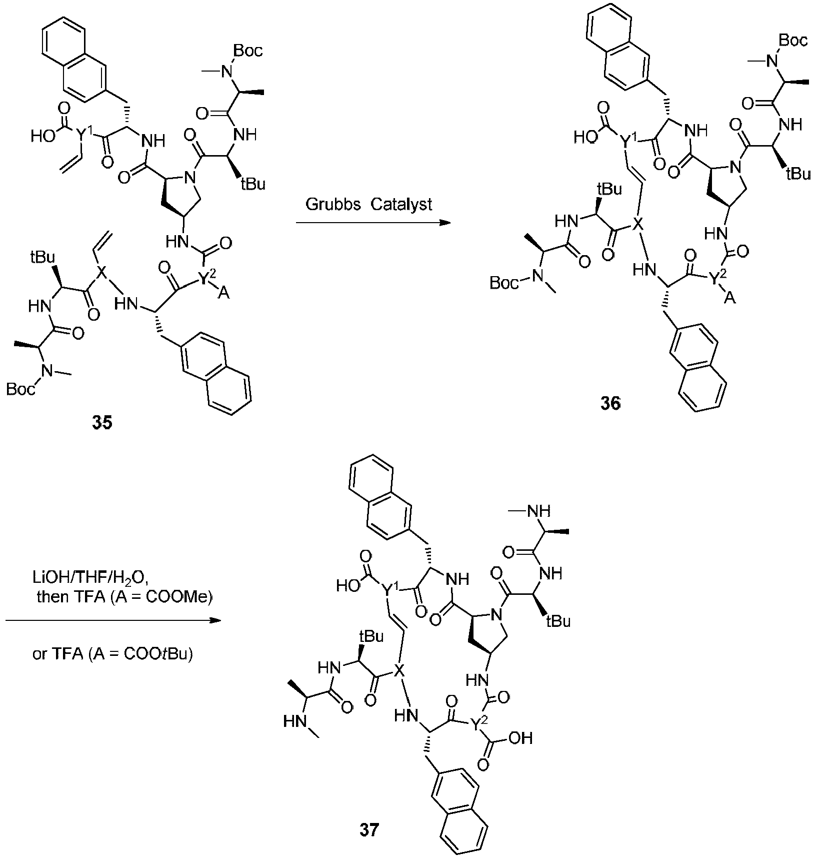

Related compounds **43** can be prepared as depicted in Scheme 6. In Scheme 6, Y¹ is a derivative of Y (as defined above), wherein the A group has been removed. In Scheme **6**, resin-linked peptides **40** can be prepared using chemistry analogous to that used for the synthesis of peptide **4** in Scheme 1. Peptide **40** and commercially available Fmoc tyrosine **41** (A ChemTek) can undergo Mitsunobu coupling in the presence DIAD and triphenylphosphine to give intermediate **42**. Elaboration to the final compounds **43** can be achieved following the sequences as described in Scheme 5.

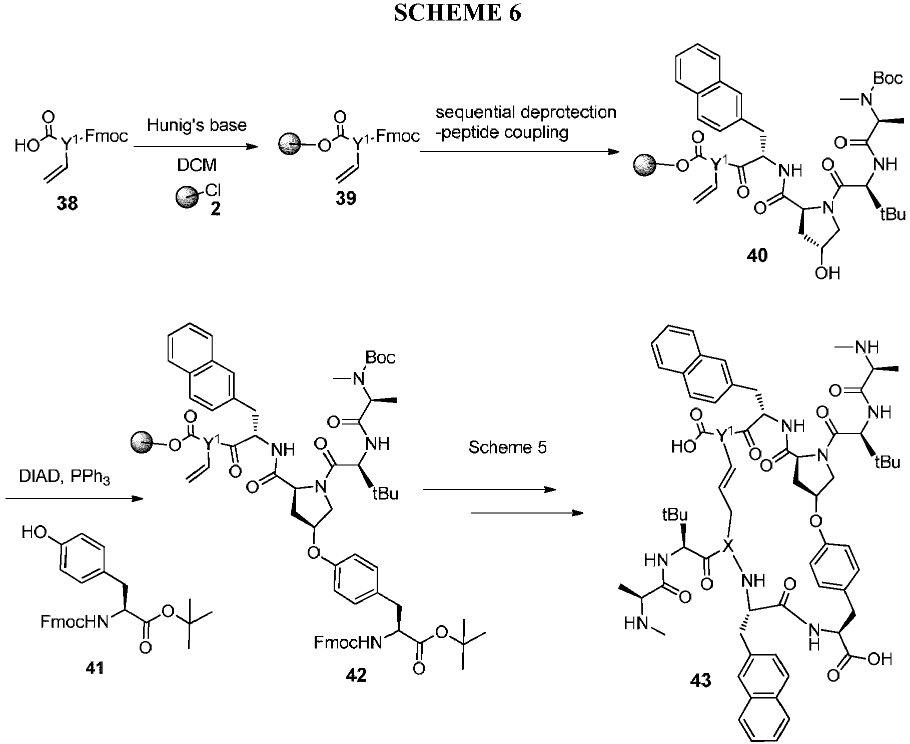

Analogs such as **52** can be accessed using the route describe in Scheme 7. In Scheme 7, Y¹ and Y² are both derivatives of Y (as defined above), wherein the A group has been removed. In Scheme **7**, resin-linked peptide **45** can be prepared using standard solid phase Fmoc peptide synthesis. Reduction of the azide with for example, trimethylphosphine can be followed by amide coupling of the resulting amine intermediate with acids **46** to give resin-linked intermediates **47**. Compounds **47** can then be converted into resin-linked compounds **48** through standard solid-phase Fmoc peptide synthesis. Further elaboration to intermediates **50** can be accomplished by reducing the azide and then coupling the requisite amine with acids **49**. After removal of the Fmoc group and cleavage from the resin, macrolactamization (mediated by a coupling reagent, such as HATU) can provide cyclic peptide intermediates **51**. Hydrolysis of **51** and subsequent *N-*Boc-deprotection with acid (e.g., TFA) can afford analogs **52**.

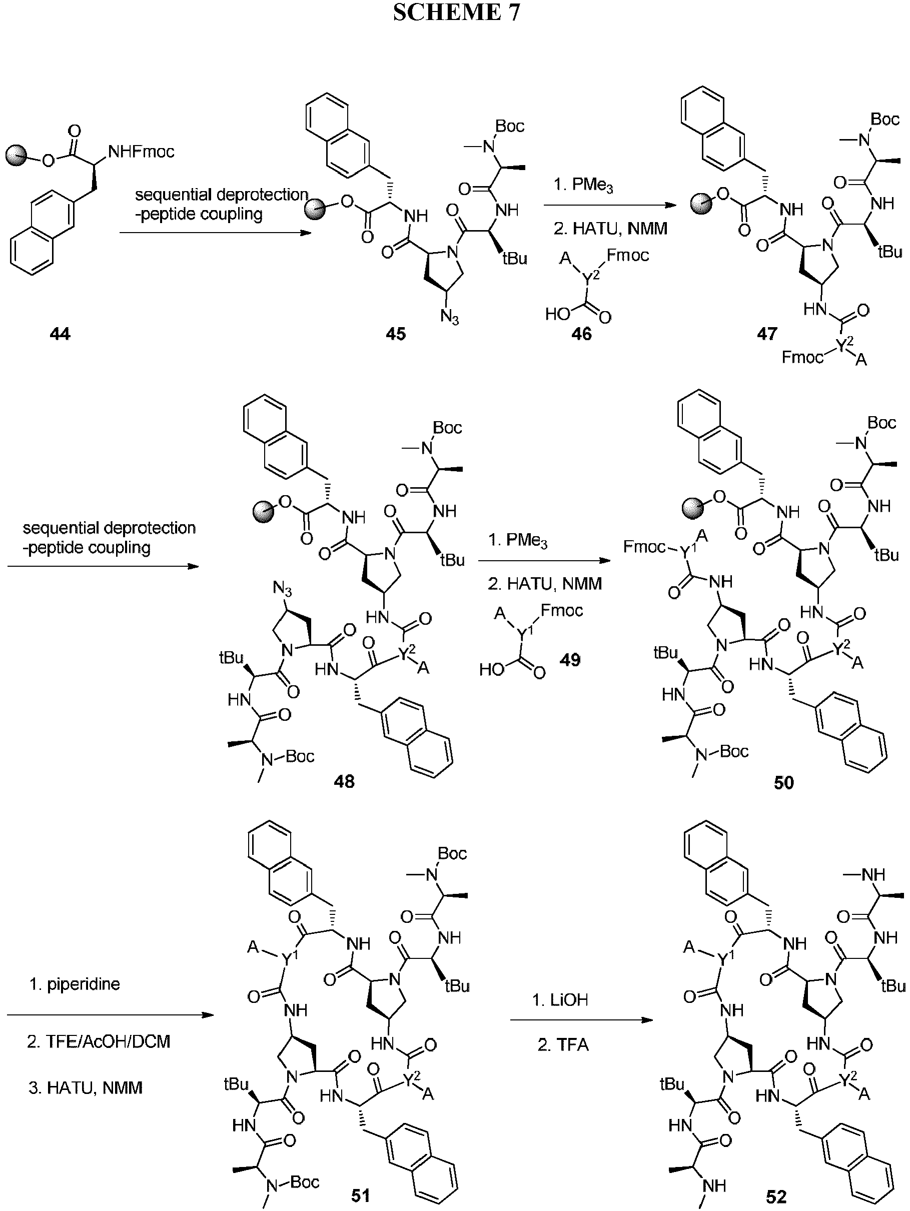

Analogs such as **58** can be prepared using the synthetic route illustrated in Scheme 8. Peptide **21**, which can be derived by methods depicted in Scheme 5, and compound **53** can undergo a cross-metathesis reaction in the presence of a ruthenium catalyst to give compound **54**. Amide bond formation between **54** and amine **55**, followed by removal of the Fmoc group under basic condition and amide coupling with peptide **21** can furnish intermediates **56**. Conversion of **56** to macrocycles **57** can then be accomplished by ring-closing metathesis using a ruthenium-based catalyst. Hydrolysis of the methyl ester **57** can then provide analogs **58** after *N-*Boc-deprotection. Reduction of the double bond of **58** using, for example Pd/C in the presence of hydrogen then provides analogs **59**.

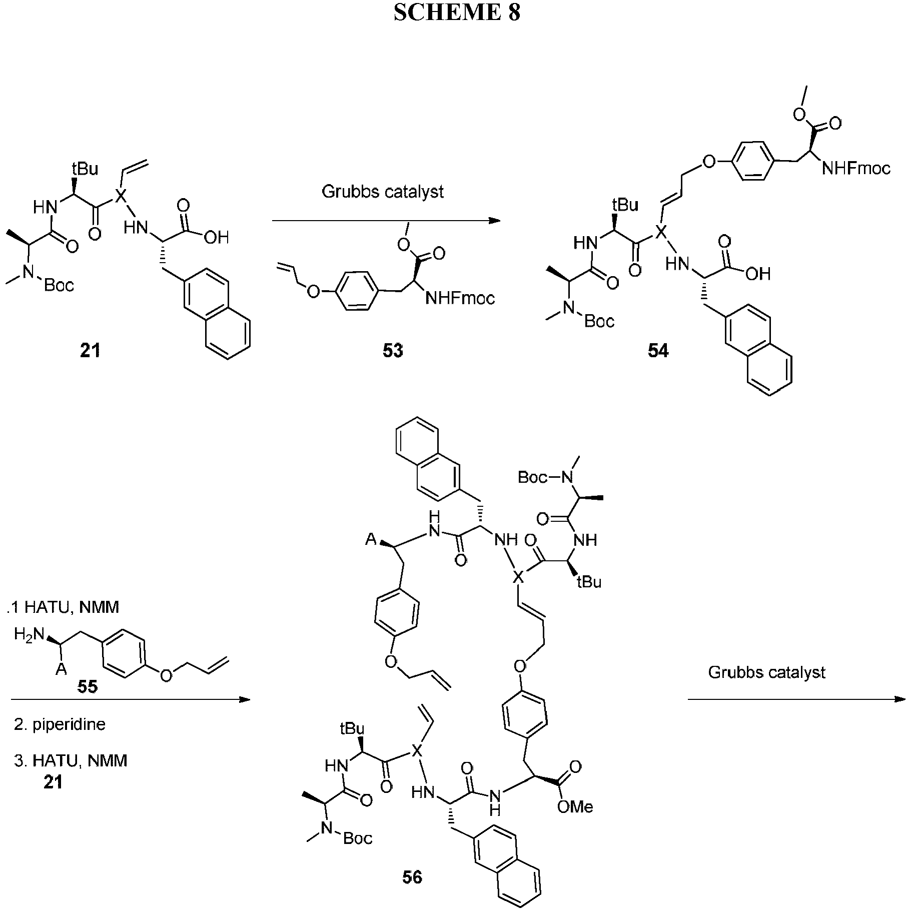

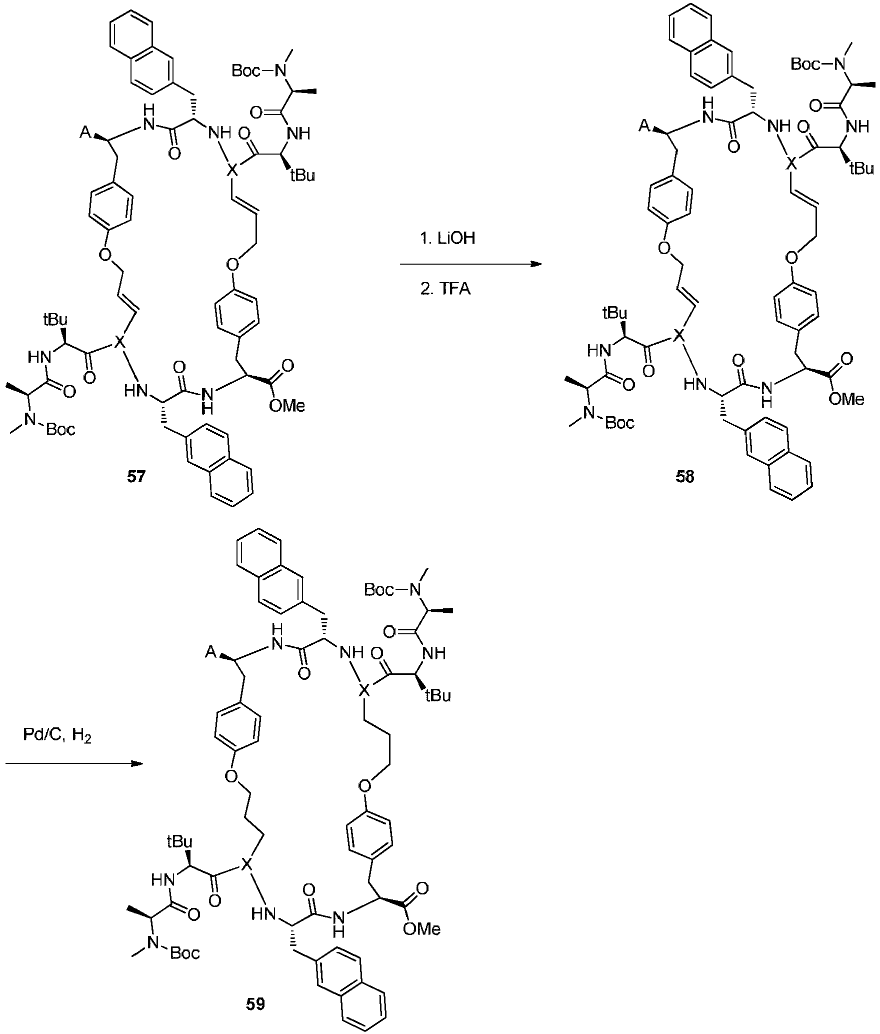

Analogs such as **65** and **66** can be prepared as outlined in Scheme 9. Peptides **60** and **61**, which can be derived by methods depicted in Scheme 5, can undergo a cycloaddition reaction in the presence of a copper catalyst to give triazole **62**. Amide bond formation with amines **63** can be followed by ring-closing metathesis using a ruthenium catalyst to provide macrocycles **65** after deprotection. Reduction of the double bond of compounds **65** as described above provides analogs **66**.

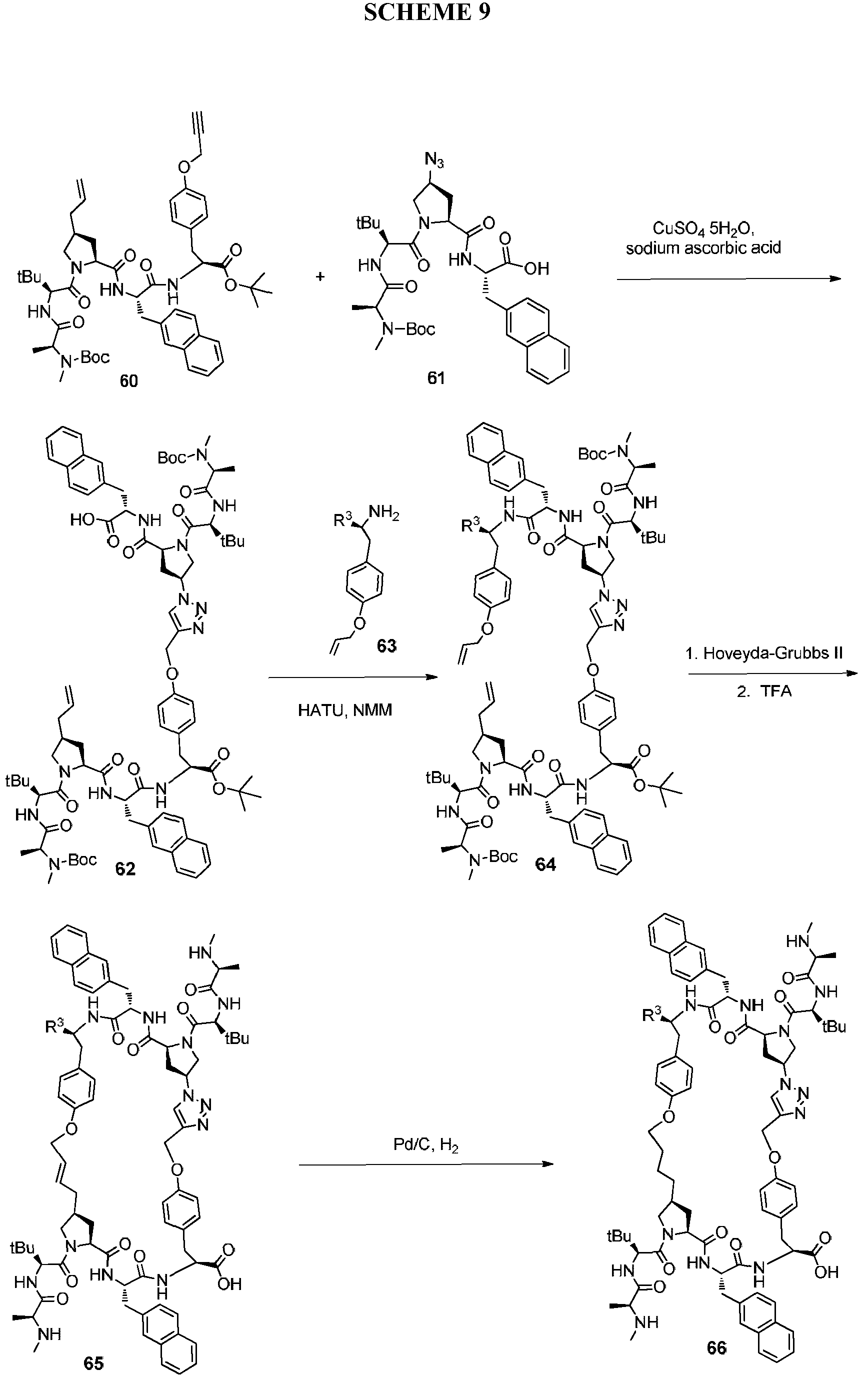

### EXAMPLES

### General Experimental

All reactions were carried out with continuous magnetic stirring under an atmosphere of dry nitrogen or argon. All evaporations and concentrations were carried out on a rotary evaporator under reduced pressure. Commercial reagents were used as received without additional purification. Solvents were commercial anhydrous grades and were used without further drying or purification. Flash chromatography was performed using prepacked RediSep^{®} R_{f} silica gel columns or prepacked RediSep^{®} R_{f} Gold C18 columns on a CombiFlash Rf machine.

Preparative Reverse Phase HPLC was performed with a linear gradient elution using H₂O/MeOH or H₂O/MeCN mixtures buffered with 0.1% trifluoroacetic acid or 10 mM NH₄OAc and detection at 220 nm on one of the following columns: Shimadzu Sunfire S10 30 × 250 mm (flow rate = 40 mL/min), or C18 Phenenomenex Luna S5 ODS 21 × 100 mm (flow rate = 20 mL/min), or YMC S5 ODS 20 × 100 mm (flow rate = 20 mL/min) or Waters XBridge C18 19 × 250 mm (flow rate = 20 mL/min). Preparative Supercritical Fluid Chromatography (SFC) was performed using 78% CO₂/MeOH buffered with 0.1% diethylamine and detection at 220 nm on a Chiralpak AS-H IDS 25 x 3 cm column (flow rate = 85 mL/min).

All final products were characterized by ¹H NMR, RP HPLC and electrospray ionization (ESI) or atmospheric pressure ionization (API) mass spectrometry (MS). ¹H NMR spectra were obtained a 500 MHz or a 400 MHz Bruker instrument. Field strengths are expressed in units of δ (parts per million, ppm) relative to the solvent peaks, and peak multiplicities are designated as follows: s, singlet; d, doublet; dd, doublet of doublets; t, triplet; q, quartet; sxt, sextet; br s, broad singlet; m, multiplet.

**ABBREVIATIONS**

| | |
|---|---|
| AcOH | acetic acid |
| aq. | aqueous |
| Bn | benzyl |
| Boc | *t*-butyl carbamate |
| Boc₂O | di-*t*-butyl dicarbonate |
| Burgess reagent | *N-*(triethylammoniumsulfonyl)carbamate |
| conc. | Concentrated |
| CDI | 1, 1'-carbonyldiimidazole |
| DBU | 1,8-Diazabicyclo[5.4.0]undec-7-ene |
| DCE | dichloroethane |
| DCM | dichloromethane |
| DIAD | diisopropyl azodicarboxylate |
| DIPEA | diisopropylethylamine |
| DMAP | 4-*N*,*N-*dimethylaminopyridine |
| DMF | dimethyl formamide |
| DMSO | dimethyl sulfoxide |
| EDC | 1-(dimethylaminopropyl)-3-ethylcarbodiimide hydrochloride |
| Et | ethyl |
| EtOAc | ethyl acetate |
| EtOH | ethanol |
| Et₂O | diethyl ether |
| Et₃N | triethyl amine |
| Fmoc-OSu | *N-*(9-Fluorenylmethoxycarbonyloxy) succinimide |
| h | hour(s) |
| HATU | O-(7-azabenzotriazol-1-yl)-*N*,*N*,*N'*,*N'*-tetramethyluronium hexafluorophosphate |
| HOAt | 1-hydroxy-7-azabenzotriazole |
| HPLC | high pressure liquid chromatography |
| *i*-PrOH | isopropanol |
| min | minute(s) |
| Me | methyl |
| MeCN | acetonitrile |
| MeOH | methanol |
| NMM | *N*-methylmorpholine |
| NMP | *N*-Methyl-2-pyrrolidone |
| NMR | nuclear magnetic resonance |
| Pd/C | palladium on carbon |
| Ph | phenyl |
| PhMe | toluene |
| PPh₃ | triphenyl phosphorus |
| sat. | saturated |
| *t*-Bu | tertiary butyl |
| *t*-BuOH | tertiary butanol |
| TFA | trifluoroacetic acid |
| THF | tetrahydrofuran |
| TMS | trimethylsilyl |

### EXAMPLE 1

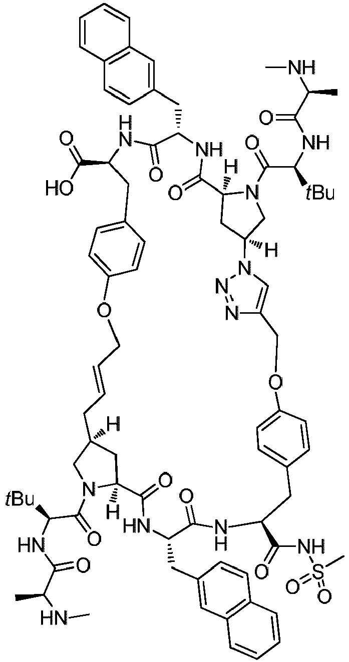

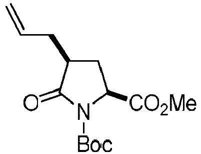

### A) (2S,4S)-1-tert-Butyl 2-methyl 4-allyl-5-oxopyrrolidine-1,2-dicarboxylate

To a solution of (*S*)*-*1*-tert*-butyl 2-methyl 5-oxopyrrolidine-1,2-dicarboxylate (Arkpharma, 8.0 g, 32.9 mmol) in THF (60 mL) at -78 °C was added slowly a solution of lithium bis(trimethylsilyl)amide (LHMDS, Aldrich, 32.9 mL, 32.9 mmol, 1 M in toluene). The reaction was stirred at -78 °C for 1 h before 3-iodoprop-1-ene (Aldrich, 8.29 g, 49.3 mmol) was added dropwise. After stirring at -78 °C for 3 h, the reaction was quenched with a solution of acetic acid (Aldrich, 4 mL) in THF (4 mL). The mixture was then poured into aq. NaHCO₃ soln. (150 mL) and extracted with EtOAc (3x). The combined organic extracts were dried over MgSO₄, concentrated *in vacuo,* and purified by flash column chromatography (ISCO, 0 - 50% EtOAc in hexane) to give the title compound as a cis:trans mixture with a ratio of 1:2.

To a -78 °C solution of the *cis*/*trans* mixture in THF (60 mL) was added dropwise a solution of LHMDS (1M in toluene, 41 mL, 41 mmol). The reaction was stirred at -78 °C for 1 h before a solution of 2,6-di-*tert*-butylphenol (Aldrich, 10.2 g, 49.3 mmol) in THF (10 mL) was added dropwise. The resulting mixture was stirred at -78 °C for 2 h at before it was quenched with aq. NH₄Cl soln. The mixture was extracted with EtOAc (3x). The combined organic extracts were dried over MgSO₄ and concentrated *in vacuo.* The residue was purified with flash column chromatography (ISCO, 0 - 30% EtOAc in hexane) to provide the title compound (7.40 g, 65%) as a white solid. ¹H NMR (CD₃OD) δ 5.96 - 5.60 (m, 1H), 5.28 - 5.00 (m, 2H), 4.60 (dd, *J*= 8.8, 7.0 Hz, 1H), 3.79 (s, 3H), 2.92 - 2.66 (m, 1H), 2.62 - 2.44 (m, 2H), 2.34 - 2.12 (m, 1H), 1.68 (ddd, *J*= 13.1, 8.2, 7.3 Hz, 1H), 1.48 (s, 9H); MS(ESI⁺) *mlz* 284.1 (M + H)⁺.

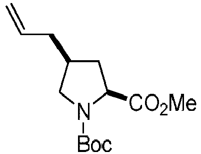

### B) (2S,4S)-1-tert-Butyl 2-methyl 4-allylpyrrolidine-1,2-dicarboxylate

To a solution of (2*S*,4*S*)-1-*tert*-butyl 2-methyl 4-allyl-5-oxopyrrolidine-1,2-dicarboxylate (7.40 g, 26.1 mmol) in THF (60 mL) at -78 °C was added dropwise a solution of lithium triethylhydroborate (Aldrich, 1M in THF, 26.1 mL, 26.1 mmol). The reaction mixture was stirred at -78 °C for 2 h before it was quenched with aq. NaHCO₃ soln. (30 mL). The resulting mixture was then allowed to warm to 0 °C. A solution of hydrogen peroxide (Aldrich, 50% in H₂O, 8.88 g, 131 mmol) was added dropwise. The mixture was stirred at room temperature for 30 min and concentrated *in vacuo* to remove THF. The residue was extracted with EtOAc (3x), and the combined organic layers were dried over MgSO₄ and concentrated *in vacuo.* The residue was purified using flash column chromatography (ISCO, 0 - 50% EtOAc in hexane) to provide the hemi-aminal intermediate.

To a cooled solution of the hemi-aminal intermediate in DCM (100 mL) at -78 °C was added triethylsilane (Aldrich, 4.59 mL, 28.7 mmol) followed by BF₃ 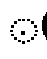 OEt₂ (Aldrich, 3.55 mL, 28.7 mmol). The reaction mixture was stirred at -78 °C for additional 2 h before it was quenched with aq. NaHCO₃ soln. (60 mL). The resulting mixture was allowed to warm to room temperature and extracted with DCM (3x). The combined organic layers were dried over MgSO₄, concentrated *in vacuo*, and purified using flash column chromatography (ISCO, 0 - 50% EtOAc in hexane) to provide the title compound as a colorless oil (5.50 g, 78%). ¹H NMR (400 MHz, CDCl₃) δ 5.83 - 5.70 (m, 1H), 5.12 - 4.99 (m, 2H), 4.32 - 4.17 (m, 1H), 3.83 - 3.77 (m, 1H), 3.76 (s, 3H), 3.17 - 2.99 (m, 1H), 2.46 - 2.37 (m, 1H), 2.30 - 2.12 (m, 2H), 1.68 - 1.58 (m, 2H), 1.40 (3, 9H); MS(ESI⁺) *m*/*z* 292.2 (M + Na)⁺.

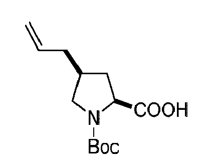

### C) (2S,4S)-4-Allyl-1-(tert-butoxycarbonyl)pyrrolidine-2-carboxylic acid

To a solution of (2*S*,4*S*)-1-*tert*-butyl 2-methyl 4-allylpyrrolidine-1,2-dicarboxylate (5.5 g, 20.42 mmol) in THF (50 mL) and MeOH (10 mL) was added aq. LiOH (1M, 30.6 mL, 30.6 mmol). The resulting mixture was stirred at room temperature for 5 h before it was cooled to 0 °C, acidified with 1 N HCl to pH 3-4, and extracted with DCM (3x). The combined organic extracts were dried over MgSO₄ and concentrated *in vacuo* to give the title compound (5.11 g, 98%) as a white solid. MS(ESI⁺) *mlz* 288.3 (M + Na)⁺.

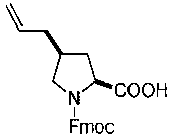

### D) (2S,4S)-1-(((9H-Fluoren-9-yl)methoxy)carbonyl)-4-allylpyrrolidine-2-carboxylic acid

To a solution of (2*S*,4*S*)-4-allyl-1-(*tert*-butoxycarbonyl)pyrrolidine-2-carboxylic acid (420 mg, 1.67 mmol) in DCM (4.5 mL) was added TFA (1.5 mL). The reaction was stirred at room temperature for 3 h before it was concentrated *in vacuo* and basified with aq. NaHCO₃ soln. To the resulting suspension was then added THF (10 mL) and Fmoc-OSu (564 mg, 1.67 mmol). The reaction was stirred at room temperature for 3 h before it was acidified with IN aq. HCl and extracted with EtOAc (3x). The combined organic extracts were washed with brine, dried over sodium sulfate, and concentrated *in vacuo.* The residue was then purified using flash column chromatography (ISCO, 0-10% MeOH/DCM) to give the title compound (370 mg, 59%) as a solid. MS(ESI⁺) *mlz* 378.1 (M + H)⁺.

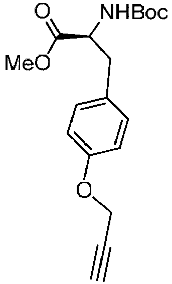

### E) (S)-Methyl 2-((tert-butoxycarbonyl)amino)-3-(4-(prop-2-yn-1-yloxy)phenyl) propanoate

To a solution of (*S*)-methyl-2-((*tert*-butoxycarbonyl)amino)-3-(4-hydroxphenyl) propanoate (Aldrich, 8.8 g, 30 mmol) in DMF (50 mL) was added potassium carbonate (Aldrich, 6.2 g, 45 mmol) and 3-bromoprop-1-yne (Aldrich, 80% in toluene, 8.9 g, 60 mmol). The reaction was heated to 70 °C for 4 h before it was cooled to room temperature and diluted with EtOAc and H₂O. The resulting mixture was extracted with EtOAc (3x) and the combined organic layers were washed with water, and then brine, dried over sodium sulfate, and concentrated *in vacuo.* The residue was purified using flash column chromatography (ISCO, 0-50% of EtOAc in hexane) to give the title compound as a white foam (7.5 g, 76%). ¹H NMR (400 MHz, CDCl₃) δ 7.09 (d, *J*= 8.6 Hz, 2H), 6.94 (d, *J*= 8.6 Hz, 2H), 4.99 (d, *J*= 7.0 Hz, 1H), 4.70 (d, *J*= 2.4 Hz, 2H), 4.58 (d, *J*= 7.0 Hz, 1H), 3.74 (s, 3H), 3.06 (dd, *J*= 12.7, 5.8 Hz, 2H), 2.55 (t, *J*= 2.4 Hz, 1H), 1.45 (s, 9H).

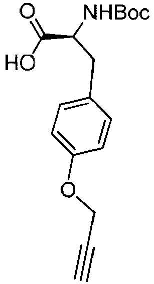

### F) (S)-2-((tert-Butoxycarbonyl)amino)-3-(4-(prop-2-yn-1-yloxy)phenyl) propanoic acid

To a solution of (*S*)-methyl 2-((*tert*-butoxycarbonyl)amino)-3-(4-(prop-2-yn-1-yloxy) phenyl) propanoate (4.1 g, 12.3 mmol) in THF (100 mL) was added aq. LiOH soln. (1M, 25 mL). After stirring at room temperature for 5 h, the reaction was acidified with IN HCl and extracted with ethyl acetate (3x). The combined organic extracts were washed with brine, dried over sodium sulfate, and concentrated *in vacuo* to give the title compound as a colorless oil (4.0 g, 99%). MS(ESI⁺) *mlz* 342.3 (M + Na)⁺.

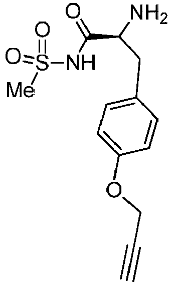

### G) (S)-2-Amino-N-(methylsulfonyl)-3-(4-(prop-2-yn-1-yloxy)phenyl) propanamide

To a solution (*S*)-2-((*tert*-butoxycarbonyl)amino)-3-(4-(prop-2-yn-1-yloxy) phenyl)propanoic acid (319 mg, 1 mmol) in THF (10 mL) was added CDI (Aldrich, 178 mg, 1.10 mmol). The resulting solution was stirred at 40 °C for 1 h. After cooled to room temperature, a solution of methanesulfonamide (Aldrich, 143 mg, 1.5 mmol) and DBU (Aldrich, 0.30 mL, 2.0 mmol) in THF (2 mL) was added. The reaction mixture was stirred at room temperature for 2 h before it was quenched with IN aq. HCl and extracted with EtOAc (2x). The combined organic extracts were washed with brine, dried over sodium sulfate, and concentrated *in vacuo.* The residue was purified using flash column chromatography (ISCO, 40 g column, 0-5% MeOH/DCM) to give a cololess liquid, which was was dissolved in 4N HCl/dioxane (5 mL) and stirred at room temperature for 2 h before it was concentrated *in vacuo* to give the title compound (245 mg, 83%) as a colorless liquid which slowly solidified to a white solid. MS(ESI⁺) *m*/*z* 297.2 (M + H)⁺.

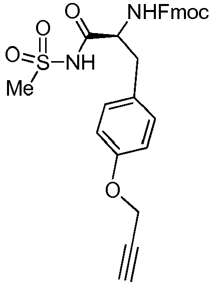

### H) (S)-(9H-Fluoren-9-yl)methyl(1-(methylsulfonamido)-1-oxo-3-(4-(prop-2-yn-1-yloxy)phenyl)propan-2-yl)carbamate

To a solution of (*S*)-2-amino-*N*-(methylsulfonyl)-3-(4-(prop-2-yn-1-yloxy) phenyl)propanamide (270 mg, 0.91 mmol) in THF/H₂O (1:1, 12 mL) was added sodium carbonate (145 mg, 1.37 mmol) and Fmoc-OSu (307 mg, 0.91 mmol). The reaction was stirred at room temperature for 2 h before it was acidified with IN aq. HCl and extracted with EtOAc (3x). The combined organic extracts were washed with brine, dried over sodium sulfate, and concentrated *in vacuo.* The residue was then purified using flash column chromatography (ISCO, 0-6% MeOH/DCM) to give the title compound (302 mg, 64%) as a colorless oil. MS(ESI⁺) *m*/*z* 519.3 (M + H)⁺.

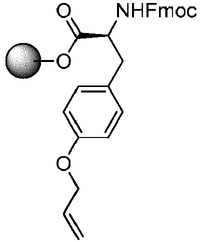

### I) Compound I

A solution of (*S*)-2-((((9*H*-fluoren-9-yl)methoxy)carbonyl)amino)-3-(4-(allyloxy)phenyl)propanoic acid (Chem-Impex Int'l Inc, 1.00 mmol, 443 mg) and DIPEA (Aldrich, 10 mmol, 1.75 mL) in DCM (20 mL) was added to 2-chlorotrityl resin (Annaspec, 1.58 mmol/g, 3.0 mmol, 1.9 g) in a Biorad (Bio-Rad Laboratories) preparative column. The resin was rocked for 2 h and MeOH (4 mL) was then added, followed by rocking for an additional 1 h. The solvent was removed by filtration, and the solid resin was washed with DMF (2 x 10 mL) and then DCM (3 x 10 mL). The resulting resin was then dried under N₂ overnight to give the resin-linked product.

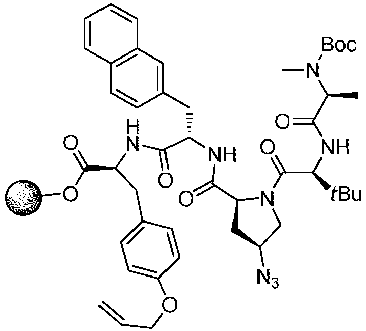

### J) Compound J

On a Prelude Peptide Synthesizer (Protein Technology Inc. Tucson, AZ), resin-linked compound from the previous step (0.25 mmol) was swelled with DMF (7 mL x 4 min) and mixed with a gentle stream of N₂ every 30 seconds. The solvent was drained and the following method was used to couple the first amino acid: the Fmoc group was removed from the resin-supported building block by washing the resin twice with a solution of 20% piperidine in DMF (5 mL and 2.5 minutes per wash) and mixing with a gentle stream of N₂ every 30 seconds. The resin was washed three times with DMF (8 mL and 1.5 min per wash). (*S*)-2-((((9*H-*Fluoren-9-yl)methoxy)carbonyl)amino)-3-(naphthalen-2-yl)propanoic acid (Chem-Impex Int'l Inc, 0.2 M solution in DMF, 5 mL, 1 mmol) was then added, followed by HATU (Oakwood, 0.4M solution in DMF, 2.5 mL, 1 mmol) and NMM (Aldrich, 0.8 M in DMF, 2.5 mL, 2 mmol). The reaction mixture was agitated by a gentle stream of nitrogen for 1 h. The reagents were drained from the reaction vessel, and the resin was washed three times with DMF (8 mL x 1.5 min).

The resulting resin-supported Fmoc-protected dipeptide was then sequentially deprotected and coupled with (2*S*,4*S*)-1-(((9*H*-fluoren-9-yl)methoxy)carbonyl)-4-azidopyrrolidine-2-carboxylic acid (Chem-Impex Int'l Inc, 1 mmol, 1 h), followed by (*S*)-2-((((9*H-*fluoren-9-yl)methoxy)carbonyl)amino)-3,3-dimethylbutanoic acid (Chem-Impex Int'l Inc, 1 mmol, 3 h), and then (*S*)-2-((*tert*-butoxycarbonyl)(methyl)amino) propanoic acid (Chem-Impex Int'l Inc, 1 mmol, 1 h) to give the resin-supported product.

LC-MS analysis was performed on peptide cleaved from the resin (analytical amount of the resin was treated with TFA (1% solution in DCM, 0.2 mL) at room temperature for 1 min and then filtered through a syringe filter to give a solution of the free peptide). MS(ESI⁺) *mlz* 855.5 (M + H)⁺.

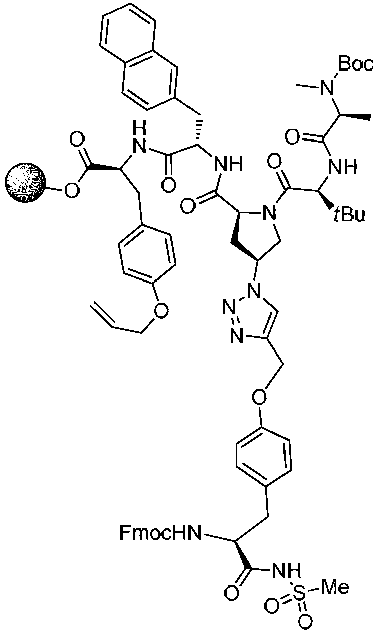

### K) Compound K

To a suspension of the resin-linked peptide **Compound J** (0.25 mmol) and Fmoc protected acylsulfonamide **Compound G** (130 mg, 0.25 mmol) in DMF (3 mL) in a preparative column was added a freshly made solution of copper(II) (Z)-2,2,6,6-tetramethyl-5-oxohept-3-en-3-olate (Strem, 0.125 mmol, 54 mg), sodium ascorbic acid (Aldrich, 0.75 mmol, 140 mg), DIPEA (Aldrich, 2.5 mmol, 0.45 mL), 2,6-dimethylpyridine (Aldrich, 2.5 mmol, 0.3 mL) in DMF (3 mL) and THF (3 mL). The reaction mixture was rocked at room temperature for 4 h. The reagents were drained from the reaction vessel, and the resin was washed three times with DMF (8 mL x 5 min) to give the resin-supported product.

LC-MS analysis was performed on peptide cleaved from the resin (analytical amount of the resin was treated with TFA (1% solution in DCM, 0.2 mL) at room temperature for 1 min and then filtered through a syringe filter to give a solution of the free peptide). MS(ESI⁺) *mlz* 1373.6 (M + H)⁺.

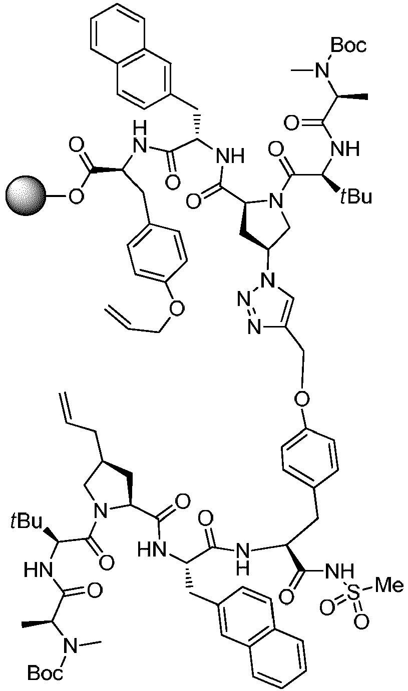

### L) Compound L

The resin-supported Fmoc-protected peptide from the previous step was sequentially deprotected and coupled with (*S*)-2-((((9*H*-fluoren-9-yl)methoxy)carbonyl) amino)-3-(naphthalen-2-yl)propanoic acid (1 mmol, 1 h), Fmoc protected acid **Compound C** (1 mmol, 1 h), followed by (*S*)-2-((((9*H*-fluoren-9-yl)methoxy)carbonyl)amino)-3,3-dimethylbutanoic acid (1 mmol, 3 h), and then (S)-2-((*tert*-butoxycarbonyl) (methyl)amino)propanoic acid (1 mmol, 1 h) to give the resin-supported product.

LC-MS analysis was performed on peptide cleaved from the resin using the following protocol: analytical amount of the resin was treated with TFA (1% solution in DCM, 0.2 mL) at room temperature for 1 min and then filtered through a syringe filter to give a solution of the free peptide. MS(ESI⁺) *mlz* 1783.9 (M + H)⁺.

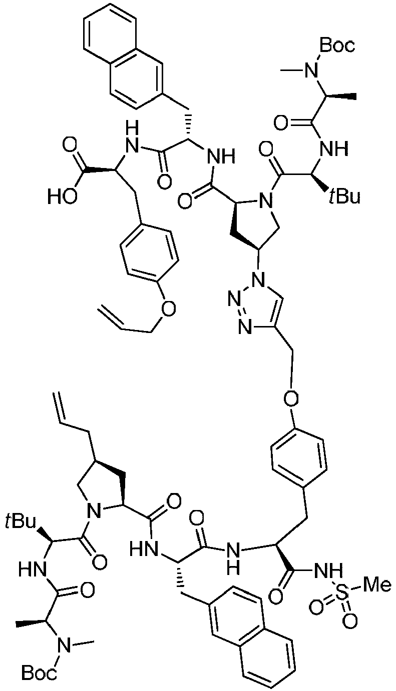

### M) (S)-2-((S)-2-((2S,4S)-4-(4-((4-((S)-2-((S)-2-((2S,4S)-4-Allyl-1-((S)-2-((S)-2-((tert-butoxycarbonyl)(methyl)amino)propanamido)-3,3-dimethylbutanoyl)pyrrolidine-2-carboxamido)-3-(naphthalen-2-yl)propanamido)-3-(methylsulfonamido)-3-oxopropyl)phenoxy)methyl)-1H-1,2,3-triazol-1-yl)-1-((S)-2-((S)-2-((tert-butoxycarbonyl)(methyl)amino)propanamido)-3,3-dimethylbutanoyl)pyrrolidine-2-carboxamido)-3-(naphthalen-2-yl)propanamido)-3-(4-(allyloxy)phenyl)propanoic acid

The resin from previous step was washed three times with CH₂Cl₂ (8 mL and 30 seconds per wash), and then with a mixture of CH₂Cl₂:AcOH: CF₃CH₂OH (3:1:1, 15 mL) The reaction mixture was rocked for 2 h. The AcOH washings were combined and the solvents were removed *in vacuo* to give the title compound. MS(ESI⁺) *m*/*z* 1783.9 (M + H)⁺.

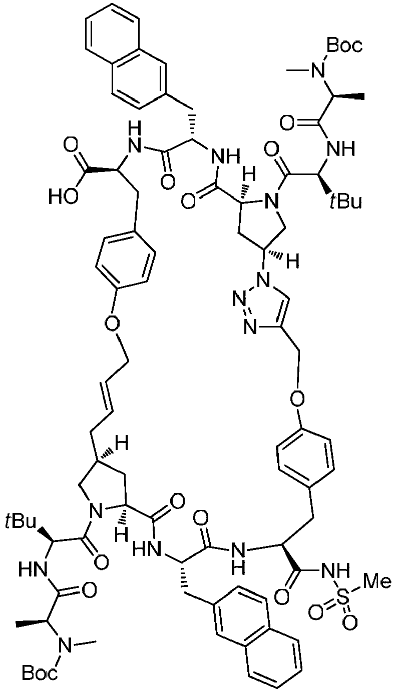

### N) Compound N

To a solution of the peptide from the previous step (211 mg, 0.118 mmol) in DCE (40 mL) was added a solution of Grubbs II catalyst (Aldrich, 10.04 mg, 0.012 mmol) in DCE (1 mL). The resulting reaction mixture was purged with N₂ for 5 min and stirred at 70 °C for 1 h. A second batch of Crrubbs II catalyst (10.0 mg, 0.012 mmol in DCE (1 mL)) was added and the reaction was stirred at 70 °C for 12 h. The reaction mixture was then cooled to room temperature and concentrated *in vacuo.* The crude oil was purified using preparative HPLC to give the title compound (101 mg, 49%) as a white solid after lyophilization. MS(ESI⁺) *m*/*z* 1755.8 (M + H)⁺.

### O) Example 1

To a solution of the macrocyclic peptide from the previous step (101 mg, 0.058 mmol) in CH₂Cl₂ (5 mL) was added TFA (2 mL). The resulting reaction mixture was stirred at room temperature for 1 h and then concentrated *in vacuo.* The resulting oil was purified using preparative HPLC to give the TFA salt of the title compound as a white solid after lyophilization. The TFA salt was then dissolved in THF/H₂O (1:2, 2 mL) and treated with IN aq. HCl (0.1 mL). The resulting solution was lyophilized to give the HCl salt of the title compound (87 mg, 88%) as a white solid. MS(ESI⁺) *m*/*z* 779.0 (M + 2H)⁺.

### EXAMPLE 2

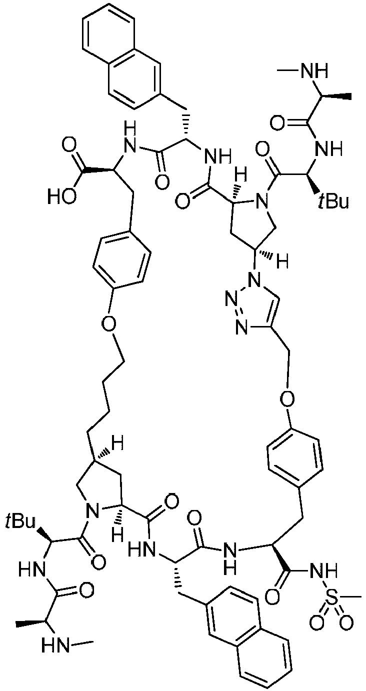

To 10% Pd/C (5 mg) in a hydrogenation flask was added a solution of **Example 1** (10 mg, 0.006 mmol) in MeOH (5 mL). The resulting suspension was then purged with H₂ and stirred under H₂ (50 psi) for 16 h. The reaction mixture was filtered through Celite^{®}, washed with MeOH, concentrated *in vacuo* and purified using preparative HPLC to give the title compound as a white solid after lyophilization. MS(ESI⁺) *m*/*z* 779.8 (M + 2H)⁺.

### EXAMPLES 3 TO 7

The following examples were prepared according to the procedures described for the synthesis of Example 1.

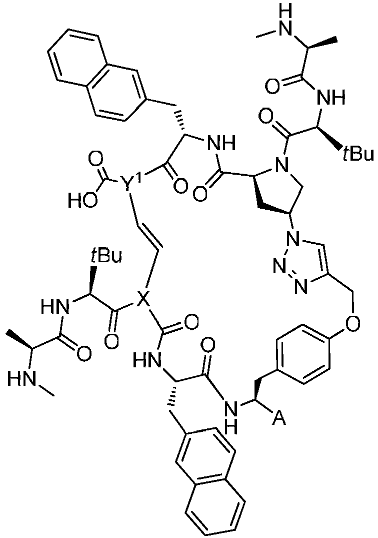

| **Ex. No.** | **Y¹** | **X** | **A** | **LCMS (M+H)** |
|---|---|---|---|---|
| **3** | 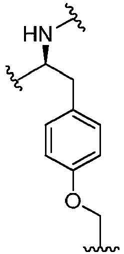 | 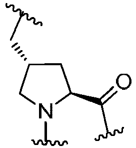 | 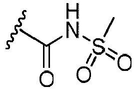 | 1556.4 |
| **4** | 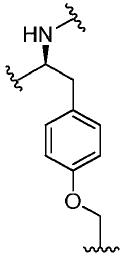 | 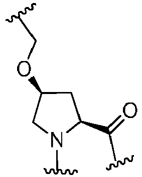 | 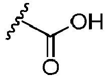 | 1495.5 |
| **5** | 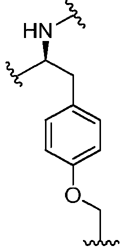 | 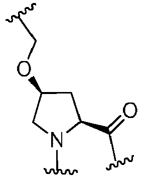 | 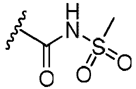 | 1572.9 |
| **6** | 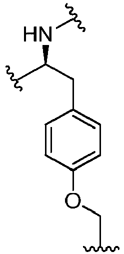 | 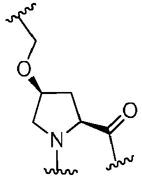 | 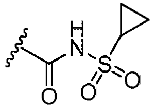 | 1599.4 |
| **7** | 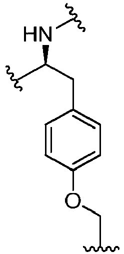 | 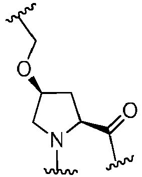 | 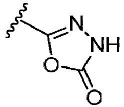 | 768.4 |

### EXAMPLES 8 TO 10

The following examples were prepared according to the procedures described for the synthesis of Example 2.

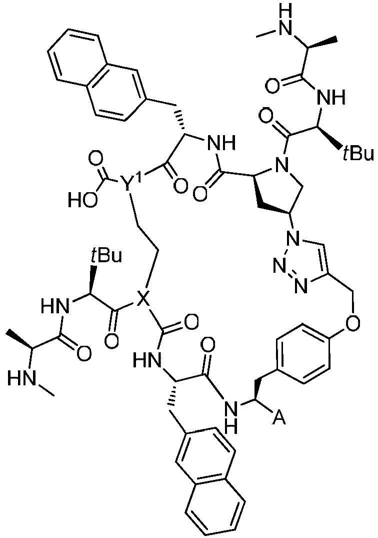

| **Ex. No.** | **Y¹** | **X** | **A** | **LCMS (M+H)** |
|---|---|---|---|---|
| **8** | 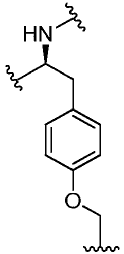 | 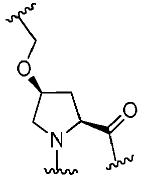 | 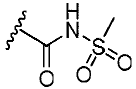 | 1573.2 |
| **9** | 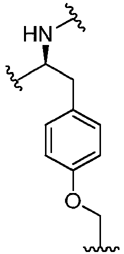 | 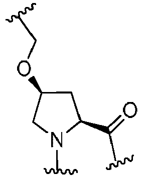 | 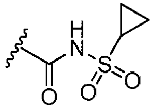 | 801.0 |
| **10** | 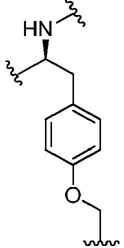 | 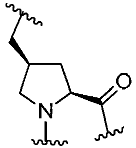 | 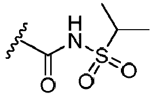 | 1585.4 |

### EXAMPLES 11 TO 21

The following examples of Formula 1, wherein each X is independently selected from 3-substituted prolines, were prepared according to the procedures analogous to that for the synthesis of Example 1.

| **Ex No.** | **Structure** | **Predicted MS** | **Observed MS** |
|---|---|---|---|
| **11** | 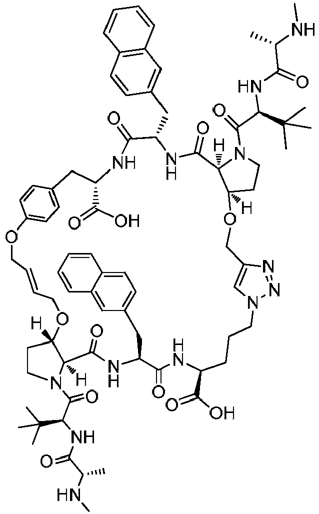 | 1446.71 | 724.4 |
| **12** | 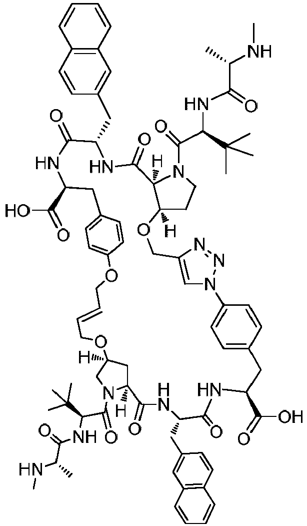 | 1494.75 | 1496.1 |
| **13** | 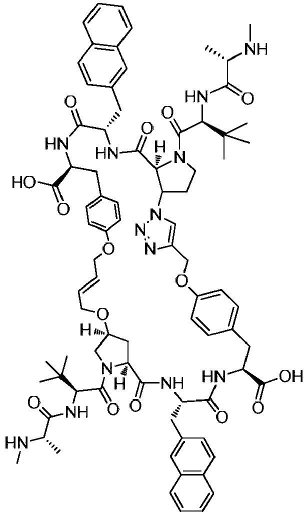 | 1494.75 | 748.2 |
| **14** | 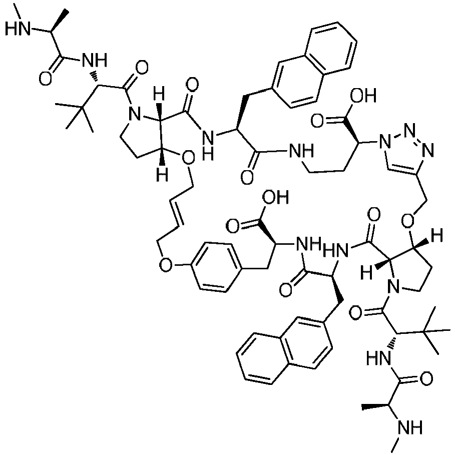 | 1432.68 | 1433.9 |
| **15** | 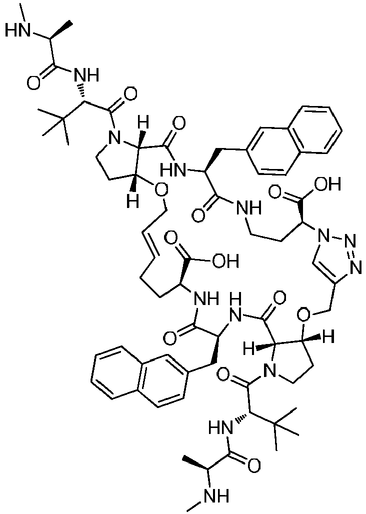 | 1340.58 | 671.0 |
| **16** | 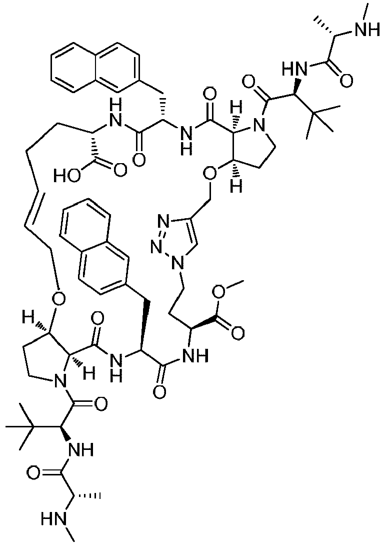 | 1354.61 | 678.4 |
| **17** | 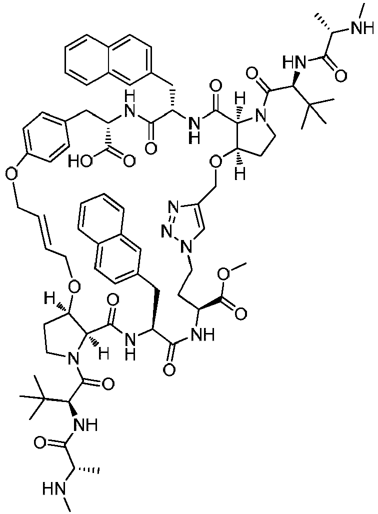 | 1446.71 | 724.0 |
| **18** | 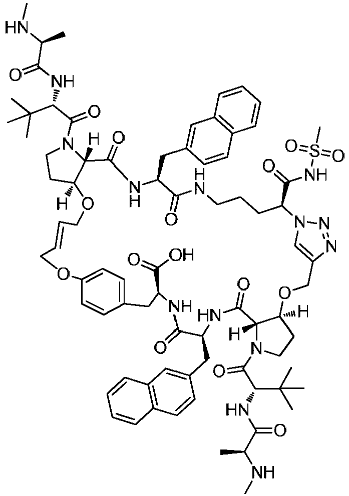 | 1523.81 | 762.5 |
| **19** | 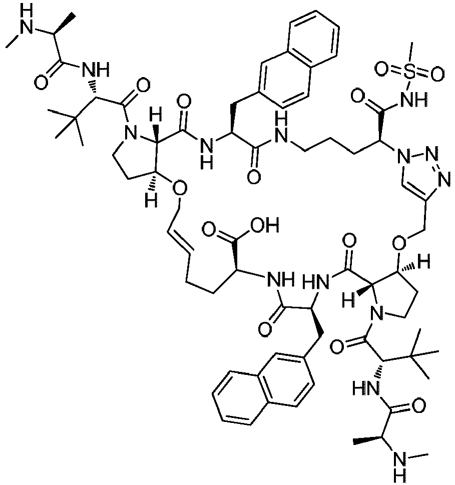 | 1431.72 | 716.5 |
| **20** | 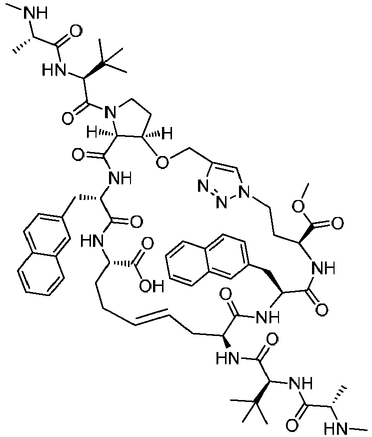 | 1298.54 | 1299.6 |
| **21** | 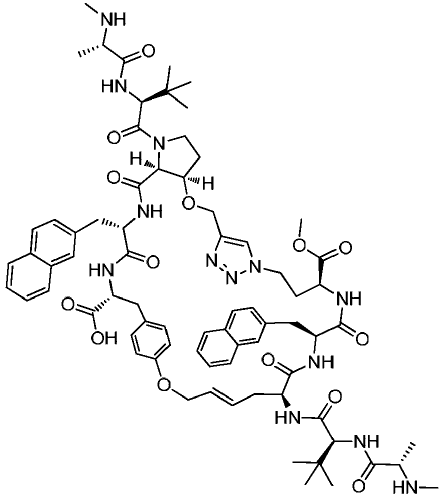 | 1390.64 | 696.1 |

### EXAMPLE 22

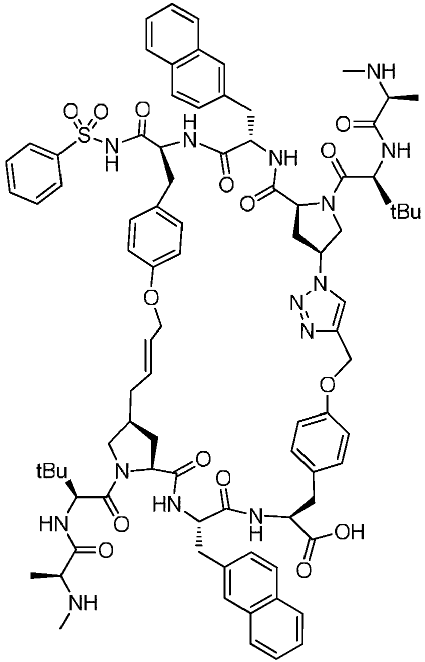

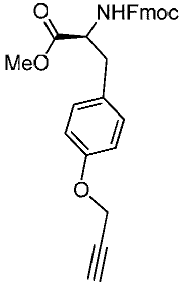

### A) (S)-Methyl 2-((((9H-fluoren-9-yl)methoxy)carbonyl)amino)-3-(4-(prop-2-yn-1-yloxy)phenyl)propanoate

To a solution of (*S*)-methyl 2-((*tert*-butoxycarbonyl)amino)-3-(4-(prop-2-yn-1-yloxy)phenyl)propanoate (**Compound E of Example 1**, 3.4 g, 10.2 mmol) in DCM (42 mL) was added TFA (18 mL). After 2 h, the reaction was concentrated *in vacuo* and basified with aq. NaHCO₃ soln. To the resulting suspension ws added THF (30 mL) and Fmoc-OSu (3.37g, 10 mmol). The reaction mixture was then stirred at room temperature for 2 h before it was acidified with aq. HCl and extracted with EtOAc (3x). The combined organic extracts were dried over sodium sulfate and concentrated *in vacuo.* The residue was purified using flash column chromatography (ISCO, 0-100% of EtOAc in hexane) to give the title compound (4.3 g, 94%) as a white foam. MS(ESI⁺) *m*/*z* 456.3 (M + H)⁺.

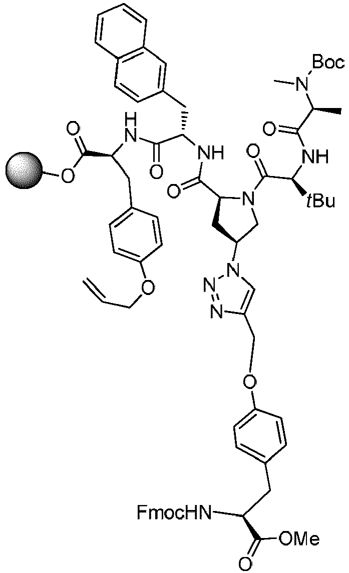

### B) Compound B

Following a procedure analogous to that for the synthesis of **Compound K of Example 1,** the resin-linked peptide **Compound J of Example 1** (0.25 mmol) and (*S*)-methyl 2-((((9*H*-fluoren-9-yl)methoxy)carbonyl)amino)-3-(4-(prop-2-yn-1-yloxy)phenyl) propanoate (**Compound A above,** 0.25 mmol) were converted to the resin-linked product using copper-mediated cycloaddition reaction.

LC-MS analysis was performed on peptide cleaved from the resin using the following protocol: analytical amount of the resin was treated with TFA (1% solution in DCM, 0.2 mL) at room temperature for 1 min and then filtered through a syringe filter to give a solution of the free peptide. MS(ESI⁺) *m*/*z* 1310.8 (M + H)⁺.

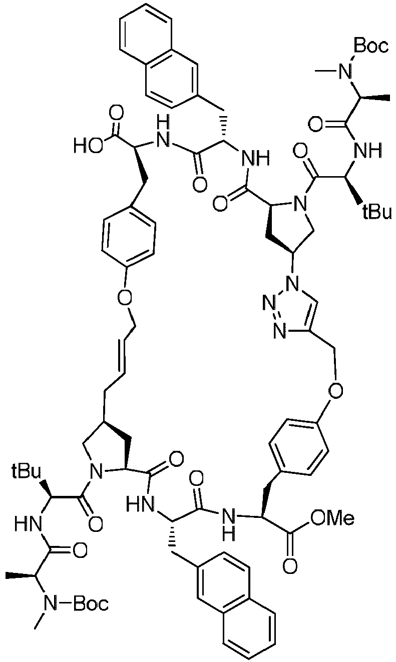

### C) Compound C

Following a procedure analogous to that for the synthesis of **Compound N of Example** 1, the peptide from the previous step (0.25 mmol) was converted to the title compound (97 mg, 25%). MS(ESI⁺) *m*/*z* 1692.8 (M + H)⁺.

### D) Example 22

To a solution of the compound from the previous step (6 mg, 0.0035 mmol) in THF (2.5 mL) was added a solution of CDI (1.7 mg, 0.011 mmol) in DCM (0.3 mL). After 1 h, a solution of DBU (5.4 µl, 0.035 mmol) and benzenesulfonamide (Aldrich, 5.6 mg, 0.035 mmol) in THF (1 mL) was added dropwise. The resulting solution was stirred at room temperature for 1 h before aq. LiOH (1M, 0.4 mL) was added. After 3 h, the reaction mixture was concentrated *in vacuo,* acidified with IN aq. HCl, and extracted with EtOAc (3 x). The combined organic extracts were washed with brine, and then dried over MgSO₄, filtered and concentrated *in vacuo.* To the resulting oil was added 30% TFA in DCM (3 mL). After 2 h, the solution was concentrated *in vacuo* and purified by preparative HPLC to give the title compound as a white solid after lyophilization. MS(ESI⁺) *m*/*z* 1618.3 (M + H)⁺.

### EXAMPLES 23 TO 26

The following examples were prepared according to the procedures described for the synthesis of Example 11.

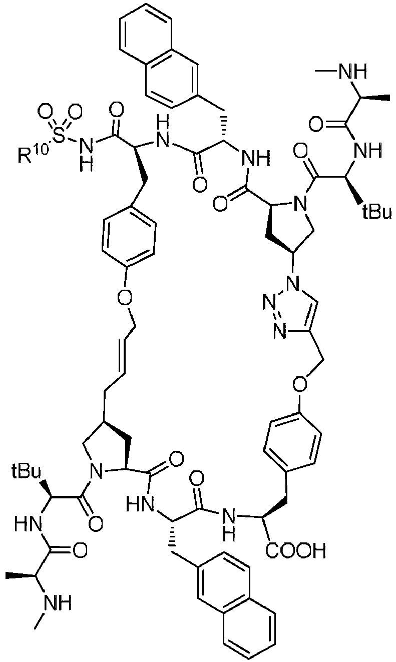

| **Ex. No.** | **R¹⁰** | **LCMS (M+H)** |
|---|---|---|
| **23** | 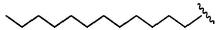 | 1709.8 |
| **24** | 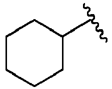 | 1624.8 |
| **25** | 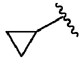 | 1581.8 |
| **26** | 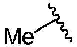 | 1555.6 |

### EXAMPLE 27

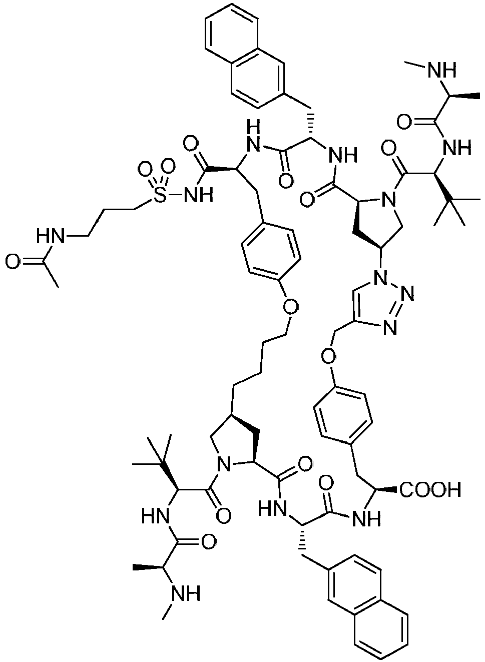

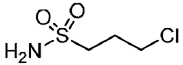

### A) 3-Chloropropane-1-sulfonamide

To a solution of 3-chloropropane-1-sulfonyl chloride (Aldrich, 1.4 g, 8 mmol) in THF (10 mL) at 0 °C was added dropwise aq. ammonia (15 mL). The solution was allowed to warm to room temperature and stirred at room temperature for 1 h. The resulting suspension was extracted with DCM (2x). The combined organic extracts were washed with water and then aq. HCl, dried over sodium sulfate, filtered, and concentrated *in vacuo* to give the title compound as a white solid. ¹H NMR (400 MHz, CDCl₃) δ 4.63 (br. s., 2H), 3.55 (t, *J* = 6.5 Hz, 2H), 3.39 - 3.19 (m, 2H), 2.32 - 2.11 (m, 2H).

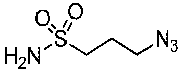

### B) 3-Azidopropane-1-sulfonamide

To a solution of the above chloride in DMF (30 mL) was added sodium azide (Aldrich, 1.04 g, 16 mmol) and tetrabutylammonium iodide (Aldrich, 100 mg, 0.27 mmol). The resulting suspension was stirred at 70 °C for 12 h and then allowed to room temperature before it was diluted with water and extracted with EtOAc (3x). The combined organic extracts were washed with water and then brine, dried over sodium sulfate, and concentrated *in vacuo* to give the title compound (805 mg, 62% over two steps) as a white solid. ¹H NMR (400 MHz, CDCl₃) δ 4.63 (br. s., 2H), 3.71 (t, *J* = 6.2 Hz, 2H), 3.40 - 3.09 (m, 2H), 2.63 - 2.28 (m, 2H)

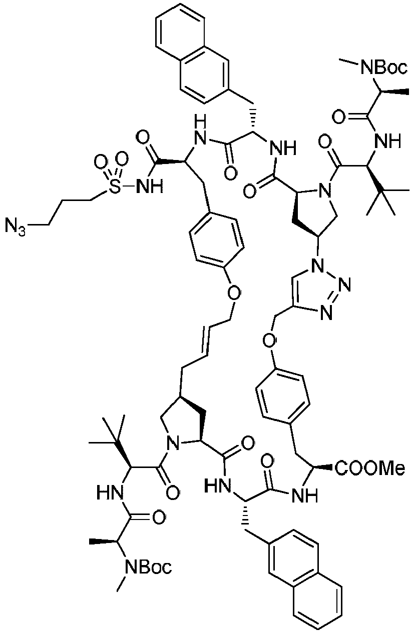

### C) Compound C

Following a procedure analogous to that for the synthesis of **Compound D of Example 22,** 3-azidopropane-1-sulfonamide (38 mg, 0.23 mmol) and **Compound C of Example 22,** (130 mg, 0.077 mmol) were converted to the title compound as a white solid. MS(ESI⁺) *m*/*z* 1839.1 (M + H)⁺.

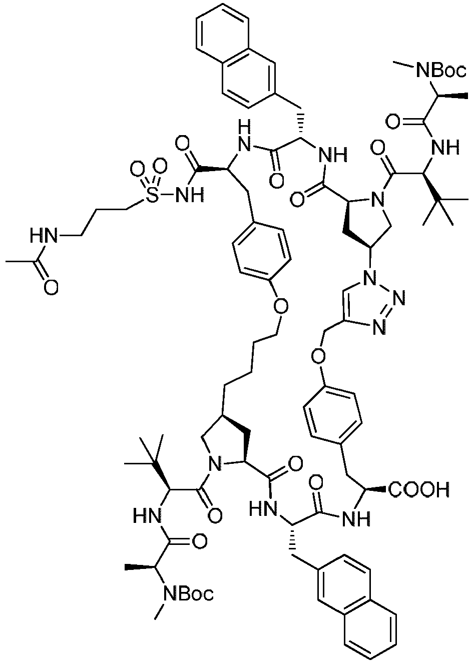

### D) Compound D

To 10% Pd/C (2 mg) was added a solution of the peptide from the previous step (3.2 mg, 0.0017 mmol) in MeOH (2 mL). The resulting suspension was stirred under H₂ (50 psi) at room temperature for 2 h and then purged with N₂. To the reaction was then added NEt₃ (Aldrich, 0.05 mL, 0.35 mmol) and acetyl chloride (Aldrich, 0.02 mL, 0.28 mmol). After 1 h, the reaction was concentrated *in vacuo* to remove organic solvent. The residue was dissolved in THF (1 mL) and aq. LiOH (1M, 0.3 mL, 0.3 mmol) was added. The resulting reaction was stirred at room temperature for 2 h before it was acidified with aq. HCl and extracted with EtOAc (2x). The combined organic extracts were dried over sodium sulfate and concentrated *in vacuo* to give the crude title compound which was used in the next step without further purification. MS(ESI⁺) *m*/*z* 922.1 (M + 2H)⁺.

### E) Example 27

To the crude compound from the previous step in DCM (0.7 mL) was added TFA (0.3 mL). The resulting reaction was stirred at room temperature for 2 h before it was concentrated *in vacuo* and purified using preparative HPLC to give the title compound (1 mg, 30%) as a white solid after lyophilization. MS(ESI⁺) *m*/*z* 822.5 (M + 2H)⁺.

### EXAMPLE 28

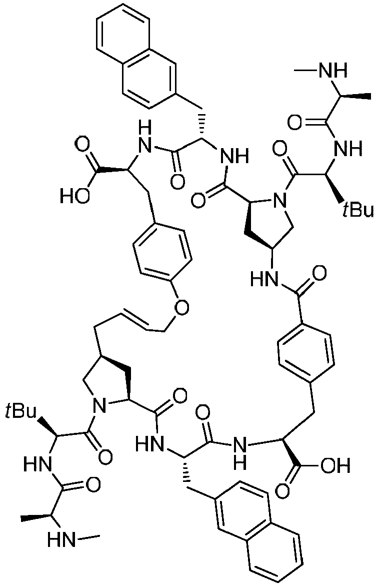

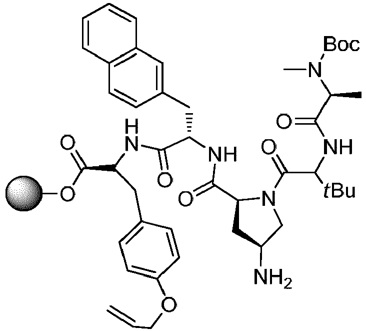

### A) Compound A

**Resin/Compound J of Example 1** (0.1 mmol) was swelled with THF (5mL) in a Biorad prep column. A solution of trimethylphosphine (Aldrich, 0.5 mL, 1M in toluene) was then added, followed by addition of H₂O (0.1 mL). The resin was rocked for 1 h and reagents were drained by filtration. The procedure was repeated one more time and the resulting solid resin was washed with THF (2 x 10 mL) and then DCM (3 x 10 mL).

LC-MS analysis was performed on peptide cleaved from the resin using the following protocol: analytical amount of the resin was treated with TFA (1% solution in DCM, 0.2 mL) at room temperature for 1 min and then filtered through a syringe filter to give a solution of the free peptide. MS(ESI⁺) *m*/*z* 829.2 (M + H)⁺.

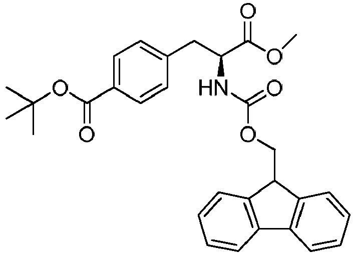

### B) (S)-tert-Butyl 4-(2-((((9H-fluoren-9-yl)methoxy)carbonyl)amino)-3-methoxy-3-oxopropyl)benzoate

To a solution of (*S*)-2-((((9*H*-fluoren-9-yl)methoxy)carbonyl)amino)-3-(4-(*tert-*butoxycarbonyl)phenyl)propanoic acid (Chem-Implex, Int', 1.6 g, 3.4 mmol) in DMF (15 mL) was added HATU (1.30 g, 3.4 mmol), methanol (2.18 g, 68.1 mmol), and NMM (1.19 mL, 6.8 mmol). The reaction mixture was stirred at room temperature for 12 h before it was quenched with aq. LiCl and extracted with EtOAc (3x). The combined organic extracts were washed with aq. LiCl, dried over sodium sulfate, filtered and concentrated *in vacuo.* The residue was purified by silicagel flash chromtography to afford the title compound (1.7 g, 100 %) as a white solid. MS(ESI⁺) *m*/*z* 502.1 (M + H)⁺.

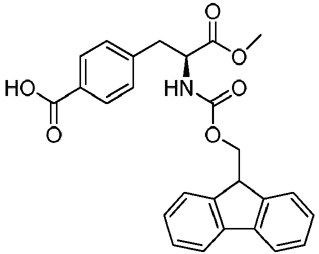

### C) (S)-4-(2-((((9H-Fluoren-9-yl)methoxy)carbonyl)amino)-3-methoxy-3-oxopropyl)benzoic acid

To a solution of (*S*)-*tert*-butyl 4-(2-((((9*H*-fluoren-9-yl)methoxy)carbonyl)amino) -3-methoxy-3-oxopropyl)benzoate (1.7 g, 3.4 mmol) in DCM (20 mL) was added TFA (6 mL). The reaction mixture was stirred at room temperature for 12 h before it was concentrated *in vacuo.* The residue was dissolved in DCM and treated with aq. HCl. The organic fraction was separated and the aqeous layer was extracted with DCM (2x). The combined organic extracts were dried over sodium sulfate, filtered, and concentrated *in vacuo* to afford the product (1.5 g, 98%) as a white solid. MS(ESI⁺) *m*/*z* 446.1 (M + H)⁺.

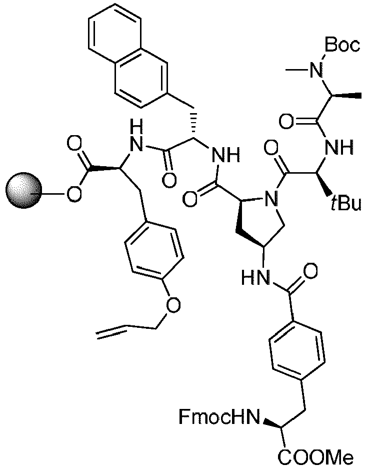

### D) Compound D

To resin-linked peptide **A** (0.1 mmol) in a Biorad column was added a solution of Fmoc protected acid C (0.1 M solution in DMF, 3 mL, 0.3 mmol), HATU (117 mg, 0.3 mmol), and *N*-methyl morpholine (0.072 mL, 0.6 mmol). The reaction mixture was rocked for 12 h at room temperature. The reagents were drained from the reaction vessel, and the resin was washed with DMF (2 x 10 mL) and then DCM (3 x 10 mL).

LC-MS analysis was performed on peptide cleaved from the resin using the following protocol: analytical amount of the resin was treated with TFA (1% solution in DCM, 0.2 mL) at room temperature for 1 min and then filtered through a syringe filter to give a solution of the free peptide. MS(ESI⁺) *m*/*z* 1257.3 (M + H)⁺.

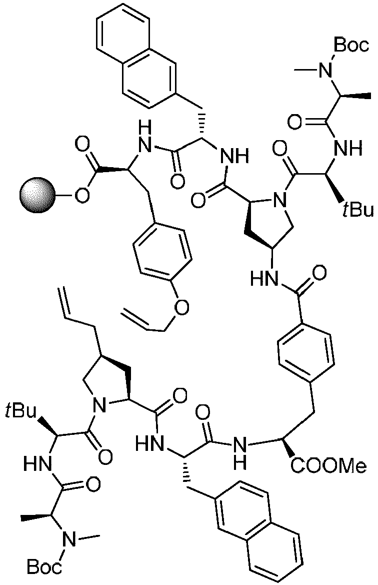

### E) Compound E

The resin-supported Fmoc-protected peptide from the previous step (0.1 mmol) was sequentially deprotected and coupled with (*S*)-2-((((9*H*-fluoren-9-yl)methoxy) carbonyl)amino)-3-(naphthalen-2-yl)propanoic acid (0.4 mmol, 1 h), **Fmoc-protected acid D of Example 1** (0.4 mmol, 1 h), followed by (*S*)-2-((((9*H*-fluoren-9-yl)methoxy)carbonyl)amino)-3,3-dimethylbutanoic acid (0.4 mmol, 3 h), and then (*S*)-2-((*tert*-butoxycarbonyl)(methyl) amino)propanoic acid (0.4 mmol, 1 h) to give the resin-supported product.

LC-MS analysis was performed on peptide cleaved from the resin using the following protocol: analytical amount of the resin was treated with TFA (1% solution in DCM, 0.2 mL) at room temperature for 1 min and then filtered through a syringe filter to give a solution of the free peptide. MS(ESI⁺) *m*/*z* 1666.6 (M + H)⁺.

### F) Example 28

Following a procedure analogous to that for the synthesis of **Compound N of Example 1,** the resin-linked peptide from the previous step (0.1 mmol) was converted to the title compound. MS(ESI⁺) *m*/*z* 1425.3 (M + H)⁺.

### EXAMPLES 29 TO 43

The following examples were prepared according to the procedures described for the synthesis of Example **28.**

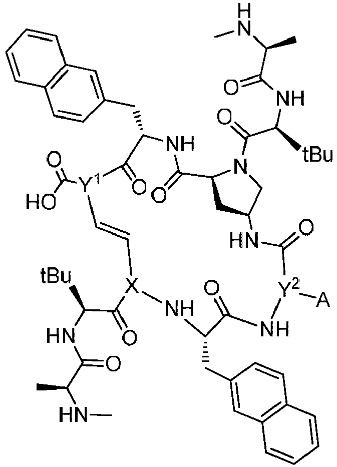

| **Ex. No.** | **Y¹** | **Y²** | **X** | **A** | **LCMS (M+H)** |
|---|---|---|---|---|---|
| **29** | 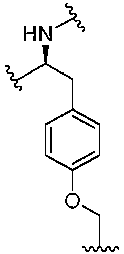 | 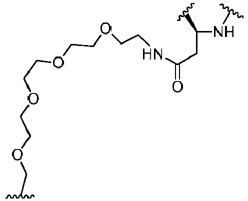 | 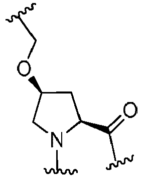 | 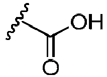 | 1612.1 |
| **30** | 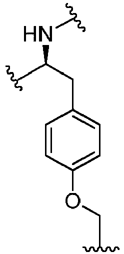 | 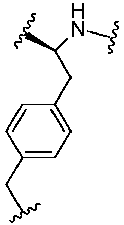 | 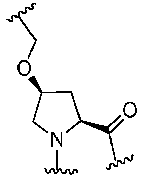 | 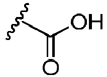 | 1455.0 |
| **31** | 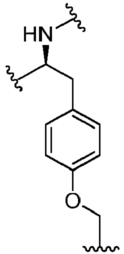 | 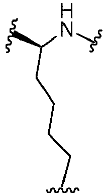 | 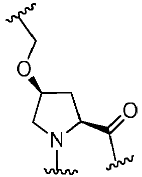 | 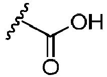 | 1406.3 |
| **32** | 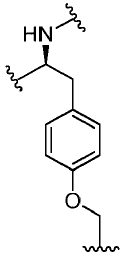 | 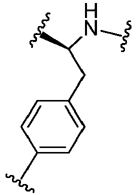 | 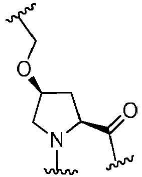 | 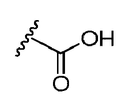 | 1441.2 |
| **33** | 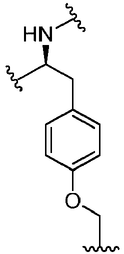 | 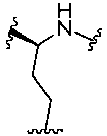 | 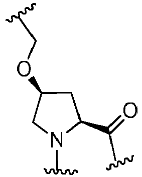 | 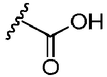 | 1378.6 |
| **34** | 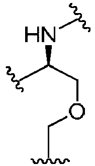 | 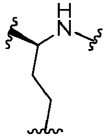 | 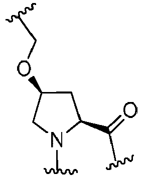 | 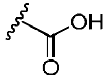 | 1303.5 |
| **35** | 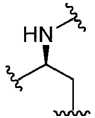 | 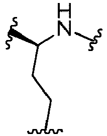 | 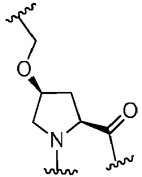 | 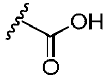 | 1272.4 |
| **36** | 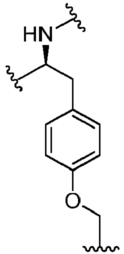 | 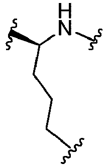 | 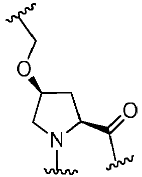 | 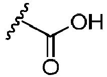 | 1392.9 |
| **37** | 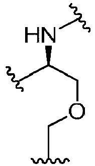 | 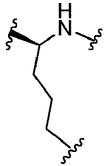 | 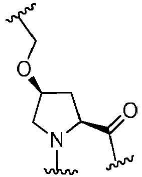 | 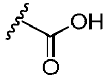 | 1316.8 |
| **38** | 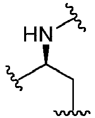 | 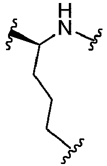 | 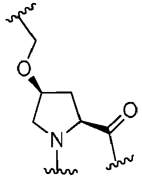 | 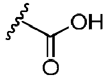 | 1286.7 |
| **39** | 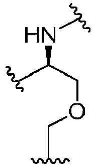 | 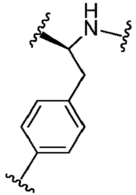 | 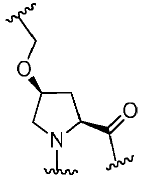 | 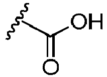 | 1365.3 |
| **40** | 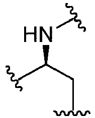 | 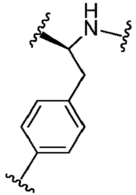 | 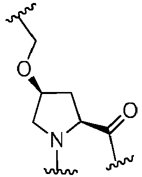 | 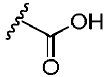 | 1334.3 |
| **41** | 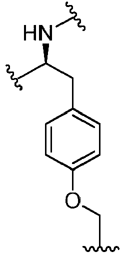 | 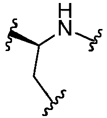 | 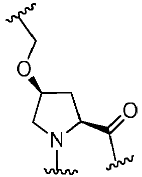 | 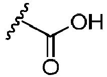 | 1364.4 |
| **42** | 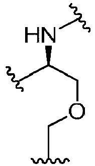 | 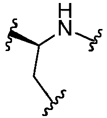 | 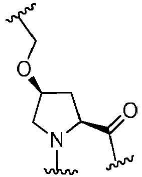 | 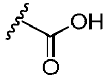 | 1289.4 |
| **43** | 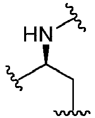 | 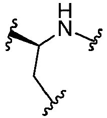 | 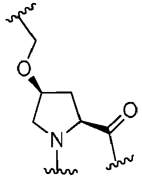 | 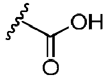 | 1258.5 |

### EXAMPLES 44 TO 54

The following examples were prepared according to the procedures described for the synthesis of Example **28.**

| **Ex No.** | **Structure** | **Predicted MS** | **Observed MS** |
|---|---|---|---|
| **44** | 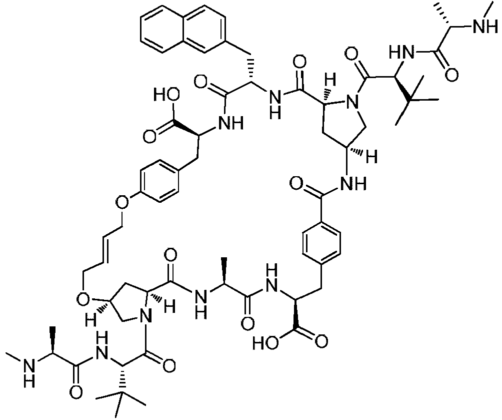 | 1314.54 | 1316.6 |
| **45** | 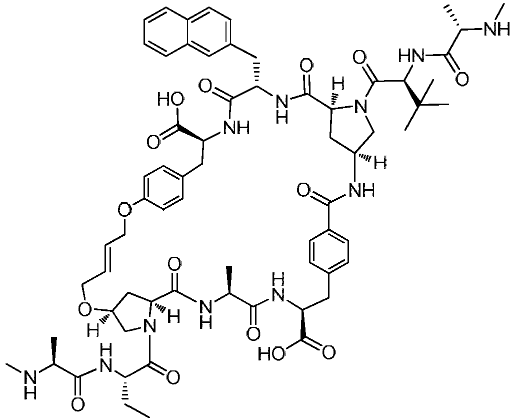 | 1286.49 | 1288.6 |
| **46** | 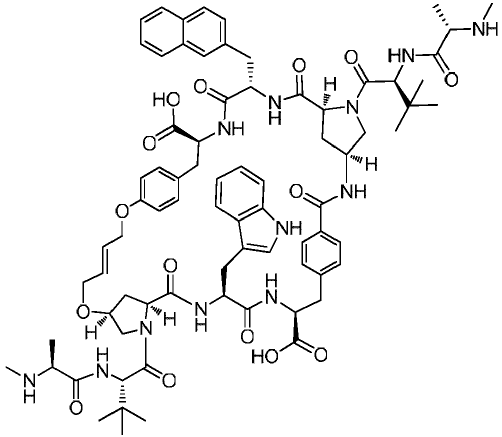 | 1429.67 | 1431.7 |
| **47** | 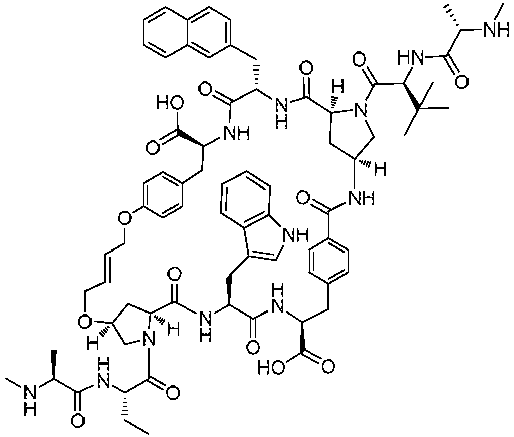 | 1401.62 | 1402.6 |
| **48** | 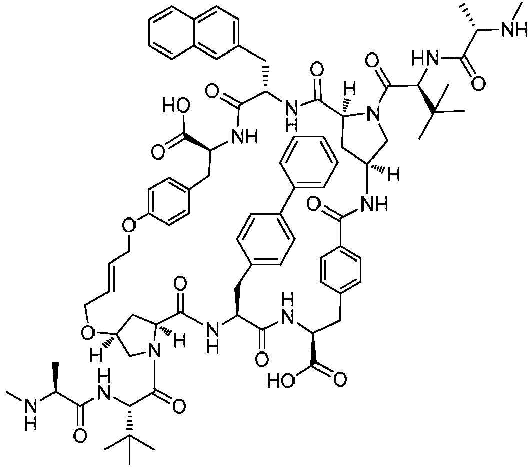 | 1466.74 | 1466.6 |
| **49** | 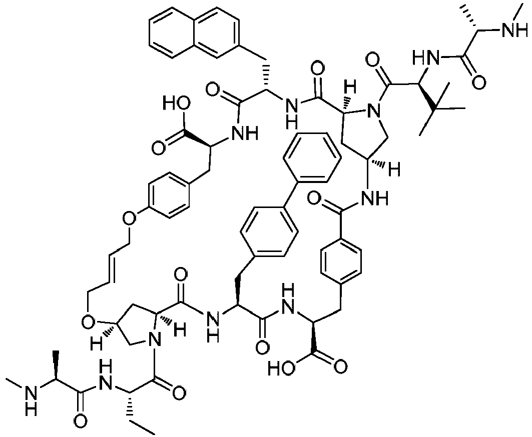 | 1438.68 | 1440.7 |
| **50** | 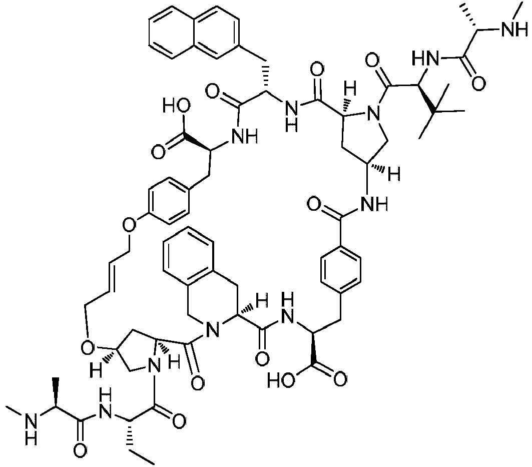 | 1374.59 | 1376.5 |
| **51** | 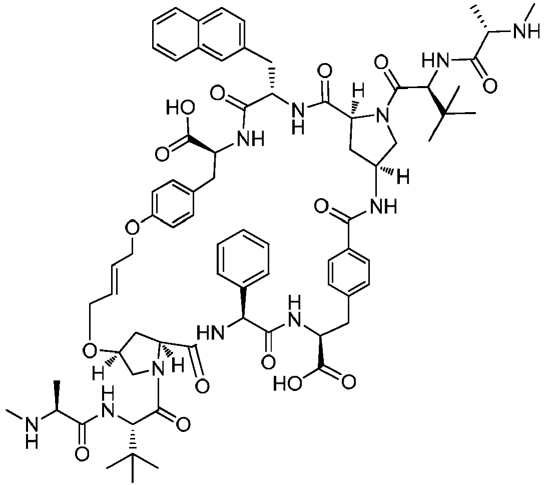 | 1376.61 | 1378.7 |
| **52** | 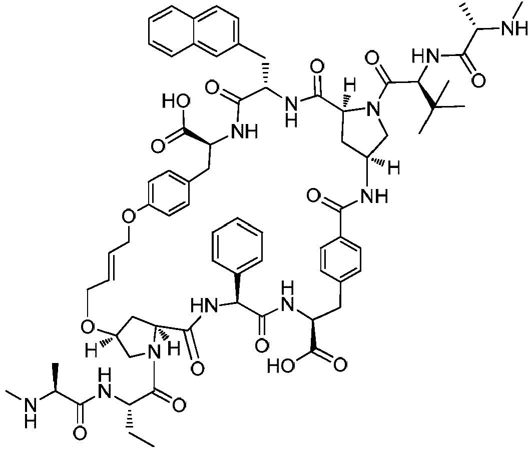 | 1348.56 | 1350.7 |
| **53** | 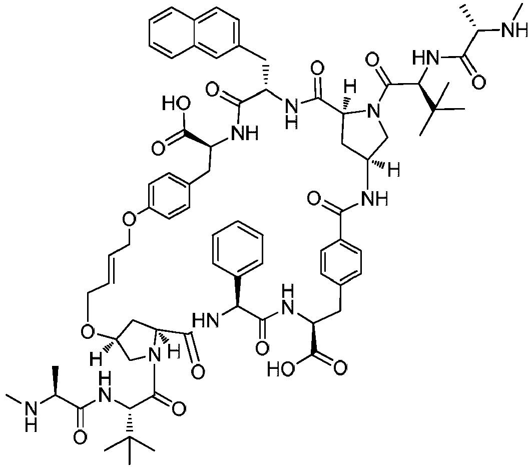 | 1376.61 | 1378.7 |
| **54** | 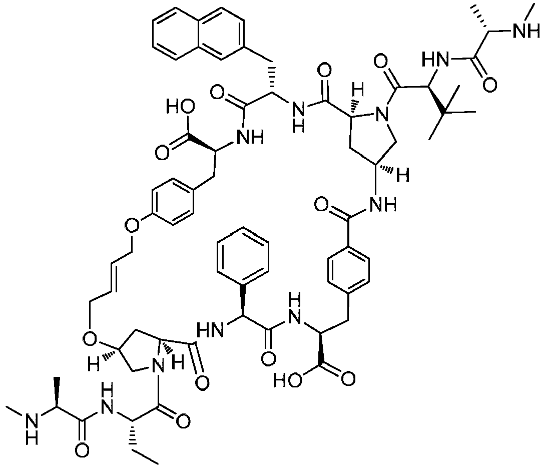 | 1348.56 | 1350.7 |

### EXAMPLES 55 TO 60

The following examples were prepared according to the procedures described for the synthesis of Example **28.**

| **Ex No.** | **Structure** | **Predicted MS** | **Observed MS** |
|---|---|---|---|
| **55** | 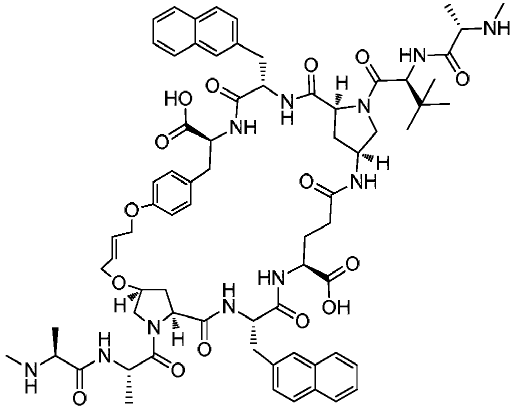 | 1336.55 | 669 M+2 |
| **56** | 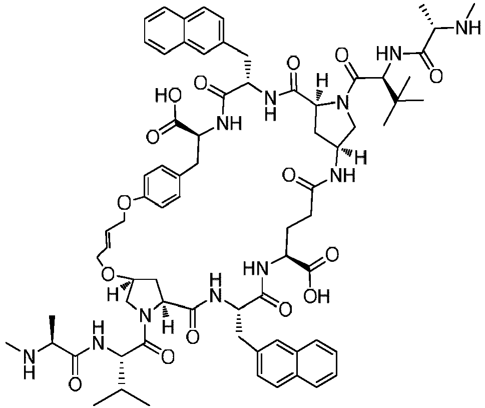 | 1364.6 | 683 M+2 |
| **57** | 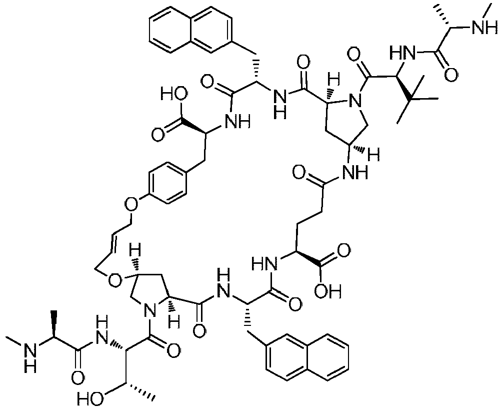 | 1366.57 | 684 M+2 |
| **58** | 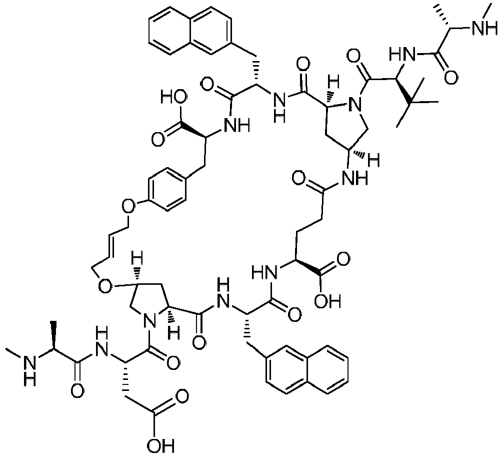 | 1380.56 | 691.2 M+2 |
| **59** | 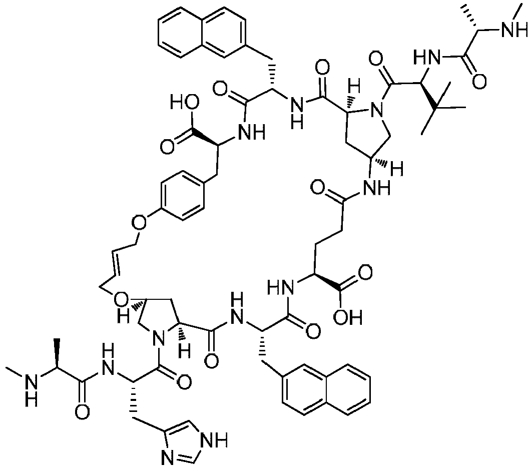 | 1402.61 | 701.9 M+2 |
| **60** | 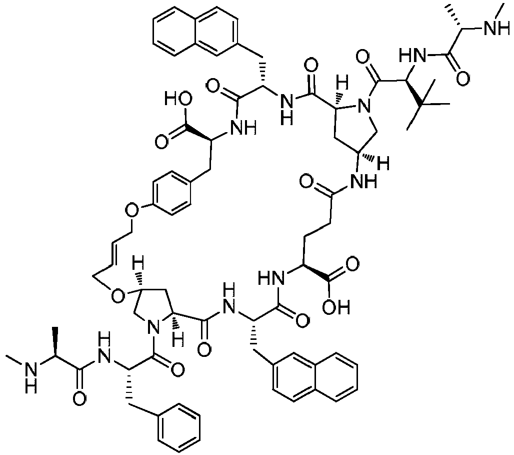 | 1412.64 | 707 |

### EXAMPLES 61 TO 75

The following examples were prepared according to the procedures described for the synthesis of Example **28.**

| **Ex No.** | **Structure** | **Predicted MS** | **Observed MS** |
|---|---|---|---|
| **61** | 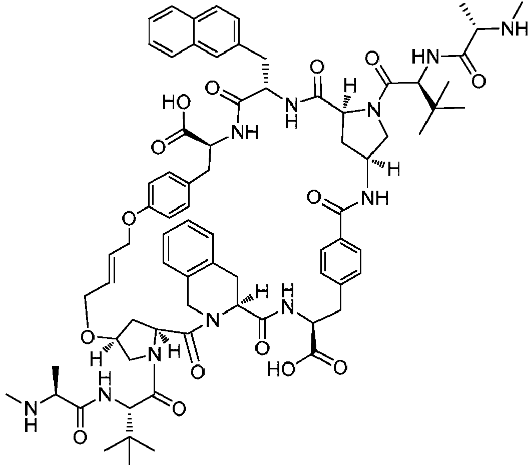 | 1402.65 | 1403.7 |
| **62** | 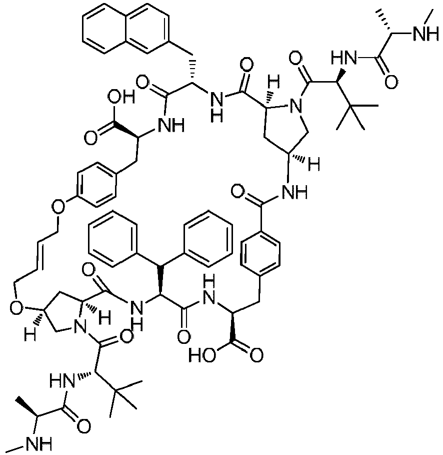 | 1466.74 | 1467.7 |
| **63** | 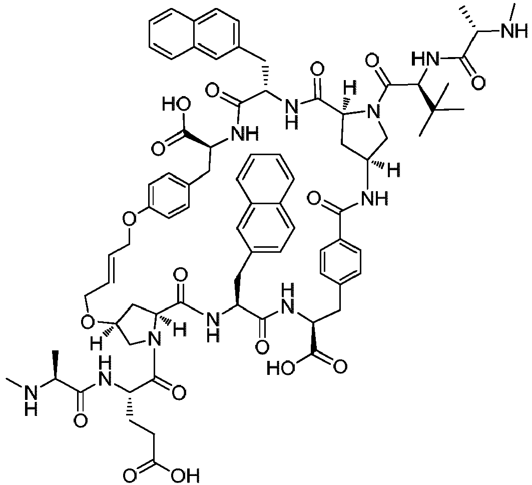 | 1456.65 | 1458.6 |
| **64** | 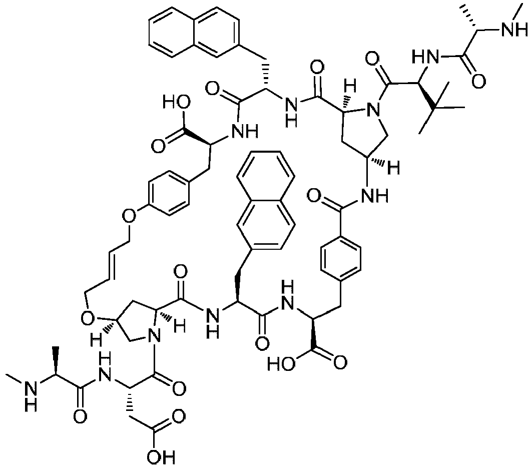 | 1442.63 | 1444.6 |
| **65** | 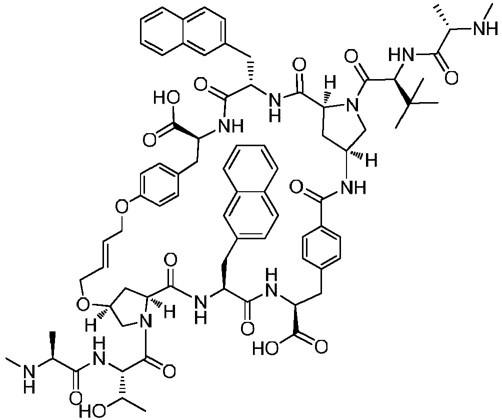 | 1428.64 | 1430.6 |
| **66** | 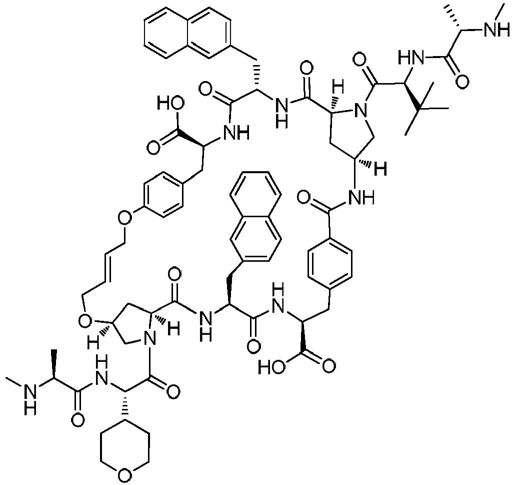 | 1468.71 | 1470.6 |
| **67** | 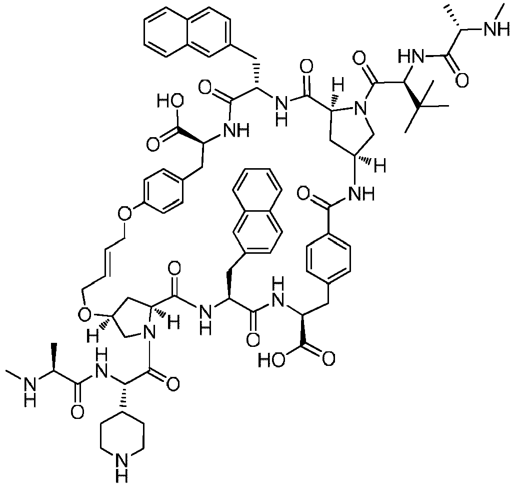 | 1467.72 | 1468.6 |
| **68** | 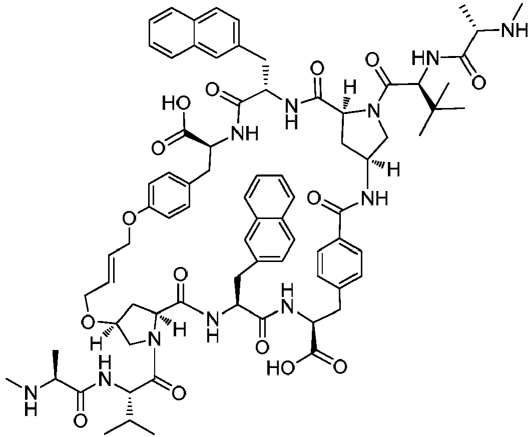 | 1426.67 | 1428.6 |
| **69** | 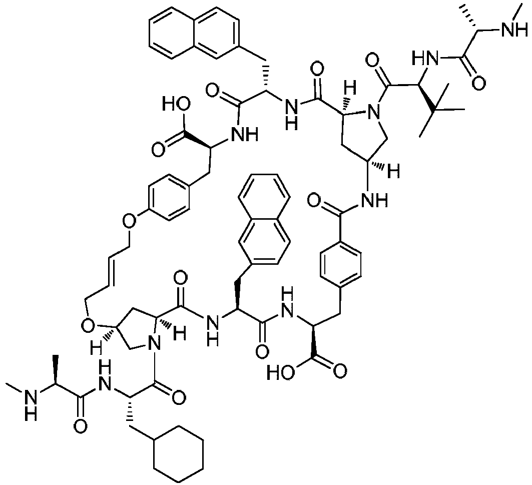 | 1480.76 | 1482.7 |
| **70** | 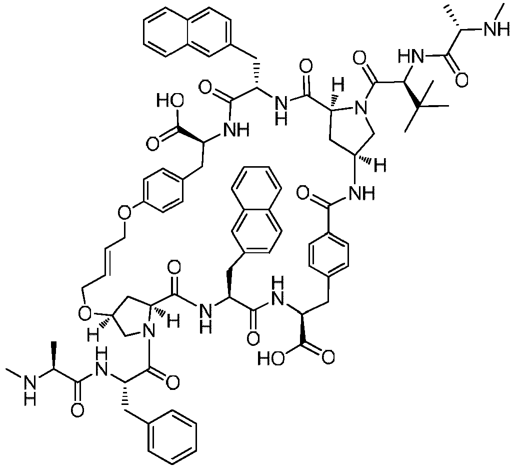 | 1474.71 | 1475.7 |
| **71** | 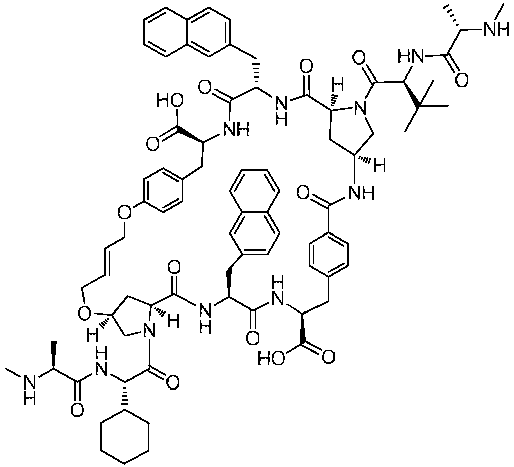 | 1466.74 | 1468.7 |
| **72** | 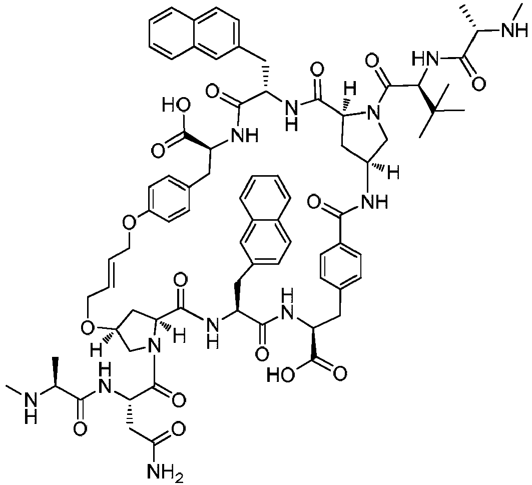 | 1441.64 | 1443.6 |
| **73** | 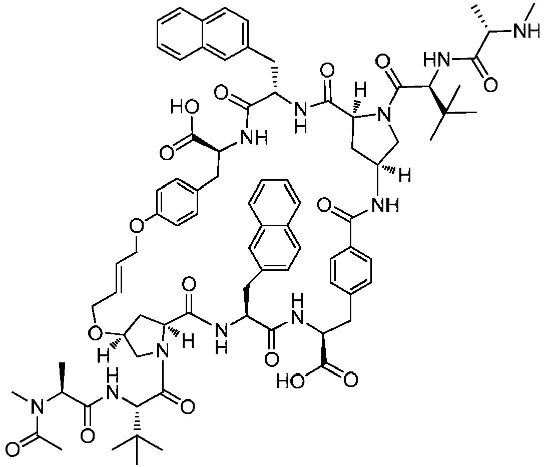 | 1482.73 | 1483.6 |
| **74** | 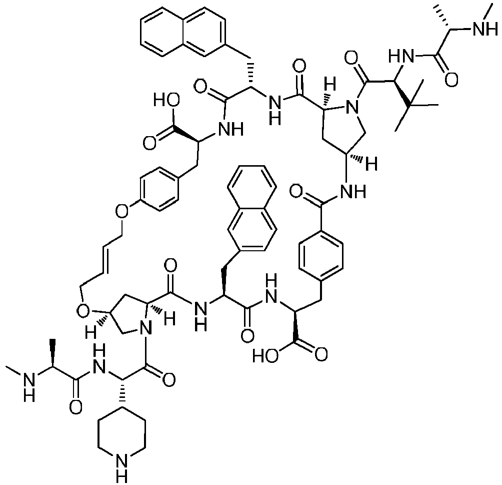 | 1467.72 | 1468.7 |
| **75** | 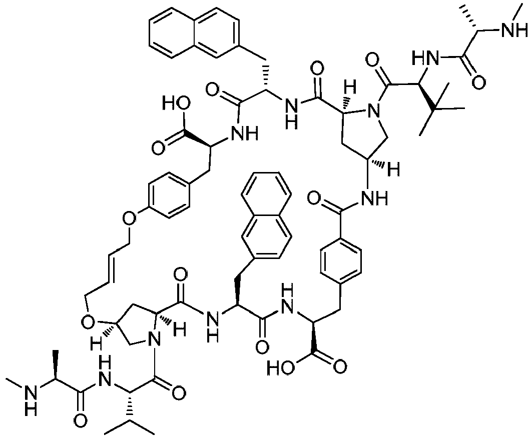 | 1426.67 | 1428.6 |

### EXAMPLES 76 TO 82

The following examples of Formula 1, wherein one of the X is a substituted piperazine were prepared according to the procedures described for the synthesis of Example **28.**

| **Ex No.** | **Structure** | **Predicted MS** | **Observed MS** |
|---|---|---|---|
| **76** | 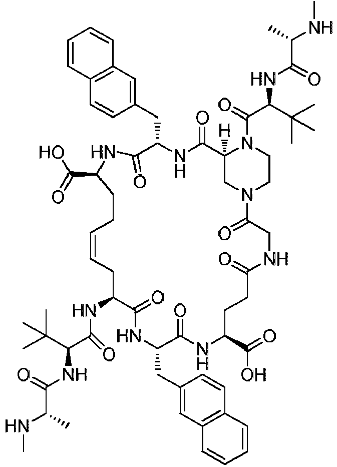 | 1287.52 | 644.5 M+2 |
| **77** | 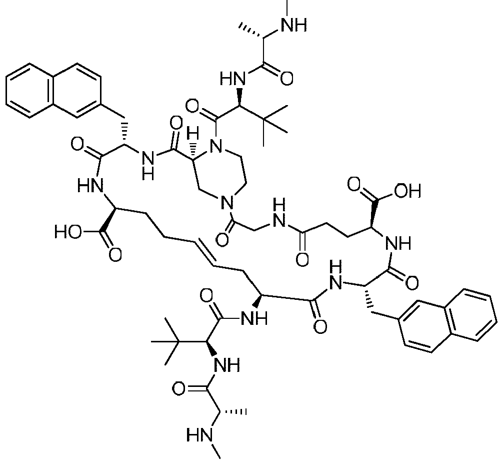 | 1287.52 | 644.5 M+2 |
| **78** | 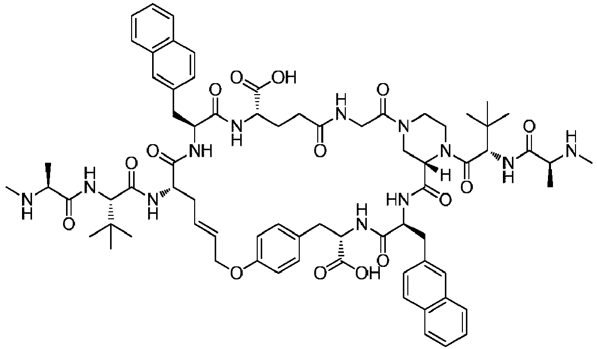 | 1379.61 | 1380.6 |
| **79** | 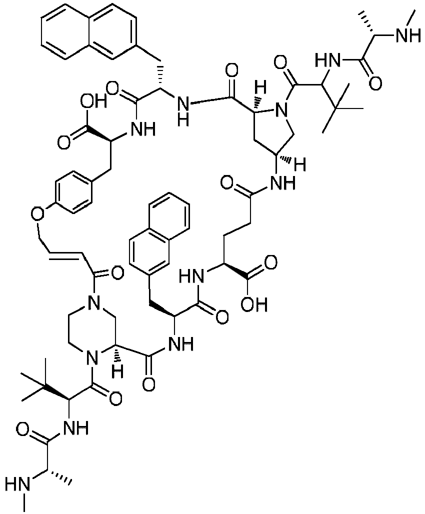 | 1391.63 | 1392.8 |
| **80** | 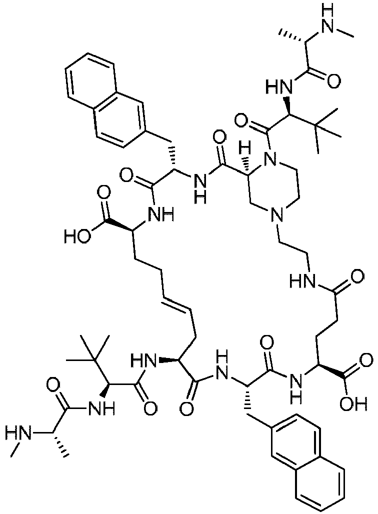 | 1273.53 | 637.3 M+2 |
| **81** | 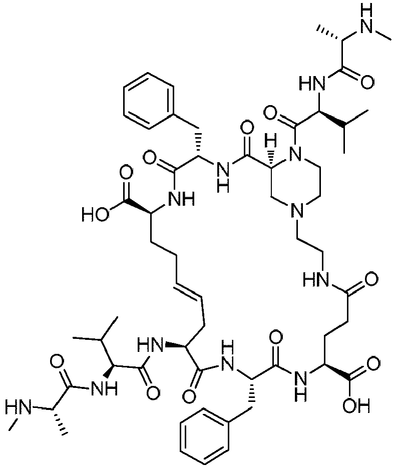 | 1145.36 | 573.3 M+2 |
| **82** | 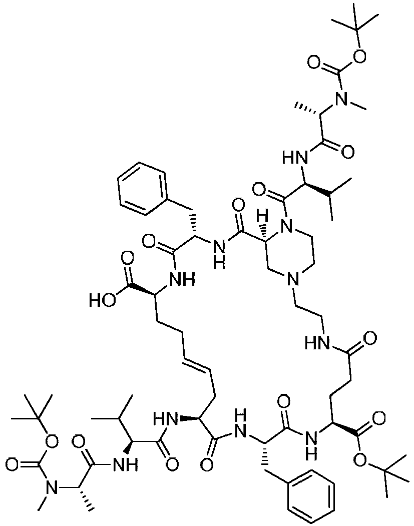 | 1401.7 | 1402.5 |

### EXAMPLE 83

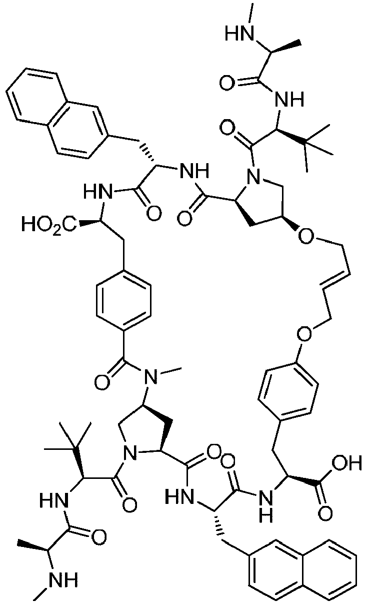

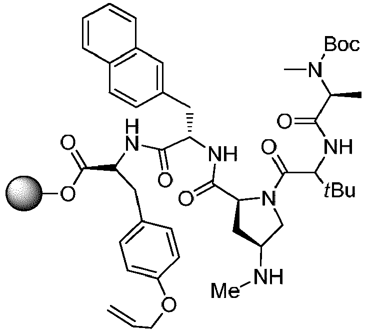

### A) Compound A

A solution of 2-nitrobenzenesulfonyl chloride (Aldrich, 0.11 g, 0.5 mmol) and 2,4,6-trimethylpyridine (Aldrich, 0.165 mL, 1.25 mmol) in NMP (3 mL) was added to **Compound A of Example 28** (0.125 mmol). The reaction was rocked for 15 min at room temperature. The resin was washed 5 times with NMP. A solution of DBU (0.056 mL, 0.38 mmol) in NMP (3 mL) was then added to the resin. The reaction was rocked for 3 min before a solution of dimethyl sulfate (0.12 mL, 1.250 mmol) in NMP was added. After 5 min, the resin was filtered and the latter procedure was repeated. The resin was washed five times with NMP. The rsulting resin was then treated with a solution of 2-mercaptoethanol (0.088 mL, 1.250 mmol) and DBU (0.056 mL, 0.375 mmol) in NMP for 5 min. The reageant was then filtered and washed with NMP (5x), followed by DCM (3x) and then dried.

LC-MS analysis was performed on peptide cleaved from the resin using the following protocol: analytical amount of the resin was treated with TFA (1% solution in DCM, 0.2 mL) at room temperature for 1 min and then filtered through a syringe filter to give a solution of the free peptide. MS(ESI⁺) *m*/*z* 843.0 (M + H)⁺.

### B) Example 83

Following procedures analogous to that for the synthesis of **Compound F of Example 28** (0.125 mmol), the resin-linked peptide from the previous step (0.1 mmol) was converted to the title compound. MS(ESI⁺) *m*/*z* 1654.5 (M + H)⁺.

### EXAMPLE 84

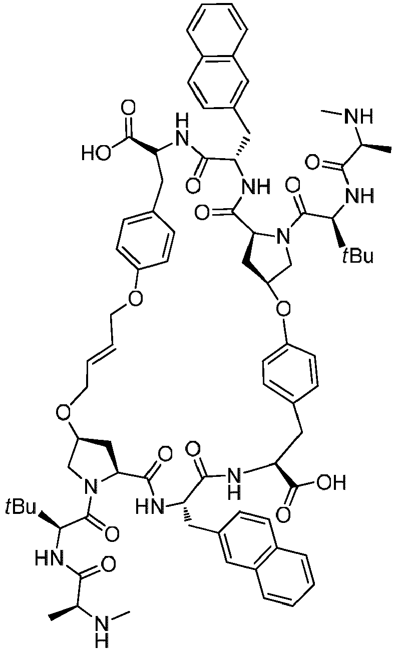

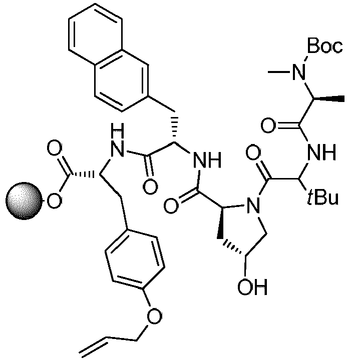

### A) Compound A

The resin-supported Fmoc-protected peptide / **Compound I of Example 1** (0.25 mmol) was sequentially deprotected and coupled with (*S*)-2-((((9*H*-fluoren-9-yl)methoxy)carbonyl)amino)-3-(naphthalen-2-yl)propanoic acid (1 mmol, 1 h), (2*S*,4*R*)-1-(((9H-fluoren-9-yl)methoxy)carbonyl)-4-hydroxypyrrolidine-2-carboxylic acid (Chem-Impex Int'l Inc, 1 mmol, 1 h), followed by (*S*)-2-((((9*H*-fluoren-9-yl)methoxy)carbonyl)amino)-3,3-dimethylbutanoic acid (1 mmol, 3 h), and then (*S*)-2-((*tert*-butoxycarbonyl)(methyl) amino)propanoic acid (1 mmol, 1 h) to give the resin-supported product.

LC-MS analysis was performed on peptide cleaved from the resin using the following protocol: analytical amount of the resin was treated with TFA (1% solution in DCM, 0.2 mL) at room temperature for 1 min and then filtered through a syringe filter to give a solution of the free peptide. MS(ESI⁺) *m*/*z* 830.6 (M + H)⁺.

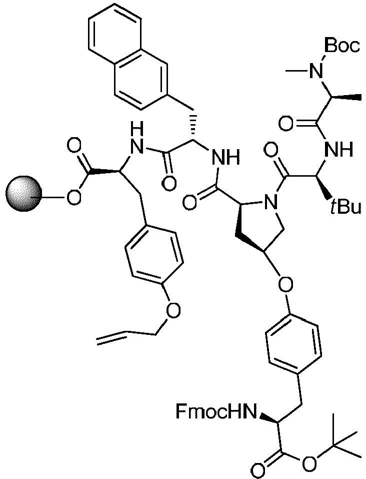

### B) Compound B

To resin-linked peptide from the previous step (0.1 mmol) in a Bio-Rad column was added a solution of triphenylphosphine (Aldrich, 131 mg, 0.5 mmol), (*S*)-*tert*-butyl 2-((((9*H*-fluoren-9-yl)methoxy)carbonyl)amino)-3-(4-hydroxyphenyl)propanoate (A Chem Tek, Inc, 230 mg g, 0.5 mmol,) in DCM/THF (1:1, 5 mL). The reaction mixture was rocked for 5 min before DIAD (Aldrich, 0.097 mL, 0.5 mmol) was added dropwise. The reaction mixture was then rocked for 12 h. The reagents were drained from the reaction vessel, and the resin was washed with DMF (3 x 5 mL) and then DCM (3 x 5 mL).

LC-MS analysis was performed on peptide cleaved from the resin using the following protocol: analytical amount of the resin was treated with TFA (1% solution in DCM, 0.2 mL) at room temperature for 1 min and then filtered through a syringe filter to give a solution of the free peptide. MS(ESI⁺) *m*/*z* 1272.3 (M + H)⁺.

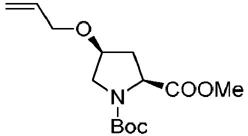

### C) (2S,4S)-1-tert-Butyl 2-methyl 4-(allyloxy)pyrrolidine-1,2-dicarboxylate

To a solution of (2*S*,4*S*)-1-*tert*-butyl 2-methyl 4-hydroxypyrrolidine-1,2-dicarboxylate (Chem-Impex Int'Inc, 3.5 g, 14.3 mmol) in acetone (50 mL) was added silver oxide ( Aldrich, 3.97 g, 17.1 mmol), allyl bromide (Aldrich, 1.85 mL, 21.4 mmol) and NEt₃ (2.98 mL, 21.4 mmol). The resulting suspension was stirred at room temperature for 6 h before it was filtered through a pad of Celite^{®} and concentrated *in vacuo.* The product was then purified using flash column chromatography (gradient from 10 - 30% EtOAc in hexane over 30 min) and isolated (2.3 g, 57%) as a colorless oil. ¹H NMR (400 MHz, CDCl₃) δ 5.87 (ddt, *J =* 17.2, 10.5, 5.4 Hz, 1H), 5.27 (dd, *J =* 17.3, 1.4 Hz, 1H), 5.18 (d, *J* = 10.6 Hz, 1H), 4.45 (dd, *J* = 8.6, 3.7 Hz, 1H), 4.33 (dd, *J* = 8.6, 4.2 Hz, 1H), 4.11 - 4.05 (m, 1H), 3.99 - 3.92 (m, 2H), 3.73 (s, 3H), 3.68 (dd, *J* = 11.6, 5.4 Hz, 1H), 3.62 (dd, *J =* 11.6, 5.4 Hz, 1H), 3.56 - 3.45 (m, 1H), 2.45 - 2.18 (m, 2H), 1.50 (s, 4H), 1.44 (s, 5H); MS(ESI⁺) *m*/*z* 286.3 (M + H)⁺.

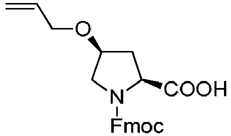

### D) (2S,4S)-1-(((9H-Fluoren-9-yl)methoxy)carbonyl)-4-(allyloxy)pyrrolidine-2-carboxylic acid

To a solution of (2S,4S)-1-*tert*-butyl 2-methyl 4-(allyloxy)pyrrolidine-1,2-dicarboxylate (2 g, 7.01 mmol) in THF/H₂O(4:1 120 mL) was added LiOH (1 g). The resulting reaction was stirred at room temperature for 12 h before it was concentrated *in vacuo* to remove the THF. The remaining aqeous layer was acidified with aq. HCl, and extracted with EtOAC (3x). The combined organic layers were washed with brine, dried over sodium sulfate and concentrated *in vacuo.*

The resulting acid was dissolved in 4N HCl/dioxane (10 mL) and stirred at room temperature for 4 h. The resulting suspension was concentrated *in vacuo* and basified with aq. NaHCO₃ soln. Fmoc-OSu (2.2g, 7.0 mmol) and THF (30 mL) was then added. The reaction mixture was stirred at room temperature for 3 h before it was acidified with aq. IN HCl and extracted with EtOAC (3x). The combined organic extracts were washed with brine, dried over sodium sulfate and concentrated *in vacuo.* The residue was purfied by flash column chromatography (ISCO, 0-10% MeOH/DCM, 80 g column) to give the desired product as awhite foam. MS(ESI⁺) *m*/*z* 394.1 (M + H)⁺.

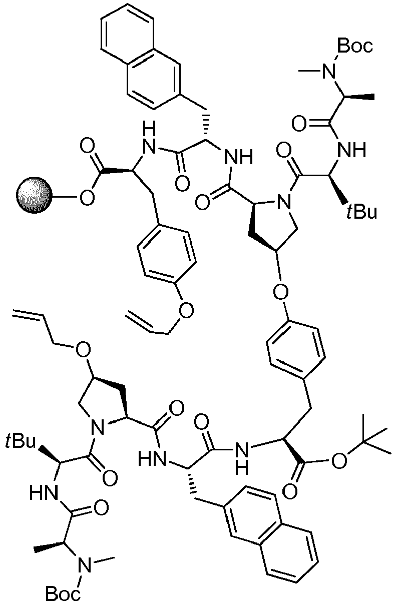

### E) Compound E

The resin-supported Fmoc-protected peptide **B** (0.10 mmol) was sequentially deprotected and coupled with (*S*)-2-((((9*H*-fluoren-9-yl)methoxy)carbonyl)amino)-3-(naphthalen-2-yl)propanoic acid (0.4 mmol, 1 h), Fmoc-protected acid **D** (0.4 mmol, 1h), followed by (*S*)-2-((((9*H*-fluoren-9-yl)methoxy)carbonyl)amino)-3,3-dimethylbutanoic acid (0.4 mmol, 3 h), and then (*S*)-2-((*tert*-butoxycarbonyl)(methyl)amino)propanoic acid (0.4 mmol, 1 h) to give the resin-supported product.

LC-MS analysis was performed on peptide cleaved from the resin using the following protocol: analytical amount of the resin was treated with TFA (1% solution in DCM, 0.2 mL) at room temperature for 1 min and then filtered through a syringe filter to give a solution of the free peptide. MS(ESI⁺) *m*/*z* 1698.6 (M + H)⁺.

### F) Example 84

Following a procedure analogous to that for the synthesis of **Compound N of Example 1**, the linear peptide on resin **E** (0.10 mmol) were converted to the title compound. MS(ESI⁺) *m*/*z* 1414.0 (M + H)⁺.

### EXAMPLE 85

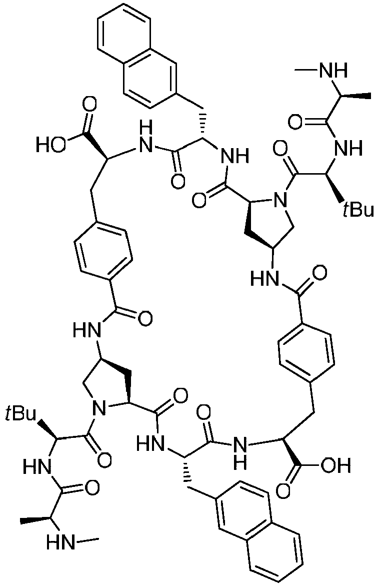

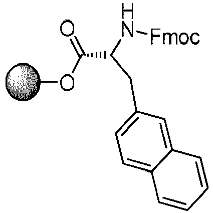

### A) Compound A

A solution of (*S*)-2-((((9*H*-fluoren-9-yl)methoxy)carbonyl)amino)-3-(naphthalen-2-yl)propanoic acid (0.5 mmol, 223 mg) and DIPEA (5 mmol, 0.87 mL) in DCM (10 mL) was added to 2-chlorotrityl resin (1.58 mmol/g, 1.58 mmol, 1.0 g) in a Biorad preparative column. The resin was rocked for 2 h and MeOH (4 mL) was added followed by rocking for an additional 1 h. The solvent was removed by filtration, and the solid resin was washed with DMF (3 x 10 mL) and then DCM (3 x 10 mL). The resulting resin was then dried uner N₂ overnight to give the resin-supported product.

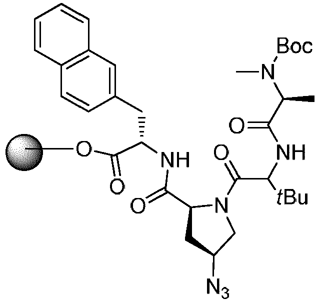

### B) Compound B

The resin-supported Fmoc-protected peptide **A** (0.25 mmol) was sequentially deprotected and coupled with (2*S*,4*S*)-1-(((9*H*-fluoren-9-yl)methoxy)carbonyl)-4-azidopyrrolidine-2-carboxylic acid (1 mmol, 1 h), followed by (*S*)-2-((((9*H*-fluoren-9-yl)methoxy)carbonyl)amino)-3,3-dimethylbutanoic acid (1 mmol, 3 h), and then (*S*)-2-((*tert*-butoxycarbonyl)(methyl)amino)propanoic acid (1 mmol, 1 h) to give the resin-supported product.

LC-MS analysis was performed on peptide cleaved from the resin using the following protocol: analytical amount of the resin was treated with TFA (1% solution in DCM, 0.2 mL) at room temperature for 1 min and then filtered through a syringe filter to give a solution of the free peptide. MS(ESI⁺) *m*/*z* 652.5 (M + H)⁺.

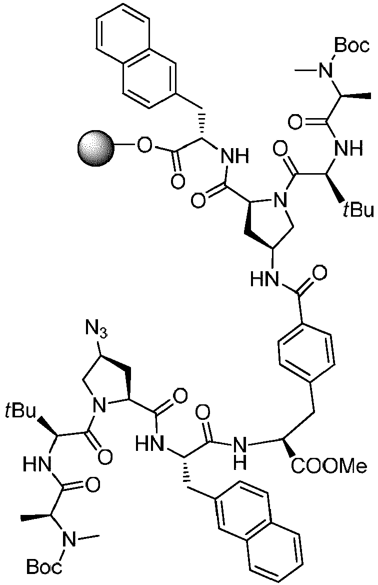

### C) Compound C

Resin **B** (0.25 mmol) was swelled with THF/H₂O ( 10:1, 5mL) in a Biorad preparative column. A solution of trimethylphosphine (0.5 mL, 1M in toluene) was then added and the resin was rocked for 1 h. The reagents were then drained by filtration and the resulting solid resin was washed with THF (2 x 10 mL) and then DCM ( 3 x 10 mL).

The resulting resin was then sequentially deprotected and coupled with Fmoc protected acid /**Compound C of Example 28**, (*S*)-2-((((9*H*-fluoren-9-yl)methoxy)carbonyl)amino)-3-(naphthalen-2-yl)propanoic acid (1 mmol, 1 h), (2*S*,4*S*)-1-(((9H-fluoren-9-yl)methoxy)carbonyl)-4-azidopyrrolidine-2-carboxylic acid (1 mmol, 1 h), followed by (*S*)-2-((((9*H*-fluoren-9-yl)methoxy)carbonyl)amino)-3,3-dimethylbutanoic acid (1 mmol, 3 h), and then (*S*)-2-((*tert-*butoxycarbonyl)(methyl)amino)propanoic acid (1 mmol, 1 h) to give the resin-supported product.

LC-MS analysis was performed on peptide cleaved from the resin using the following protocol: analytical amount of the resin was treated with TFA (1% solution in DCM, 0.2 mL) at room temperature for 1 min and then filtered through a syringe filter to give a solution of the free peptide. MS(ESI⁺) *m*/*z* 1464.8 (M + H)⁺.

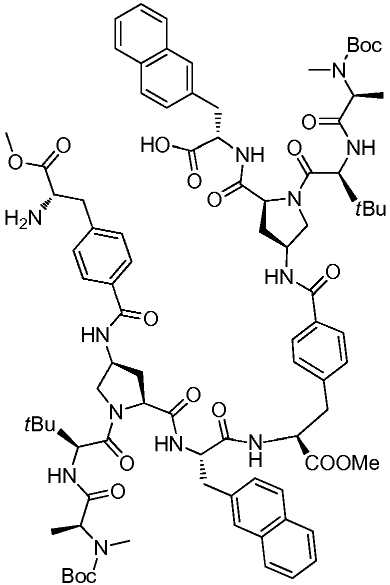

### D) Compound D

Resin C (0.25 mmol) was swelled with THF/H₂O (10:1, 5 mL) in a Biorad preparative column. A solution of trimethylphosphine (0.5 mL, 1M in toluene) was then added and the resin was rocked for 1 h. The reagents were then drained by filtration and the resulting solid resin was washed with THF (2 x 10 mL) and then DCM (3 x 10 mL).

To the resulting peptide on resin was added Fmoc protected acid / **Compoud C of Example 28** (0.2 M solution in DMF, 5 mL, 1 mmol), HATU (0.4 M solution in DMF, 2.5 mL, 1 mmol), and *N*-methyl morpholine (0.4 M solution in DMF, 2.5 mL, 1 mmol). The reaction mixture was rocked for 12 h at room temperature. The reagents were drained from the reaction vessel and the resin was washed three times with DMF.

The resin was then washed twice with a solution of 20% piperidine in DMF (5 mL and 2.5 minutes per wash) and mixing with a gentle stream of N₂ every 30 seconds. The resin was washed three times with DMF (8 mL and 1.5 min per wash) and three times with CH₂Cl₂ (8 mL and 30 seconds per wash), and then with a mixture of CH₂Cl₂:AcOH: CF₃CH₂OH (3:1:1, 15 mL) The reaction mixture was rocked for 2 h. The AcOH washings were combined and the solvents were removed *in vacuo.* MS(ESI⁺) *m*/*z* 1643.9 (M + H)⁺.

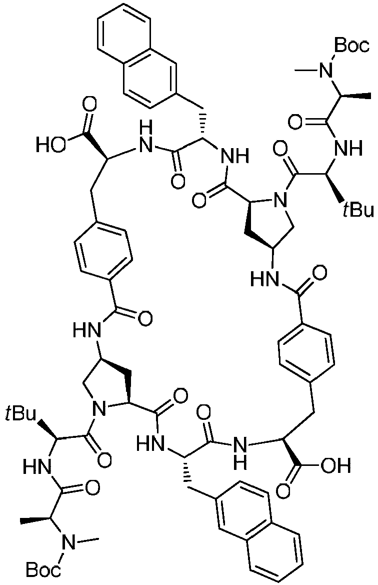

### E) Compound E

To a solution of peptide **D** (30 mg, 0.018 mmol) in acetonitrile (20 mL) was added DIEA (0.032 mL, 0.182 mmol) and HATU (17.4 mg, 0.046 mmol). The reaction was stirred at room temperature for 3 h and then concentrated *in vacuo.* The residue was dissolved in THF (3 mL) and aq. LiOH (1M, 0.5 mL) was added to the reaction. After 1h, the reaction mixture was concentrated *in vacuo* and purified by preparative HPLC to give the title compound (19 mg, 65%) as a white solid after lyophilization. MS(ESI⁺) *m*/*z* 1598.4 (M + H)⁺.

### F) Example 85

To a suspension of peptide **E** (4 mg, 0.0025 mmol) in DCM (1 mL) was added TFA (0.25 mL). The resulting solution was stirred at room temperature for 1 h before it was concentrated *in vacuo* and purified by preparative HPLC to give the title compound (2.9 mg, 64%) as a white solid after lyophlization. MS(ESI⁺) *m*/*z* 1398.4 (M + H)⁺.

### EXAMPLE 86

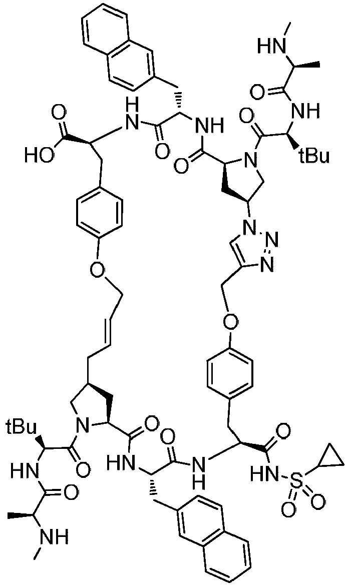

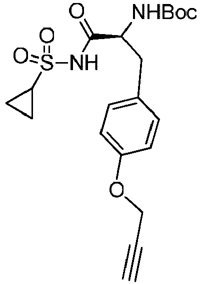

### A) (S)-tert-Butyl (1-(cyclopropanesulfonamido)-1-oxo-3-(4-(prop-2-yn-1-yloxy)phenyl)propan-2-yl)carbamate

To a solution of (*S*)-3-(4-(allyloxy)phenyl)-2-((*tert*-butoxycarbonyl)amino) propanoic acid (2.48, 7.8 mmol) in THF (30 mL) was added a solution of CDI (1.39 g, 8.5 mmol) in DCM (18 mL). The resulting solution was stirred at room temperature for 1 h. A solution of cyclopropanesulfonamide (Aldrich, 1.41 g, 11.7 mmol) in THF (5 mL) was added, followed by dropwise addition of DBU (1.76 mL, 11.7 mmol). The reaction was stirred at room temperature for 15 min before it was quenched with aq. HCl (0.5 N, 5 mL) and extracted with DCM (3x). The combined organic extracts were washed with brine, dried over sodium sulfate and concentrated *in vacuo.* The resulting oil was purified using flash column chromatography (ISCO, 0-10% MeOH/DCM) to give the title compound (2.3 g, 69%) as a colorless oil. MS(ESI⁺) *m*/*z* 423.2 (M + H)⁺.

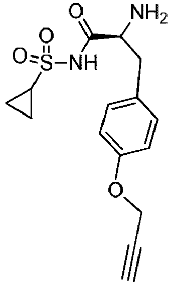

### B) (S)-2-Amino-N-(cyclopropylsulfonyl)-3-(4-(prop-2-yn-1-yloxy)phenyl)propanamide

To a solution of (*S*)-*tert*-butyl (1-(cyclopropanesulfonamido)-1-oxo-3-(4-(prop-2-yn-l-yloxy)phenyl)propan-2-yl)carbamate (850 mg, 2.0 mmol) in EtOAc (2 mL) was added 4N HCl in dioxane (10 mL). The resulting reaction was stirred at room temperature for 4 h and then concentrated *in vacuo* to give the title compound (720 mg, 99%) as a HCl salt. MS(ESI⁺) *m*/*z* 323.2 (M + H)⁺.

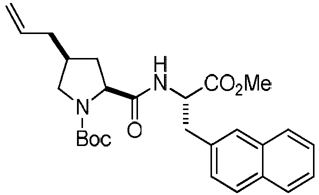

### C) (2S,4S)-tert-Butyl 4-allyl-2-(((S)-1-methoxy-3-(naphthalen-2-yl)-1-oxopropan-2-yl)carbamoyl)pyrrolidine-1-carboxylate

To a solution of (2*S*,4*S*)-4-allyl-1-(*tert*-butoxycarbonyl)pyrrolidine-2-carboxylic acid (**Compound C of Example 1,** 5.11 g, 20.0 mmol) in DMF (50 mL) at 0 °C were added EDC (Advanced Chem Tech, 4.99 g, 26.0 mmol), HOAt (4.01 g, 26.0 mmol) and 4-methylmorpholine (6.07 g, 60.0 mmol). The reaction mixture was stirred at 0 °C for 20 min. A solution of (*S*)-methyl 2-amino-3-(naphthalen-2-yl)propanoate (Chem-Impex Int'l Inc , 5.05 g, 22.02 mmol) in 10 mL of DMF was added. The reaction mixture was stirred at room temperature overnight and quenched with H₂O. The solid that formed was collected by filtration and purified by flash column chromatography (gradient elution from 0 - 50% EtOAc in DCM) to provide the title compound (7.20 g, 77%) as a light yellow foaming solid. MS(ESI⁺) *m*/*z* 467.4 (M + H)⁺.

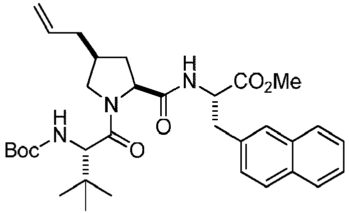

### D) (S)-Methyl 2-((2S,4S)-4-allyl-1-((S)-2-((tert-butoxycarbonyl)amino)-3,3-dimethylbutanoyl)pyrrolidine-2-carboxamido)-3-(naphthalen-2-yl)propanoate

To a solution of (2*S*,4*S*)-*tert*-butyl 4-allyl-2-(((*S*)-1-methoxy-3-(naphthalen-2-yl)-1-oxopropan-2-yl)carbamoyl)pyrrolidine-1-carboxylate (7.2 g, 15.4 mmol) in DCM (40 mL) at room temperature was added TFA (10 mL). The reaction mixture was stirred at room temperature for 3 h and concentrated *in vacuo.* The residue was dissolved in DCM (150 ml) and washed with sat. NaHCO₃ to pH ~8. The organic layer was washed with brine, dried over MgSO₄ and concentrated *in vacuo* to give the amine (5.66 g, 100%) as a light yellow solid. MS(ESI⁺) *m*/*z* 367.3 (M + H)⁺.

To a solution of the above compound (5.66 g, 15.5 mmol), (*S*)-2-((*tert-*butoxycarbonyl)amino)-3,3-dimethylbutanoic acid (3.93 g, 17.0 mmol), EDC (3.55 g, 18.5 mmol) and HOAt (2.52 g, 18.5 mmol) in DMF (50 mL) at 0 °C was added NMM (5.09 mL, 46.3 mmol). The reaction mixture was stirred and 0 °C for 30 min, gradually warmed up to room temperature and stirred at room temperature overnight. The reaction was quenched by the addition of cold water (-200 mL). The solid that formed was collected by filtration and purified with flash column chromatography (gradient elution from 0 - 60% EtOAc in hexane) to provide the title compound (7.10 g, 79%) as a light yellow solid. MS(ESI⁺) *m*/*z* 580.5 (M + H)⁺.

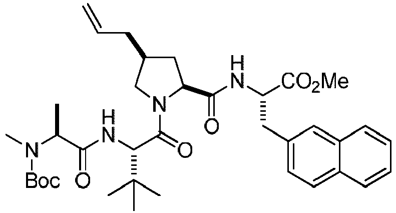

### E) (S)-Methyl2-((2S,4S)-4-allyl-1-((S)-2-((S)-2-((tert-butoxycarbonyl) (methyl)amino)propanamido)-3,3-dimethylbutanoyl)pyrrolidine-2-carboxamido)-3-(naphthalen-2-yl)propanoate

To a solution of (*S*)-methyl 2-((2*S*,4*S*)-4-allyl-1-((*S*)-2-((*tert*-butoxycarbonyl) amino) -3,3-dimethylbutanoyl)pyrrolidine-2-carboxamido)-3-(naphthalen-2-yl)propanoate (7.10 g, 12.3 mmol) in DCM (30 mL) was added TFA (12 mL) dropwise. The reaction mixture was stirred at room temperature for 2 h and concentrated *in vacuo.* The residue was dissolved in DCM (200 mL) and washed with sat. NaHCO₃ solution, and brine. The organic layer was dried over MgSO₄ and concentrated *in vacuo* to give the free amine (5.76 g, 98%) as a white solid. MS(ESI⁺) *m*/*z* 480.4 (M + H)⁺.

To a solution of (*S*)-2-((*tert*-butoxycarbonyl)(methyl)amino)propanoic acid (2.68 g, 13.2 mmol) in DMF at 0 °C were added EDC (2.76 g, 14.4 mmol), HOAt (1.96 g, 14.4 mmol) and 4-methylmorpholine (3.96 mL, 36.0 mmol). The reaction mixture was stirred and 0 °C for 20 min. The solution of the above amine (5.76 g, 12.01 mmol) in 5 mL of DMF with 1 eq of NMM was added dropwise. The reaction mixture was stirred at room temperature for 1 h. The reaction was quenched by adding cold water (-200 mL). The solid that formed was collected by filtration and purified with flash column chromatography (gradient elution from 0 - 3% MeOH in DCM) to provide the title compound (5.07 g, 64%) as a white solid. MS(ESI⁺) *m*/*z* 665.5 (M + H)⁺.

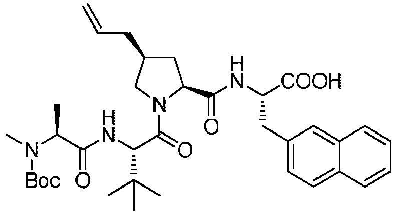

### F) (S)-2-((2S,4S)-4-Allyl-1-((S)-2-((S)-2-((tert-butoxycarbonyl)(methyl)amino) propanamido)-3,3-dimethylbutanoyl)pyrrolidine-2-carboxamido)-3-(naphthalen-2-yl)propanoic acid

To a solution of (2*S*,4*S*)-1-*tert*-butyl 2-methyl 4-allylpyrrolidine-1,2-dicarboxylate (4.5 g, 6.77 mmol) in THF (20 mL) and MeOH (5 mL) at room temperature was added aq. LiOH (1M, 20.3 mL, 20.3 mmol). The resulting mixture was stirred at room temperature for 5 h before it was cooled to 0 °C, acidified with 1 N HCl to pH 3-4 and extracted with DCM (3x). The combined organic layers were dried over MgSO₄ and concentrated *in vacuo* to give the title compound (5.11 g, 98%) as a white solid. MS(ESI⁺) *m*/*z* 651.4 (M + H)⁺.

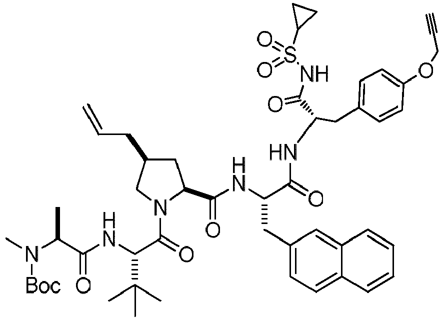

### G) tert-Butyl ((S)-1-(((S)-1-((2S,4S)-4-allyl-2-(((S)-1-(((S)-1-(cyclopropanesulfonamido) -1-oxo-3-(4-(prop-2-yn-1-yloxy)phenyl)propan-2-yl)amino)-3-(naphthalen-2-yl)-1-oxopropan-2-yl)carbamoyl)pyrrolidin-1-yl)-3,3-dimethyl-1-oxobutan-2-yl)amino)-1-oxopropan-2-yl)(methyl)carbamate

To a solution of (*S*)-2-((2*S*,4*S*)-4-allyl-1-((*S*)-2-((*S*)-2-((*tert*-butoxycarbonyl) (methyl)amino)propanamido)-3,3-dimethylbutanoyl)pyrrolidine-2-carboxamido)-3-(naphthalen-2-yl)propanoic acid (1.71 g, 2.61 mmol) in DMF (20 mL) was added HATU (0.99 g, 2.61 mmol). The resulting reaction was stirred at room temperature for 5 min before a solution of of (*S*)-2-amino-*N*-(cyclopropylsulfonyl)-3-(4-(prop-2-yn-1-yloxy)phenyl)propanamide, HCl (compound **B**, 0.98 g, 2.74 mmol) and NMM (1.06 g, 10.5 mmol) in DMF (5 mL) was added. After stirring at room temperature for 3 h, the reaction was quenched with aq. LiCl soln. and extracted with EtOAc (3x). The combined organic layers were washed with sat. NaHCO₃ soln., followed by brine, dried over sodium sulfate, and concentrated *in vacuo* to give the title compound (1.9 g, 76%) as a white foam. MS(ESI⁺) *m*/*z* 955.3 (M + H)⁺.

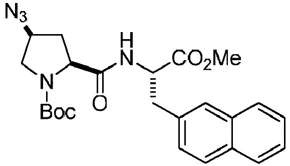

### H) (2S,4S)-tert-Butyl 4-azido-2-(((S)-1-methoxy-3-(naphthalen-2-yl)-1-oxopropan-2-yl)carbamoyl)pyrrolidine-1-carboxylate.

To a 0 °C solution of (2*S*,4*S*)-4-azido-1-(*tert*-butoxycarbonyl)pyrrolidine-2-carboxylic acid (39.8 mmol, 10.2 g) in CH₂Cl₂ (379 mL) was added EDC (45.5 mmol, 8.72 g) followed by HOAt (45.5 mmol, 6.19 g). After 0.5 h, NMM (114 mmol, 12.5 mL) and (*S*)-methyl 2-amino-3-(naphthalen-2-yl)propanoate (37.9 mmol, 8.69 g) were added to the mixture and the resulting reaction was stirred while warming to room temperature overnight. The reaction mixture was poured into EtOAc, washed with sat. aq. NaHCO₃ soln., and the aqueous layer was extracted EtOAc (3x). The combined organic extracts were washed with IN HCl and brine. The organic extracts were dried over Na₂SO₄, filtered and concentrated *in vacuo* to give an oil. The residue was purified by silica gel chromatography (ISCO, 20% -100% EtOAc in hexane) to afford the title compound (14.3 g, 81%) as an oil. MS (ESI+) *m*/*z* 468.4(M + H)⁺.

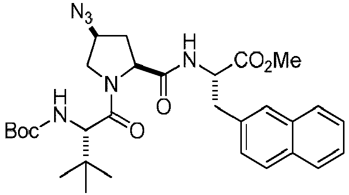

### I) (S)-Methyl2-((2S,4S)-4-azido-1-((S)-2-((tert-butoxycarbonyl)amino)-3,3-dimethylbutanoyl)pyrrolidine-2-carboxamido)-3-(naphthalen-2-yl)propanoate.

To compound **H** (30.7 mmol, 14.3 g) in CH₂Cl₂ (150 mL) was added TFA (50 mL). After 2 h, the reaction mixture was concentrated *in vacuo* and the residue was azeotroped with toluene (2x) to provide the crude free amine.

To a solution of (*S*)-2-((*tert*-butoxycarbonyl)amino)-3,3-dimethylbutanoic acid (32.2 mmol, 7.44 g) in CH₂Cl₂ (306 mL) at 0 °C was added EDC (36.8 mmol, 7.05 g) followed by HOAt (36.8 mmol, 5.01 g). After 0.5 h, a solution of *N*-methylmorpholine (92 mmol, 10.1 mL) and the crude free amine was added to the initial reaction mixture. The resulting solution was stirred while warming to room tempearture overnight. The reaction mixture was poured into CH₂Cl₂ and washed with sat. aq. NaHCO₃ soln. The aqueous layer was extracted with EtOAc (3x) and the combined organic extracts were washed with IN HCl and brine. The organic extracts were dried over Na₂SO₄, filtered and concentrated *in vacuo.* The crude oil was purified by silica gel chromatography (ISCO, 0-100% hexanes in EtOAc) to afford the title compound (16.3 g, 91%) as a white foam. MS (ESI+) rt 1.12 min, *m*/*z* 581.4 (M + H)⁺.

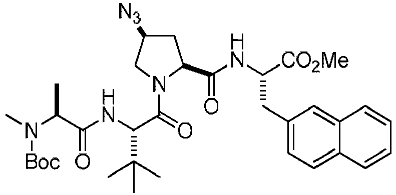

### J) (S)-Methyl 2-((2S,4S)-4-azido-1-((S)-2-((S)-2-((tert-butoxycarbonyl)(methyl)amino)-propanamido)-3,3-dimethylbutanoyl)pyrrolidine-2-carboxamido)-3-(naphthalen-2-yl)propanoate.

To compound **I** (28 mmol, 16.3 g) in CH₂Cl₂ (150 mL) was added TFA (50 mL). After stirring the reaction mixture for 1 h, the solvent was removed *in vacuo*, and the resulting residue was taken up in CH₂Cl₂. The organic layer was washed with sat. aq. NaHCO₃ soln. and then 2 N HCl. The organics were dried over Na₂SO₄, filtered and concentrated *in vacuo* to give the free amine.

To a solution of (*S*)-2-((*tert*-butoxycarbonyl)(methyl)amino)propanoic acid (29.4 mmol, 5.98 g) in CH₂Cl₂ (280 mL) at 0 °C was added EDC (33.6 mmol, 6.45 g) followed by HOAt (33.6 mmol, 4.58 g). After 30 min, a solution of NMM (84 mmol, 9.24 ml) and the free amine was added and the resulting reaction mixture was stirred overnight while warming to room temperature. The mixture was then poured into CH₂Cl₂ and sat. aq. NaHCO₃ soln. The aqueous layer was extracted with EtOAc (3x). The combined organic extracts were washed with IN HCl and brine, dried over Na₂SO₄, filtered and concentrated *in vacuo* to give an oil. The crude residue was purified by silica gel chromatography (ISCO, 0-100% EtOAc/hexane) to afford the title compound (16.6 g, 89%) as a white foam. MS (ESI+) *m*/*z* 666.4.

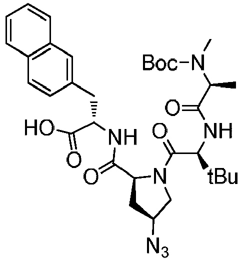

### K) (S)-2-((2S,4S)-4-Azido-1-((S)-2-((S)-2-((tert-butoxycarbonyl)(methyl)amino) propanamido) -3,3-dimethylbutanoyl)pyrrolidine-2-carboxamido)-3-(naphthalen-2-yl)propanoic acid

To a solution of compound **J** (24.9 mmol 16.6 g) in THF (22.6 mL) / MeOH (22.6 mL) was added 2M aq. LiOH (62.5 mmol, 31.2 mL). The resulting reaction mixture was stirred at room tempearture until LC-MS indicated full conversion. The reaction mixture was then acidified with IN HCl and the solution was extracted with CH₂Cl₂ (3x). The combined organic extracts were dried over Na₂SO₄, filtered, and concentrated *in vacuo* to give the title compound (16.04 g, 89%) as a white solid. MS (ESI+) rt 1.03 min, *m*/*z* 652.4. ¹H NMR (400 MHz, DMSO-*d₆*) δ 8.10 (d, *J* = 7.5 Hz, 1H), 7.92 - 7.69 (m, 3H), 7.56 - 7.28 (m, 3H), 4.68 - 4.52 (m, 2H), 4.49 - 4.21 (m, 2H), 4.07 (dd, *J* = 10.6, 6.6 Hz, 1H), 3.64 - 3.50 (m, 2H), 3.39 (dd, *J* = 10.6, 6.2 Hz, 1H), 3.22 - 3.01 (m, 2H), 2.81 - 2.63 (m, 3H), 2.45 - 2.33 (m, 1H), 1.89 - 1.65 (m, 2H), 1.48 - 1.29 (m, 9H), 1.20 (d, *J* = 6.8 Hz, 3H), 0.90 (s, 9H).

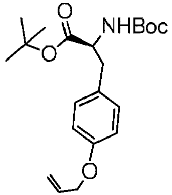

### L) (S)-tert-Butyl 3-(4-(allyloxy)phenyl)-2-((tert-butoxycarbonyl)amino) propanoate

To a solution of ((*S*)-*tert*-butyl 2-((*tert*-butoxycarbonyl)amino)-3-(4-hydroxyphenyl)propanoate (14.0 g, 41.5 mmol) (A ChemTek, 14 g, 41.5 mmol) in DMF (100 mL) was added potassium carbonate (8.6 g, 62 mmol) and allyl bromide (Aldrich, 5.4 mL, 62.2 mmol). The reaction mixture was heated to 70 °C for 5 h before it was cooled to room temperature and diluted with EtOAc and H₂O. The resulting mixture was extracted with EtOAc (3x) and the combined organic layers were washed with water, and then brine, dried over sodium sulfate, and concentrated *in vacuo.* The resulting residue was purified by flash column chromatography (gradient elution from 0 - 10% acetone in hexane) to afford the desired product (13.5 g, 86%). MS(ESI⁺) *m*/*z* 378.3 (M + H)⁺.

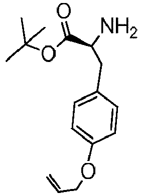

### M) (S)-tert-Butyl 3-(4-(allyloxy)phenyl)-2-aminopropanoate

To a solution of (*S*)-*tert*-butyl 3-(4-(allyloxy)phenyl)-2-((*tert*-butoxycarbonyl) amino)propanoate (3.77 g, 10.0 mmol) in EtOAc (5 mL) was added HCl in ether (2 N, 30 mL). The resulting reaction mixture was stirred at room temperature for 24 h. The product (2.57 g, 82%) was isolated by vaccum filtration on a frit and dried under vaccum overnight. MS(ESI⁺) *m*/*z* 278.3 (M + H)⁺.

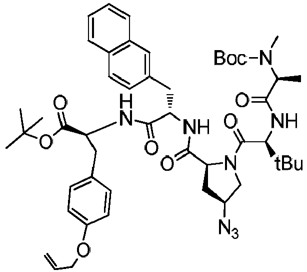

### N) (S)-tert-Butyl 3-(4-(allyloxy)phenyl)-2-((S)-2-((2S,4S)-4-azido-1-((S)-2-((S)-2-((tert-butoxycarbonyl) (methyl)amino)propanamido)-3,3-dimethylbutanoyl) pyrrolidine-2-carboxamido)-3-(naphthalen-2-yl)propanamido)propanoate

To a solution of compound **K** (1.0 g, 1.5 mmol) in DMF (15 mL) was added sequentially HATU (0.64 g, 1.69 mmol), compound L (0.51 g, 1.61 mmol), and NMM (1.07 g, 6.1 mmol). After stirring the mixture at room temperature for 12 h, the reaction was quenched with aq. LiCl and extracted with EtOAc (3x). The combined organic extracts were washed with sat. NaHCO₃ soln., followed by brine, dried over sodium sulfate, and concentrated *in vacuo* to give the title compound (1.4 g, 98%) as a white foam. MS(ESI⁺) *m*/*z* 911.6 (M + H)⁺.

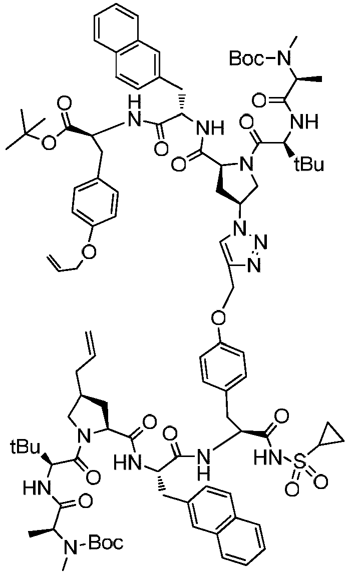

### O) (S)-tert-Butyl 2-((S)-2-((2S,4S)-4-(4-((4-((S)-2-((S)-2-((2S,4S)-4-allyl-1-((S)-2-((S)-2-((tert-butoxycarbonyl)(methyl)amino)propanamido)-3,3-dimethylbutanoyl)pyrrolidine-2-carboxamido)-3-(naphthalen-2-yl)propanamido)-3-(cyclopropanesulfonamido)-3-oxopropyl)phenoxy)methyl)-1H-1,2,3-triazol-1-yl)-1-((S)-2-((S)-2-((tert-butoxycarbonyl)(methyl)amino)propanamido)-3,3-dimethylbutanoyl)pyrrolidine-2-carboxamido)-3-(naphthalen-2-yl)propanamido)-3-(4-(allyloxy)phenyl)propanoate

To a solution of compound **N** (1.15 g, 1.26 mmol) and compound **F** (1.2 g, 1.26 mmol) in THF/t-BuOH/H₂O (1:1:1, 3 mL) was added a solution of sodium ascorbate (Aldrich, 0.1 g, 0.5 mmol) in H₂O (0.15 mL) and a solution of copper sulfate pentahydrate (Aldrich, 0.016 g, 0.063 mmol) in H₂O (0.15 mL). The resulting solution was stirred at room temperature overnight and extracted with EtOAc (3 x). The combined organic layers were washed with aq. NH₄Cl soln., dried over sodium sulfate, and concentrated *in vacuo* to give the title compound (1.6 g, 68%) as a white foam. MS(ESI⁺) *m*/*z* 1867.3 (M + H)⁺.

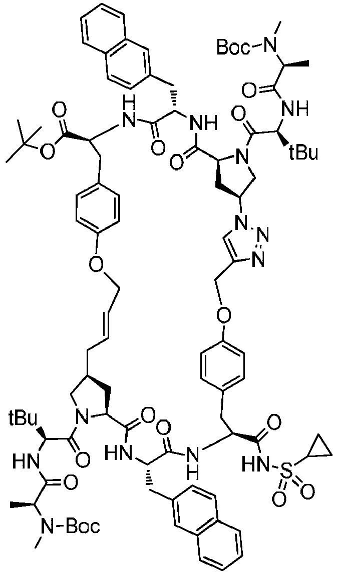

### P) Compound P

To a solution of compound **O** (1.3g, 0.70 mmol) in DCE (200 mL) was added a solution of Grubbs-Hoveyda II catalyst (Aldrich, 44 mg, 0.070 mmol) in DCE (1 mL). The resulting reaction mixture was purged with N₂ for 5 min and heated to 70 °C for 8 h. The reaction mixture was then cooled to room temperature and concentrated *in vacuo.* The crude oil was purified using preparative HPLC to give the title compound (960 mg, 75%) as a white solid after lyophilization. MS(ESI⁺) *m*/*z* 1839.1 (M + H)⁺.

### Q) Example 86

To a solution of compound **P** (0.53 g, 0.29 mmol) in DCM ( 10 mL) was added TFA (10 mL). The resulting reaction mixture was stirred at room temperature for 12 h and then concentrated *in vacuo.* The crude oil was purified using preparative HPLC to give the title compound (370 mg, 73%) as a white solid after lyophilization. MS(ESI⁺) *m*/*z* 1582.8 (M + H)⁺.

### EXAMPLE 87

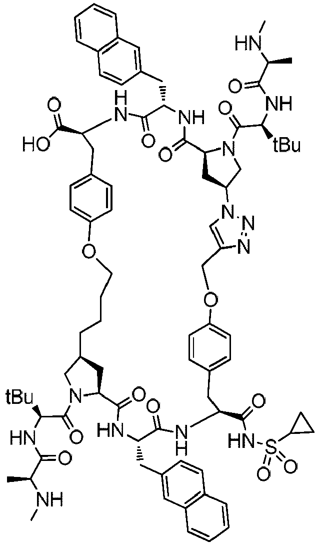

To Pd/C (10%, Aldrich, 80 mg) under N₂ was added a solution of **Example 86** (200 mg, 0.13 mmol) in MeOH (20 mL). The resulting suspension was stirred under H₂ (50 psi) for 48 h before the reaction was purged with N₂ and filtered through a pad of Celite^{®}. The filtrate was concentrated and purfied using preparative HPLC to give the TFA salt of the title compound as a white solid after lyophilization. The TFA salt was then dissolved in THF/H₂O (1:2, 2 mL) and treated with aq. HCl (IN, 0.1 mL). The resulting solution was lyophilized to give the HCl salt of the title compound (97 mg, 44%) as a white solid. MS(ESI⁺) *m*/*z* 1585.4 (M + H)⁺.

### EXAMPLE 88

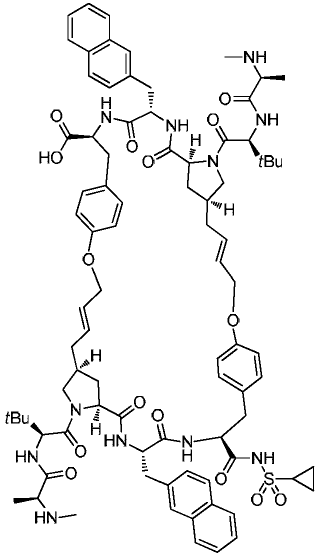

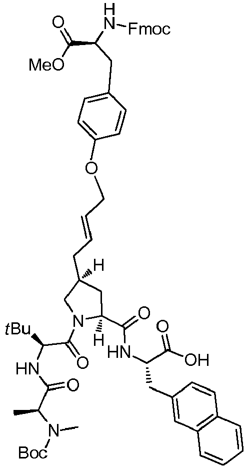

### A) (S)-2-((2S,4S)-4-((E)-4-(4-((S)-2-((((9H-Fluoren-9-yl)methoxy)carbonyl)amino)-3-methoxy-3-oxopropyl)phenoxy)but-2-en-1-yl)-1-((S)-2-((S)-2-((tert-butoxycarbonyl)(methyl)amino)propanamido)-3,3-dimethylbutanoyl)pyrrolidine-2-carboxamido)-3-(naphthalen-2-yl)propanoic acid

To a solution of **Compound F of Example 86** (120 mg, 0.18 mmol) and (*S*)-methyl 2-((((9*H-*fluoren-9-yl)methoxy)carbonyl)amino)-3-(4-(allyloxy)phenyl)propanoate (253 mg, 0.55 mmol) in DCE (3 mL) was added a solution of Crrubbs II catalyst (15.7 mg, 0.018 mmol) in DCE (0.3 mL). The resulting reaction mixture was purged with N₂ for 5 min and heated to 70 °C for 3 h. The reaction mixture was then cooled to room temperature and concentrated *in vacuo.* The residue was purified by reverse phase column chromatography (ISCO 55-100% acetonitrile/H₂O, 0.1 % TFA) to give the title compound (91 mg, 46%) as a white solid after lyophilization. MS(ESI⁺) *m*/*z* 1080.7 (M + H)⁺.

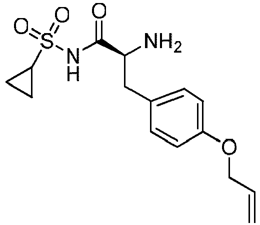

### B) (S)-3-(4-(Allyloxy)phenyl)-2-amino-N-(cyclopropylsulfonyl)propanamide

To a solution of (*S*)-3-(4-(allyloxy)phenyl)-2-((tert-butoxycarbonyl)amino) propanoic acid (Chem-Impex Int' Inc, 0.96g, 3 mmol) in THF (10 mL) was added a solution of CDI (0.58 g, 3.6 mmol) in DCM (10 mL). The resulting solution was stirred at room temperature for 1 h. A solution of cyclopropanesulfonamide (0.44 g, 3.6 mmol) in THF (5 mL) was added, followed by the dropwise addition of DBU (0.55 g, 3.6 mmol). The reaction was stirred at room temperature for 15 min before being quenched with IN HCl (10 mL). The mixture was extracted with DCM (3x) and the combined organic layers were washed with brine, dried over sodium sulfate and concentrated *in vacuo.* The resulting oil was purified on ISCO (0-10% MeOH/DCM) to give the *N*-Boc protected intermediate. ¹H NMR (CDCl₃) δ 5.96 - 5.60 (m, 1H), 5.28 - 5.00 (m, 2H), 4.60 (dd, *J* = 8.8, 7.0 Hz, 1H), 3.79 (s, 3H), 2.92 - 2.66 (m, 1H), 2.62 - 2.44 (m, 2H), 2.34 - 2.12 (m, 1H), 1.68 (ddd, *J* = 13.1, 8.2, 7.3 Hz, 1H), 1.48 (s, 9H).

The acylsulfonamide intermediate was dissolved in 4N HCl in dioxane (10 mL) and stirred at room temperature for 2 h. The resulting suspension was then concentrated *in vacuo* to give the title compound as a HCl salt. MS(ESI⁺) *m*/*z* 325.2 (M + H)⁺.

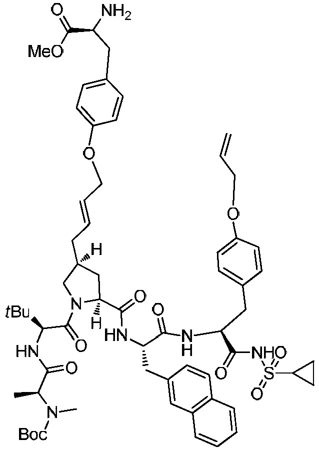

### C) (S)-Methyl 3-(4-(((E)-4-((3S,5S)-5-(((S)-1-(((S)-3-(4-(allyloxy)phenyl)-1-(cyclopropanesulfonamido)-1-oxopropan-2-yl)amino)-3-(naphthalen-2-yl)-1-oxopropan-2-yl)carbamoyl)-1-((S)-2-((S)-2-((tert-butoxycarbonyl)(methyl) amino)propanamido)-3,3-dimethylbutanoyl)pyrrolidin-3-yl)but-2-en-1-yl)oxy)phenyl)-2-aminopropanoate

To a solution of peptide **A** (85 mg, 0.079 mmol) in DMF (3 mL) was added HATU (45 mg, 0.12 mmol), followed by a solution of DIEA (0.055 mL, 0.32 mmol) and amine **B** (50 mg, 0.12 mmol). The reaction was stirred at room temperature for 1 h before it was diluted with aq. LiCl (15 mL). The mixture was extracted with EtOAc (3x). The combined organic extracts were washed with brine, dried over sodium sulfate, and concentrated *in vacuo.* The residue was dissolved in DCM (8 mL) and piperidine (1.9 mL, 19.7 mmol) was added. After stirring the solution at room temperature for 2 h, the reaction mixture was concentrated *in vacuo* and the resulting residue was purified by reverse phase column chromatography to give the desired product (62 mg, 66%). MS(ESI⁺) *m*/*z* 1164.7 (M + H)⁺.

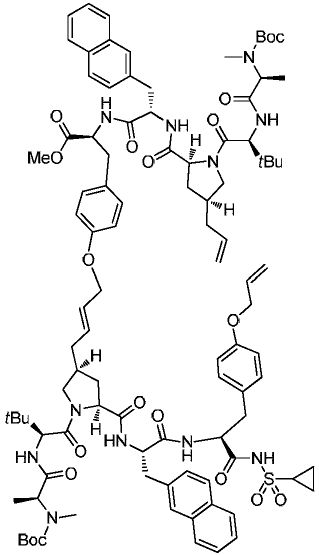

### D) (S)-Methyl 2-((S)-2-((2S,4S)-4-allyl-1-((S)-2-((S)-2-((tert-butoxycarbonyl)(methyl)amino)propanamido)-3,3-dimethylbutanoyl)pyrrolidine-2-carboxamido)-3-(naphthalen-2-yl)propanamido)-3-(4-(((E)-4-((3S,5S)-5-(((S)-1-(((S)-3-(4-(allyloxy)phenyl)-1-(cyclopropanesulfonamido)-1-oxopropan-2-yl)amino)-3-(naphthalen-2-yl)-1-oxopropan-2-yl)carbamoyl)-1-((S)-2-((S)-2-((tert-butoxycarbonyl)(methyl)amino)propanamido)-3,3-dimethylbutanoyl)pyrrolidin-3-yl)but-2-en-1-yl)oxy)phenyl)propanoate

To a solution of peptide C (40 mg, 0.062 mmol) and Compound F of Example 86 (60 mg, 0.052 mmol) in DMF (3 mL) was added HATU (29.4 mg, 0.077 mmol) and DIEA (0.045 mL, 0.26 mmol). The reaction mixture was stirred at room temperature for 1 h before it was diluted with aq. LiCl (10 mL) and extracted with EtOAc (3x). The combined organic layers were washed with sat. NaCl, dried over sodium sulfate and concentrated *in vacuo.* The residue was purified on ISCO to afford the desired product (40 mg,43%). MS(ESI⁺) *m*/*z* 1796.7 (M + H)⁺.

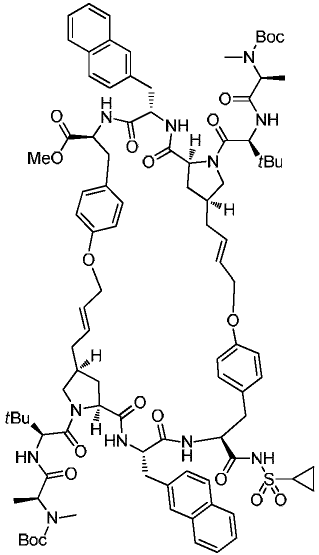

### E) Compound E

To a solution of Compound **D** (40 mg, 0.022 mmol) in DCE (20 mL) was added a solution of Crrubbs I catalyst (Aldrich, 1.9 mg, 0.002 mmol) in DCE (0.2 mL). The resulting reaction mixture was purged with N₂ for 5 min and heated to 55 °C for 3 h. A second batch of Crrubbs I catalyst (1.9 mg, 0.002 mmol) in DCE (0.2 mL) was then added and the reaction was stirred at 55 °C for 12 h. The reaction mixture was then cooled to room temperature and concentrated *in vacuo.* The crude oil was purified by reverse phase column chromatography to give the title compound (25 mg, 64%) as a white solid after lyophilization. MS(ESI⁺) *m*/*z* 1769.2 (M + H)⁺.

### F) Example 88

To a solution of **E** (25 mg, 0.014 mmol) in THF (4 mL) was added aq. LiOH (1 M, 1 mL). The resulting reaction mixture was stirred at room temperature for 1 h and then concentrated *in vacuo.* The resulting oil was acidified with IN aq.HCl and then extracted with EtOAc (3x). The combined organic extracts were dried over sodium sulfate and concentrated *in vacuo.* The resulting oil was dissolved in DCM (6 mL) and TFA (3 mL) was added. The reaction was stirred at room temperature for 1 h before it was concentrated *in vacuo.* The resulting residue was purified by preparative HPLC to give the TFA salt of the title compound (16 mg, 57%) as a white solid after lyophilization. MS(ESI⁺) *m*/*z* 1555.7 (M + H)⁺.

### EXAMPLE 89

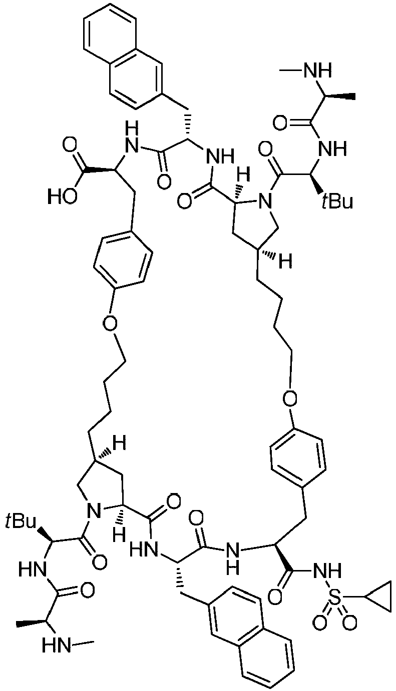

To Pd/C (10%, Aldrich, 10 mg) under N₂ was added a solution of **Compound F of Example 88** (23 mg, 0.015 mmol) in MeOH (15 mL). The resulting suspension was stirred under H₂ (50 psi) for 48 h before the reaction was purged with N₂ and filtered through a pad of Celite^{®}. The filtrate was concentrated and purfied by preparative HPLC to give the TFA salt of the title compound (13 mg, 49%) as a white solid after lyophilization. MS(ESI⁺) *m*/*z* 1560.2 (M + H)⁺.

### EXAMPLE 90

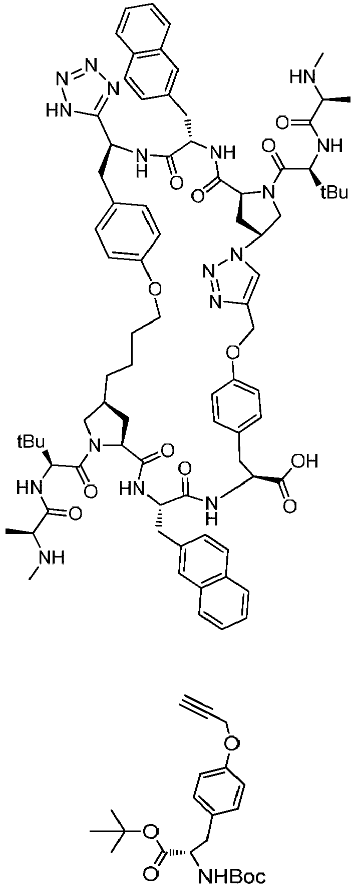

### A) (S)-tert-Butyl 2-((tert-butoxycarbonyl)amino)-3-(4-(prop-2-yn-1-yloxy)phenyl)propanoate

To a solution of (*S*)-*tert*-butyl 2-((*tert*-butoxycarbonyl)amino)-3-(4-hydroxyphenyl) propanoate (A Chem Tek, 10.0 g, 29.6 mmol) in DMF (100 mL) was added 3-bromoprop-1-yne (6.61 g, 44.5 mmol) and potassium carbonate (6.14 g, 44.5 mmol). The resulting suspension was stirred at 70 °C for 5 h. The reaction mixtue was then allowed to cool to room temperature, diluted with water (200 mL), and extracted with EtOAc (3x). The combined organic extracts were washed with brine, dried over sodium sulfate and concentrated *in vacuo.* The residue was purified by flash column chromatography (ISCO, 0-15% acetone/hexane) to give the title compound (9.2 g, 83%) as a colorless oil. MS(ESI⁺) *m*/*z* 398.3 (M + Na)⁺.

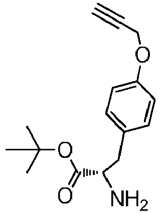

### B) (S)-tert-Butyl 2-amino-3-(4-(prop-2-yn-1-yloxy)phenyl)propanoate

To a solution of (*S*)-*tert*-butyl 2-((*tert*-butoxycarbonyl)amino)-3-(4-(prop-2-yn-1-yloxy)phenyl)propanoate (7 g, 18.6 mmol) in EtOAc (5 mL) was added HCl in diethylether (2 M, 50.0 mL, 100 mmol). The reaction was stirred at room tempearture for 24 h. The product (3.85 g, 75%) was isolated by vaccum filtration on a frit and dried under vaccum overnight. MS(ESI⁺) m/z 276.3 (M + H)⁺.

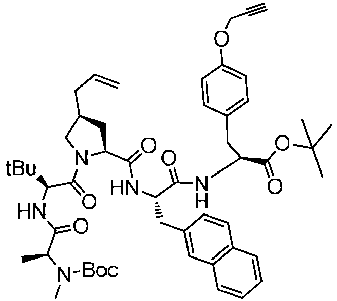

### C) (S)-tert-Butyl 2-((S)-2-((2S,4S)-4-allyl-1-((S)-2-((S)-2-((tert-butoxycarbonyl) (methyl)amino)propanamido)-3,3-dimethylbutanoyl)pyrrolidine-2-carboxamido)-3-(naphthalen-2-yl)propanamido)-3-(4-(prop-2-yn-1-yloxy)phenyl)propanoate

To a solution of **Compound F of Example 86** (1.8 g, 2.8 mmol) in DMF (25 mL) was added HATU (1.26 g, 3.3 mmol). The reaction was stirred at room temperature for 5 min before a solution of NMM (1.22 mL, 11.1 mmol) and (*S*)-*tert*-butyl 2-amino-3-(4-(prop-2-yn-1-yloxy)phenyl)propanoate (**B**, 1.04 g, 3.32 mmol) in DMF (10 mL) was added. The yellow solution was stirred at room tempearture for 2 h before it was quenched with aq. LiCl, and extracted with EtOAc (3x). The combined organic extracts were washed with IN HCl and brine, dried over sodium sulfate, and concentrated *in vacuo* to give the crude product, which was used directly in the next step without further purification. MS(ESI⁺) *m*/*z* 908.8 (M + H)⁺.

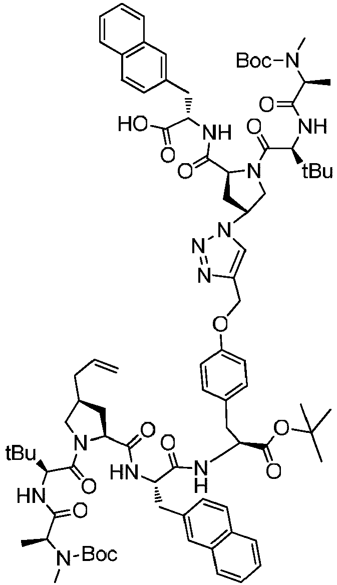

### D) (S)-2-((2S,4R)-4-(4-((4-((S)-2-((S)-2-((2S,4S)-4-Allyl-1-((S)-2-((S)-2-((tert-butoxycarbonyl)(methyl)amino)propanamido)-3,3-dimethylbutanoyl)pyrrolidine-2-carboxamido)-3-(naphthalen-2-yl)propanamido)-3-(tert-butoxy)-3-oxopropyl) phenoxy)methyl)-1H-1,2,3-triazol-t-yl)-1-((S)-2-((S)-2-((tert-butoxycarbonyl) (methyl) amino) propanamido)-3,3-dimethylbutanoyl)pyrrolidine-2-carboxamido)-3-(naphthalen-2-yl)propanoic acid

To a solution of the crude product from the previous step in THF/tBuOH/H₂O (40 mL, 1:1:1) was added **Compound K of Example 86** (1.62 g, 2.49 mmol), followed by a solution of sodium ascorbate (1.34 mmol). The reaction mixture was purged with N₂ before a solution of copper sulfate pentahydrate (69 mg, 0.28 mmol) in H₂O (1 mL) was added dropwise. After 5 h, the reaction was quenched with aq. NH₄Cl soln. (30 mL), concentrated *in vacuo*, and extracted with EtOAc(3x). The combined oganic extracts were washed with brine, dried over sodium sulfate, and concentrated *in vacuo.* The residue was purified using reverse phase column chromtography (ISCO, 70-100% acetonitrile/H₂O, 0.1% TFA) to give the title compound (2.5 g, 58% over two steps) as a white foam. MS(ESI⁺) *m*/*z* 1560.0 (M + H)⁺.

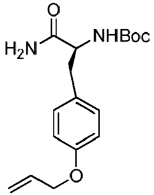

### E) (S)-tert-Butyl (3-(4-(allyloxy)phenyl)-1-amino-1-oxopropan-2-yl)carbamate

To a solution of (*S*)-3-(4-(allyloxy)phenyl)-2-((*tert*-butoxycarbonyl) amino)propanoic acid (4.0 g, 12.5 mmol) in THF (50 mL) was added DIPEA (6.46 mL, 37.3 mmol). The resulting solution was cooled to -10 °C and ethyl chloroformate (Aldrich, 1.79 mL, 18.7 mmol) was added dropwise. After the addition was complete, the reaction mixture was stirred at -10 °C for 30 minutes. The reaction mixture was then treated dropwise with 7 N NH₃ in MeOH (20 mL). The reaction mixture was then allowed to warm to room temperature and stir for 1.5 h. The reaction mixture was then quenched with IN aq. NaOH and extracted with EtOAc (3x). The combined organic extracts were washed with IN aq.NaOH soln., dried, filtered, and concentrated *in vacuo* to afford the title compound (3.9 g, 98%) as a white solid. MS(ESI⁺) *m*/*z* 321.3 (M + H)⁺.

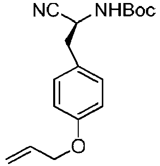

### F) (S)-tert-Butyl (2-(4-(allyloxy)phenyl)-1-cyanoethyl)carbamate

To a 0 °C solution of (*S*)*-tert-*butyl (3-(4-(allyloxy)phenyl)-1-amino-1-oxopropan-2-yl)carbamate in DCM/THF (1:1, 50 mL) was added Burgess reagent (4.0 g, 16.8 mmol) in portions over 30 minutes. The reaction was allowed to warm to room temperature and stirr for 2 h. A second batch of Burgess reagent (1.0 g, 4.2 mmol) was then added. The reaction mixture was stirred at room temperature for 30 min before it was quenched with brine and extracted with DCM (3x). The combined organic extracts were dried, filtered, and concentrated *in vacuo* to give the crude prodcut (3.76 g, 95%) as a white solid, which was used directly in the next step without any further purification. MS(ESI⁺) *m*/*z* 303.3 (M+H)+. ¹H NMR (400 MHz, DMSO-*d₆*) δ 7.14 (d, *J=* 8.6 Hz, 2H), 6.96 (br. s., 1H), 6.83 (d, *J=* 8.6 Hz, 2H), 6.71 (d, *J=* 8.8 Hz, 1H), 6.02 (ddt, *J=* 17.3, 10.5, 5.3 Hz, 1H), 5.37 (dq, *J* = 17.2, 1.8 Hz, 1H), 5.23 (dq, *J* = 10.6, 1.5 Hz, 1H), 4.60 - 4.34 (m, 2H), 4.12 - 3.97 (m, 1H), 2.87 (dd, *J* = 13.9, 4.4 Hz, 1H), 2.65 (dd, *J* = 13.6, 10.1 Hz, 1H), 1.36 - 1.23 (m, 9H).

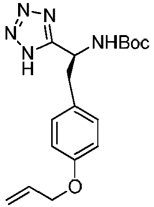

### G) (S)-tert-Butyl (2-(4-(allyloxy)phenyl)-1-(1H-tetrazol-5-yl)ethyl)carbamate

To a solution of (*S*)*-tert-*butyl (2-(4-(allyloxy)phenyl)-1-cyanoethyl)carbamate (1.0 g, 3.31 mmol) in toluene (15 mL) was added acetic acid (0.76 mL, 13.2 mmol), triethyl amine (1.84 mL, 13.2 mmol), and sodium azide (465 mg, 13.2 mmol). The resulting reaction mixture was stirred at 100 °C for 2 h. A second solution of triethyl amine (1.84 mL, 13.2 mmol) and acetic acid (0.76 mL, 13.2 mmol) in toluene (3 mL) and sodium azide (465 mg, 13.23 mmol) was added, and the resulting reaction mixture was stirred at 100 °C for 12 h. The reaction mixture was then allowed to cool to room temperature and diluted with water. The mixture was extracted with DCM (3x). The combined organic extracts were dried, filtered, and concentrated *in vacuo* to afford the product (1.2 g, 95%) as a white solid that was used in the next step without further purification. MS(ESI⁺) *m*/*z* 346.3 (M + H)⁺. ¹H NMR (400 MHz, DMSO-*d₆*) δ 7.22 - 7.13 (m, 1H), 7.06 (d, *J =* 8.4 Hz, 2H), 6.80 (d, *J =* 8.4 Hz, 2H), 6.01 (ddt, *J =* 17.3, 10.5, 5.3 Hz, 1H), 5.36 (dq, *J =* 17.3, 1.6 Hz, 1H), 5.22 (dq, *J =* 10.6, 1.5 Hz, 1H), 5.02 - 4.81 (m, 1H), 4.49 (d, *J =* 5.3 Hz, 2H), 3.16 - 2.89 (m, 2H), 1.35 - 1.20 (m, 9H).

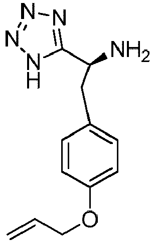

### H) (S)-2-(4-(Allyloxy)phenyl)-1-(1H-tetrazol-5-yl)ethanamine.hydrochloride salt

To a solution of (*S*)*-tert-*butyl (2-(4-(allyloxy)phenyl)-1-(1*H*-tetrazol-5-yl)ethyl)carbamate (1.2 g, 3.47 mmol) in DCM (4 mL) was added 4 N HCl in dioxane solution (8.69 mL, 34.7 mmol) and the resulting reaction mixture was stirred at room temperature for 12 h. The reaction mixture was then concentrated *in vacuo* and dried under high vacuum to afford the HCl salt of the title compound (0.98 g, 95%) as a white solid. MS(ESI⁺) *m*/*z* 246.2 (M + H)⁺.

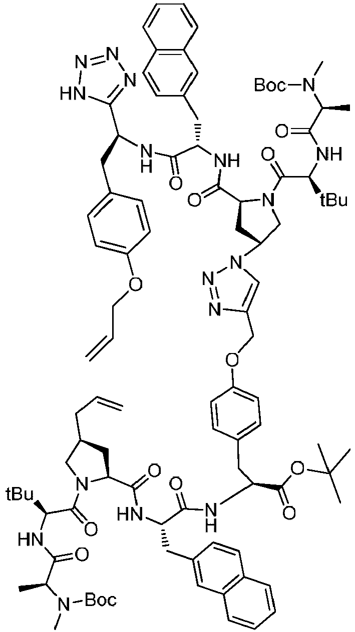

### I) (S)-tert-Butyl 2-((S)-2-((2S,4S)-4-allyl-1-((S)-2-((S)-2-((tert-butoxycarbonyl)(methyl)amino)propanamido)-3,3-dimethylbutanoyl)pyrrolidine-2-carboxamido)-3-(naphthalen-2-yl)propanamido)-3-(4-((1-((3R,5S)-5-(((S)-1-(((S)-2-(4-(allyloxy)phenyl)-1-(1H-tetrazol-5-yl)ethyl)amino)-3-(naphthalen-2-yl)-1-oxopropan-2-yl)carbamoyl)-1-((S)-2-((S)-2-((tert-butoxycarbonyl)(methyl)amino) propanamido)-3,3-dimethylbutanoyl)pyrrolidin-3-yl)-1H-1,2,3-triazol-4-yl)methoxy)phenyl)propanoate

(*S*)-2-(4-(Allyloxy)phenyl)-1-(1*H*-tetrazol-5-yl)ethanamine, HCl (0.095 g, 0.34 mmol), compound **D** (0.5 g, 0.321 mmol), HOAt (0.052 g, 0.39 mmol) and EDC (0.074 g, 0.39 mmol) were stirred in DCM (5 mL) and the resulting mixture was cooled to 0 °C. NMM (0.14 mL, 1.28 mmol) was then added and the reaction mixture was allowed to warm to room tempearture and stir at room tempearture overnight. The reaction mixture was quenched with 10% aq. NaHCO₃ soln. and the resulting mixture was extracted with DCM (3x). The combined organic extracts were washed with 1 N aq. HCl, dried, filtered, and concentrated *in vacuo.* The residue was purified by reverse phase column chromatography (ISCO, 70-100% acetonitrile/H₂O, with 0.1 % TFA) to afford the product (0.42 g, 73%) as a white solid. MS(ESI⁺) *m*/*z* 1787.1 (M + H)⁺.

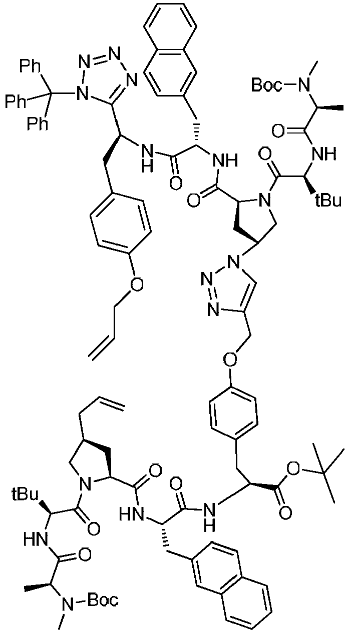

### J) (S)-tert-Butyl 2-((S)-2-((2S,4S)-4-allyl-1-((S)-2-((S)-2-((tert-butoxycarbonyl)(methyl)amino)propanamido)-3,3-dimethylbutanoyl)pyrrolidine-2-carboxamido)-3-(naphthalen-2-yl)propanamido)-3-(4-((1-((3R,5S)-5-(((S)-1-(((S)-2-(4-(allyloxy)phenyl)-1-(1-trityl-1H-tetrazol-5-yl)ethyl)amino)-3-(naphthalen-2-yl)-1-oxopropan-2-yl)carbamoyl)-1-((S)-2-((S)-2-((tert-butoxycarbonyl)(methyl) amino)propanamido)-3,3-dimethylbutanoyl)pyrrolidin-3-yl)-1H-1,2,3-triazol-4-yl)methoxy)phenyl)propanoate

To a solution of compound **I** (0.42 g, 0.23 mmol) in DCM (6 mL) was added triphenylmethyl chloride (Aldrich, 0.068 g, 0.25 mmol) at room temperature. The resulting solution was then treated with DIPEA (0.081 mL, 0.47 mmol) and the resulting reaction mixture was stirred at room tempearture overnight. The reaction mixture was then quenched with 10% aq. citric acid and the solution was extracted with DCM (3x). The combined organic extracts were dried, filtered and concentrated *in vacuo* to afford the product (0.473 g, 98%) as a white solid. LC/MS showed product minus trityl group, MS(ESI⁺) *m*/*z* 1787.0 (M + H -trityl)⁺.

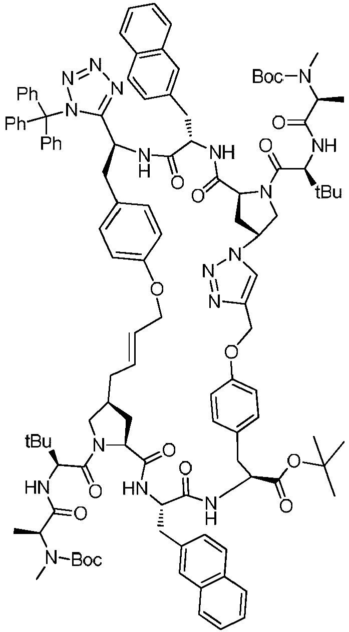

### K) Compound K

To a solution of compound **J** (0.473 g, 0.234 mmol) in DCE (100 mL) was added Hoveyda-Grubbs 2^{nd} generation catalyst (7.3 mg, 0.012 mmol). The reaction mixture was then purged with N₂ for 5 minutes and then heated to 70 °C for 2 h. A second batch of Hoveyda-Grubbs 2^{nd} generation catalyst (7.3 mg, 0.012 mmol) was added and the reaction mixture was stirred at 70 °C for 2 h. The reaction mixture was then cooled to room temperature and concentrated *in vacuo.* The residue was purified by reverse phase column chromatography (ISCO, 70-100% acetonitrile/H₂O, with 0.1%) to afford the desired product (0.235 g, 48%) as a white solid after lyophilization. MS(ESI⁺) *m*/*z* 1759.0 (M + H -trityl)⁺.

### L) Example 90

A solution of compound **K** (0.23 g, 0.115 mmol) and 5% Pd/C in MeOH (5 mL) was stirred under H₂ (50 psi) at room temperature overnight. The reaction mixture was filtered through Celite^{®}, washed with MeOH, and concentrated *in vacuo.* The residue was dissolved in DCM (10 mL) and then the resulting solution was treated with TFA (20 mL). The resulting reaction mixture was stirred at room temperature for 2 h before it was concentrated *in vacuo* and purified by reverse phase column chromatography (ISCO 20-50% acetonitrile/H₂O, with 0.1% TFA). After lyophilization, the crude product and 5% Pd/C were suspended in MeOH (5 mL), charged with H₂ (50 psi) and stirred at room tempearture overnight. The reaction mixture was filtered through Celite^{®}, washed with MeOH, and concentrated *in vacuo.* The residue was purified using preparative HPLC to afford the title compound (0.025 g, 14%) as a white solid after lyophilization. MS(ESI⁺) *m*/*z* 1504.7 (M + H)⁺.

### EXAMPLE 91

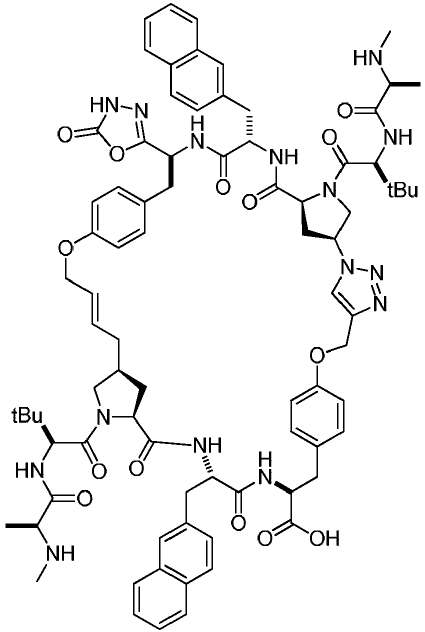

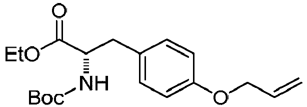

### A) (S)-Ethyl 3-(4-(allyloxy)phenyl)-2-((tert-butoxycarbonyl)amino) propanoate

To a solution of (*S*)-3-(4-(allyloxy)phenyl)-2-((*tert*-butoxycarbonyl)amino) propanoic acid (0.80 g, 2.49 mmol, Aldrich) in ethanol (5 mL) was added conc. H₂SO₄ (1 mL). The reaction mixture was heated at 80 °C overnight and concentrated *in vacuo.* The resulting residue was dissolved in DCM (-100 mL) and washed with sat. aq. NaHCO₃ soln. The organic layer was dried over MgSO₄ and concentrated *in vacuo* to give a clear oil. The clear oil was dissolved in THF (6 mL) and water (6 mL). Sodium bicarbonate (0.418 g, 4.98 mmol, Aldrich) and di-*tert*-butyl dicarbonate (0.694 mL, 2.99 mmol, Aldrich) were added. The reaction mixture was stirred at room temperature for 2 h and concentrated *in vacuo* to remove volatiles. The residue was neutralized with1 N aq. HCl to pH ~3-4, and then extracted with DCM (3x). The combined organic extracts were dried over MgSO₄, filtered and concentrated *in vacuo* to give the desired product (0.59 g, 71%) as a thick oil. ¹H NMR (CDCl3) δ 7.05 (d, *J* = 8.6 Hz, 2H), 6.85 (d, *J* = 8.6 Hz, 2H), 6.05 (ddt, *J* = 17.2, 10.6, 5.3 Hz, 1H), 5.41 (dq, *J* = 17.2, 1.6 Hz, 1H), 5.28 (dq, *J* = 10.6, 1.6 Hz, 1H), 4.97 (d, *J* = 7.0 Hz, 1H), 4.52 (dt, *J* = 5.4, 1.4 Hz, 2H), 4.16 (q, *J=* 7.2 Hz, 2H), 3.12 - 2.94 (m, 2H), 1.43 (s, 9H), 1.24 (t, *J* = 7.2 Hz, 3H); MS(ESI⁺) *m*/*z* 350.3 (M + H)⁺.

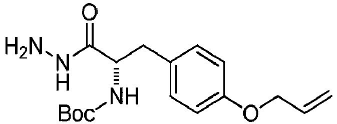

### B) ((S)-tert-Butyl (3-(4-(allyloxy)phenyl)-1-hydrazinyl-1-oxopropan-2-yl)carbamate

To a solution of (*S*)-ethyl 3-(4-(allyloxy)phenyl)-2-((*tert*-butoxycarbonyl) amino)propanoate (1.96 g, 5.61 mmol) in DMF (5 mL) was added hydrazine (98%, 0.539 g, 16.8 mmol). The reaction mixture was heated at 80 °C for 1 h. After cooling to room temperature, the reaction mixture was diluted with cold water (50 mL). The white solid that formed was collected by filtration, and purified with flash column chromatography (gradient elution from 0 - 5% MeOH in DCM) to provide the title compound (1.53 g, 81%) as a white solid. ¹H NMR (400 MHz, CDCl₃) δ 7.12 (d, *J=* 8.6 Hz, 2H), 7.07 (s, 1H), 6.84 (d, *J* = 8.6 Hz, 2H), 6.08 (ddt, *J* = 17.2, 10.6, 5 .3 Hz, 1H), 5.44 (dq, *J* = 17.2, 1.6 Hz, 1H), 5.32 (dd, *J* = 10.6, 1.6 Hz, 1H), 5.00 (br. s., 1H), 4.54 (dt, *J* = 5.3, 1.6 Hz, 2H), 4.28 (q, *J* = 7.0 Hz, 1H), 3.94 - 3.72 (m, 1H), 3.02 (dd, *J* = 7.0, 3.3 Hz, 2H), 1.45 (s, 9H); MS(ESI⁺) *m*/*z* 336.3 (M + H)⁺.

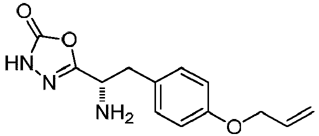

### C) (S)-5-(2-(4-(Allyloxy)phenyl)-1-aminoethyl)-1,3,4-oxadiazol-2(3H)-one

To a solution of (*S*)*-tert-*butyl (3-(4-(allyloxy)phenyl)-1-hydrazinyl-1-oxopropan-2-yl)carbamate (360 mg, 1.07 mmol) in THF (5 mL) and DMF (1 mL) were added CDI (226 mg, 1.395 mmol, Aldrich) and triethylamine (0.299 mL, 2.147 mmol). The reaction mixture was heated at 75° C for 1 h. After cooling to room temperature, the reaction mixture was extracted with DCM (3x). The combined org. extracts were dried over MgSO₄, filtered and concentrated *in vacuo.* The resulting thick oil was purified by flash column chromatography (gradient elution from 0 - 50% EtOAc in DCM) to provide the desired product (350 mg, 90%) as a white solid. MS(ESI⁺) *m*/*z* 362.2 (M + H)⁺.

To a solution of the above compound (350 mg, 0.968 mmol) in DCM (5 mL) at room temperature was added TFA (1 mL). The reaction mixture was stirred at rt for 2 h and then concentrated *in vacuo.* The residue was dissolved in DCM (-30 mL) and washed with sat. aq. NaHCO₃ soln. The organic layer was washed with brine, dried over MgSO₄, filtered and concentrated *in vacuo* to give the desired product (240 mg, 95%) as a white solid. ¹H NMR (400 MHz, CD₃OD) δ 7.16 (d, *J* = 0.9 Hz, 1H), 7.10 (d, *J* = 8.6 Hz, 2H), 6.90 - 6.84 (d, *J=* 8.6 Hz, 2H), 6.04 (ddt, *J=* 17.3, 10.5, 5.2 Hz, 1H), 5.42 - 5.34 (m, 1H), 5.27 - 5.19 (m, 1H), 4.50 (dt, *J =* 5.2, 1.5 Hz, 2H), 4.10 (t, *J =* 7.2 Hz, 1H), 3.03 (m, 2H); MS(ESI⁺) *m*/*z* 262.2 (M + H)⁺.

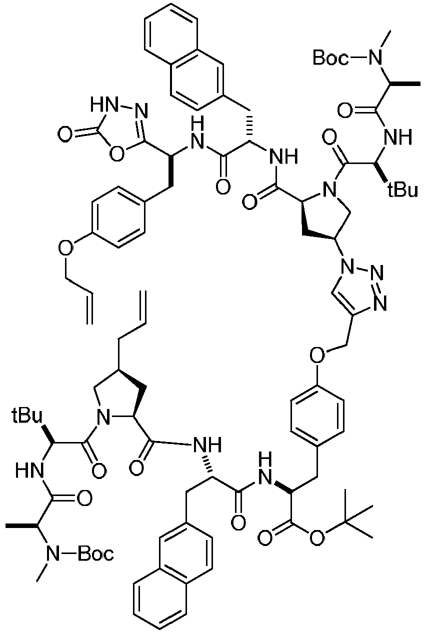

### D) (S)-tert-Butyl 2-((S)-2-((2S,4S)-4-allyl-1-((S)-2-((S)-2-((tert-butoxycarbonyl)(methyl)amino)propanamido)-3,3-dimethylbutanoyl)pyrrolidine-2-carboxamido)-3-(naphthalen-2-yl)propanamido)-3-(4-((1-((3S,5S)-5-(((S)-1-(((S)-2-(4-(allyloxy)phenyl)-1-(5-oxo-4,5-dihydro-1,3,4-oxadiazol-2-yl)ethyl)amino)-3-(naphthalen-2-yl)-t-oxopropan-2-yl)carbamoyl)-1-((S)-2-((S)-2-((tert-butoxycarbonyl)(methyl)amino)propanamido)-3,3-dimethylbutanoyl)pyrrolidin-3-yl)-1H-1,2,3-triazol-4-yl)methoxy)phenyl)propanoate

To a solution of (*S*)-2-((2*S*,4*R*)-4-(4-((4-((*S*)-2-((*S*)-2-((2*S*,4*S*)-4-allyl-1-((*S*)-2-((*S*)-2-((*tert*-butoxycarbonyl)(methyl)amino)propanamido)-3,3-dimethylbutanoyl)pyrrolidine-2-carboxamido)-3-(naphthalen-2-yl)propanamido)-3-(*tert-*butoxy)-3-oxopropyl)phenoxy)methyl)-1*H*-1,2,3-triazol-1-yl)-1-((*S*)-2-((*S*)-2-((*tert-*butoxycarbonyl)(methyl)amino)propanamido)-3,3-dimethylbutanoyl)pyrrolidine-2-carboxamido)-3-(naphthalen-2-yl)propanoic acid (**Compound D of Example 89,** 120 mg, 0.077 mmol) in DMF (1 mL) was added HATU (35 mg, 0.092 mmol) and DIEA (0.020 mL, 0.115 mmol). The reaction was stirred at room temperature for 5 min before a solution of (*S*)-5-(2-(4-(allyloxy)phenyl)-1-aminoethyl)-1,3,4-oxadiazol-2(3*H*)-one (**C**, 34.6 mg, 0.092 mmol) and DIEA in DMF (0.5 mL). The reaction mixture was stirred at room temperature for 3 h. The reaction was quenched with aq. LiCl (5 mL), extracted with DCM (3 x 10 mL), washed with brine, dried over MgSO₄, filtered and concentrated *in vacuo.* The residue was purified by reverse phase column chromatography (ISCO, 70% -100% acetonitrile/H₂O, with 0.1% TFA) to provide the title compound (102 mg, 74%) as a white solid. MS(ESI⁺) *m*/*z* 1803.2 (M + H)⁺.

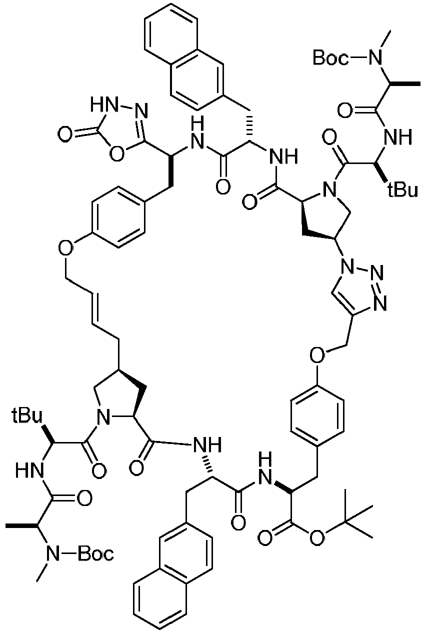

### E) Compound E

To a solution of the compound from the previous step (100 mg, 0.055 mmol) in DCE (10 mL) was added a solution of Hoveyda-Grubbs II catalyst (3.48 mg, 5.55 µmol) in DCE (0.5 mL). The reaction mixture was heated at 70 °C overnight. The reaction was then cooled to room temperature and concentrated *in vacuo.* The residue was purified by preparative HPLC to give the desired product (51 mg, 52%). MS(ESI⁺) *m*/*z* 1776.1 (M + H)⁺.

### F) Example 91

To a solution of compound **E** (50 mg, 0.028 mmol) in DCM (3 mL) at room temperature was added TFA (1.5 mL). The reaction mixture was stirred at room temperature for 5 h, and then concentrated *in vacuo.* The residue was purified by preparative HPLC to give the title compound (20 mg, 42%) as a white solid after lyophilization. MS(ESI⁺) *m*/*z* 1518.8 (M + H)⁺.

### EXAMPLE 92

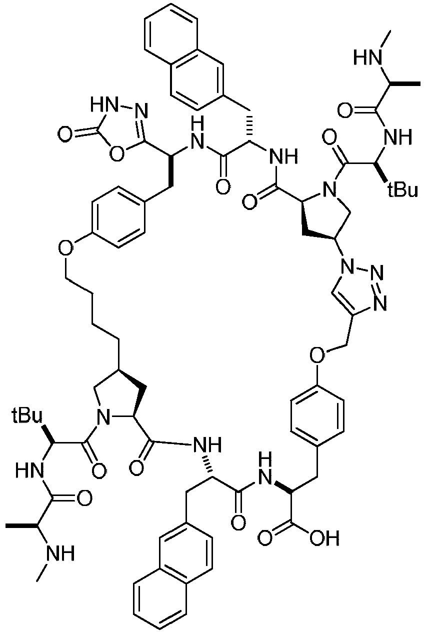

To a solution of **Example 91** (30 mg, 0.020 mmol) in MeOH (8 mL) was added 5% Pd/C (6 mg, 0.056 mmol). The resulting suspension was stirred under H₂ (50 psi) for 16 h. The reaction mixture was diluted with EtOAc and filtered through Celite^{®}. The filtrate was concentrated *in vacuo* and purified by preparative HPLC to give the title compound (7 mg, 21%) as a white solid after lyophilization. MS(ESI⁺) *m*/*z* 1522.3 (M + H)⁺.

### EXAMPLES 93 TO 98

The following examples were prepared according to the procedures described for the synthesis of Example **91.**

| **Ex No.** | **Structure** | **Observed MS** |
|---|---|---|
| **93** | 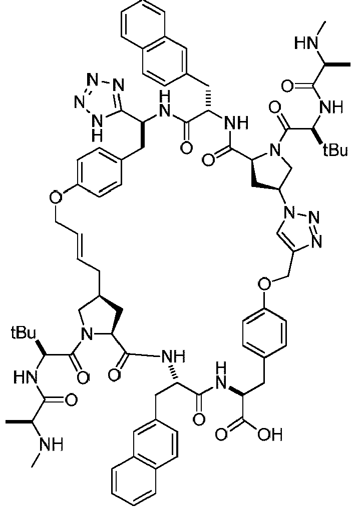 | 752.1 |
| **94** | 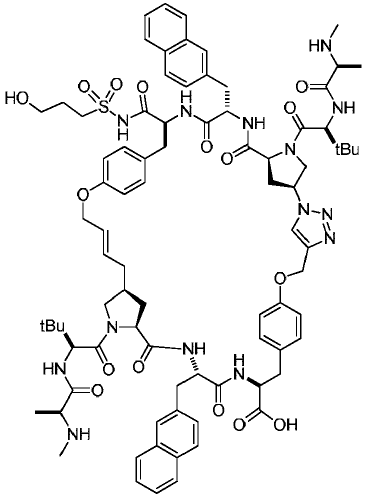 | 800.4 |
| **95** | 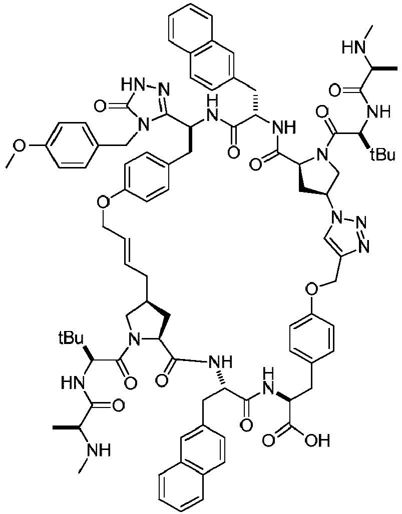 | 1638.9 |
| **96** | 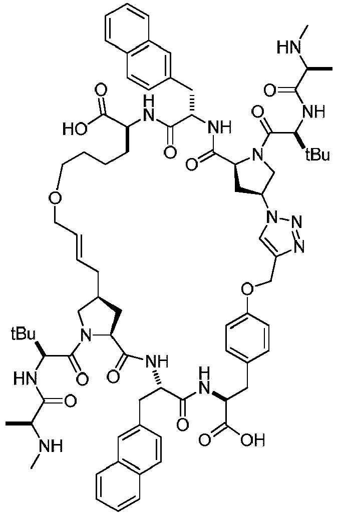 | 1446.1 |
| **97** | 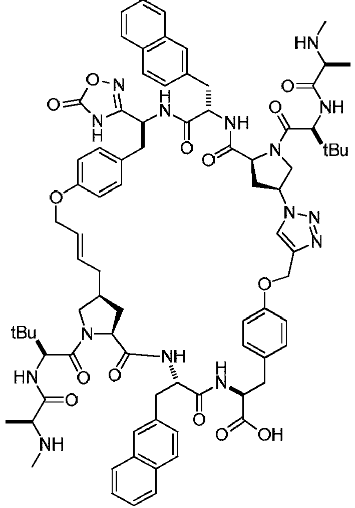 | 1519.0 |
| **98** | 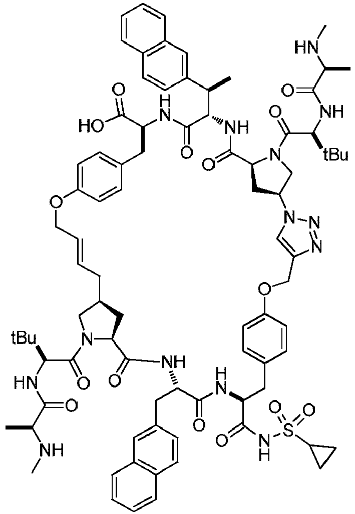 | 1595.9 |

### EXAMPLES 99 TO 101

The following examples were prepared according to the procedures described for the synthesis of Example **92**.

| **Ex No**. | **Structure** | **Observed MS** |
|---|---|---|
| **99** | 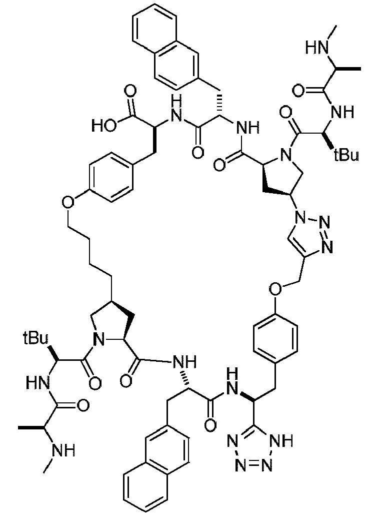 | 1504.8 |
| **100** | 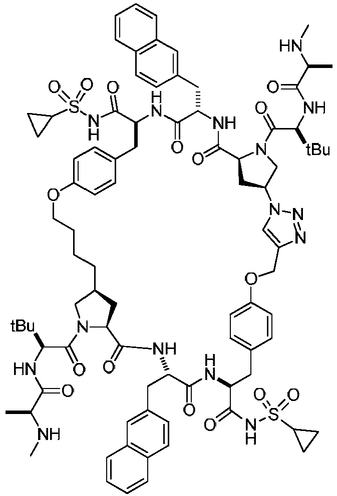 | 1686.7 |
| **101** | 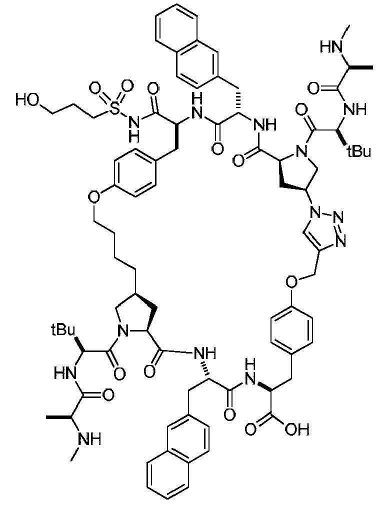 | 801.5 |

### EXAMPLE 102

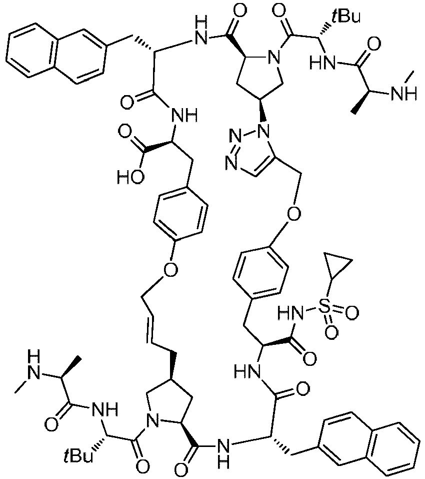

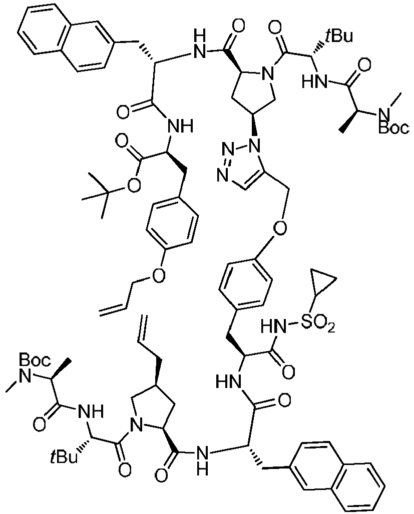

### A) (S)-tert-butyl 2-((S)-2-((2S,4S)-4-(5-((4-((S)-2-((S)-2-((2S,4S)-4-allyl-1-((S)-2-((S)-2-((tert-butoxycarbonyl)(methyl)amino)propanamido)-3,3-dimethylbutanoyl)pyrrolidine-2-carboxamido)-3-(naphthalen-2-yl)propanamido)-3-(cyclopropanesulfonamido)-3-oxopropyl)phenoxy)methyl)-1H-1,2,3-triazol-1-yl)-1-((S)-2-((S)-2-((tert-butoxycarbonyl)(methyl)amino)propanamido)-3,3-dimethylbutanoyl)pyrrolidine-2-carboxamido)-3-(naphthalen-2-yl)propanamido)-3-(4-(allyloxy)phenyl)propanoate

A solution of **Compound N** of **Example 86** (60 mg, 0.066 mmol), **Compound F** of **Example 86** (62.9 mg, 0.066 mmol) and pentamethylcyclopentadienylbis (triphenylphosphine)ruthenium(II) chloride ( Aldrich, 5.26 mg, 6.59 µmol) in toluene (3 mL) was heated at 90 °C for 6 h. The resulting solution was cooled to rt and then concentrated *in vacuo.* The residue was purified by reversed phase column chromtography (ISCO, 70-100% acetonitrile/H₂O, 0.1 % TFA, 26 g column) to give the title compound (49 mg, 40%) as a white solid after lyophilization. MS(ESI⁺) *m*/*z* 1866.7.

### B) Example 102

Following a procedure analogous to that for the synthesis of **Compound O of Example 1,** the peptide from the previous step (49 mg, 0.026 mmol) was converted to the title compound (2.0 mg, 2%). MS(ESI⁺) m/z 1582.1

### EXAMPLES 103 TO 117

The following examples were prepared according to the procedures described for the synthesis of Example **1**.

| **Ex No**. | **Structure** | **Predicted MS** | **Observed MS** |
|---|---|---|---|
| **103** | 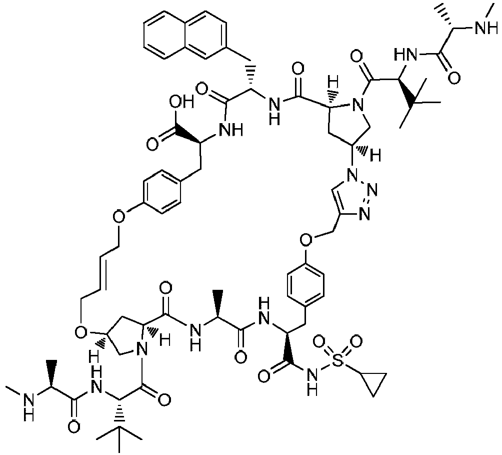 | 1471.74 | 737.0 M+2 |
| **104** | 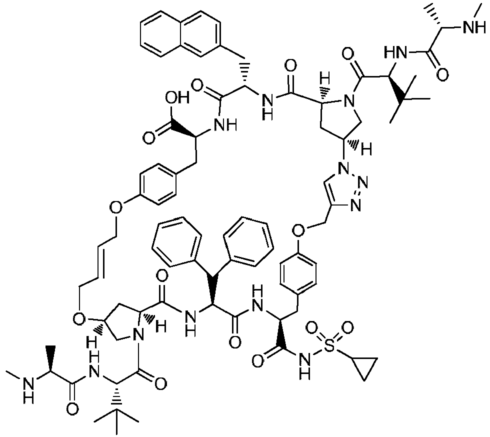 | 1623.93 | 813.0 M+2 |
| **105** | 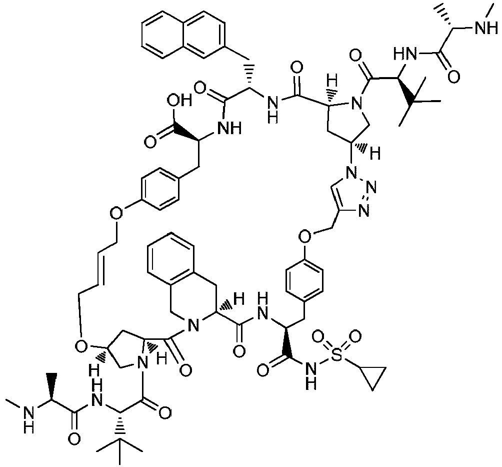 | 1559.84 | 781.0 M+2 |
| **106** | 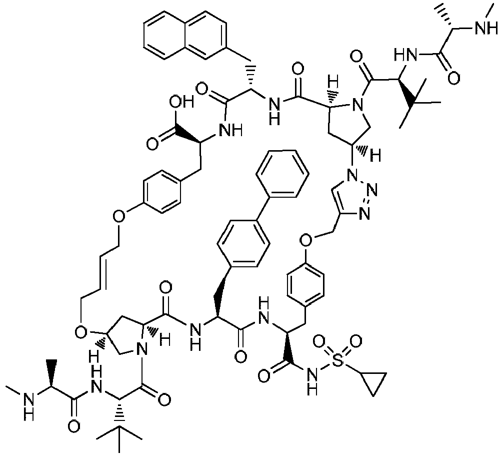 | 1623.93 | 813.0 M+2 |
| **107** | 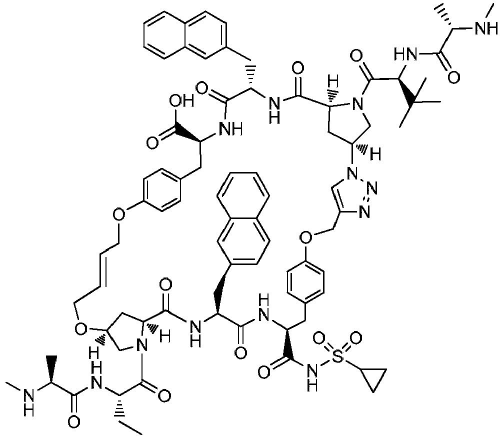 | 1569.84 | 785.6 M+2 |
| **108** | 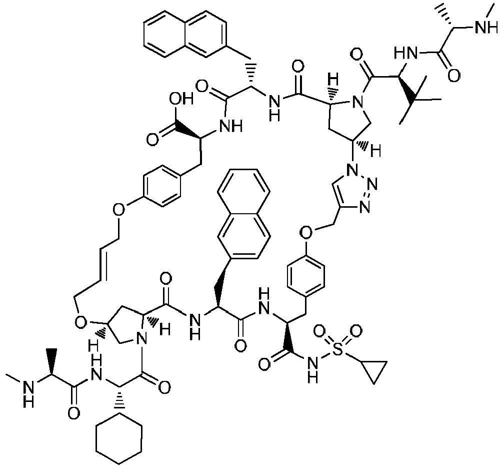 | 1623.93 | 813.0 M+2 |
| **109** | 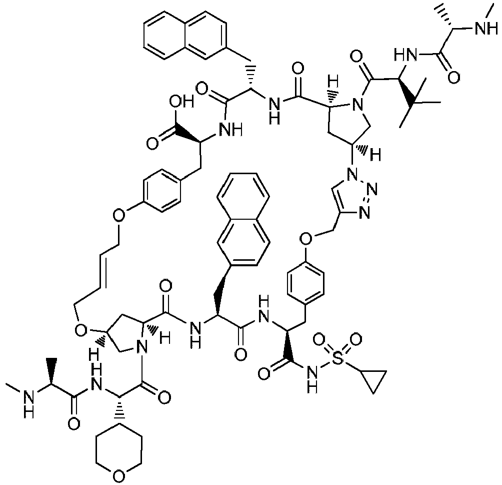 | 1625.9 | 814.0 M+2 |
| **110** | 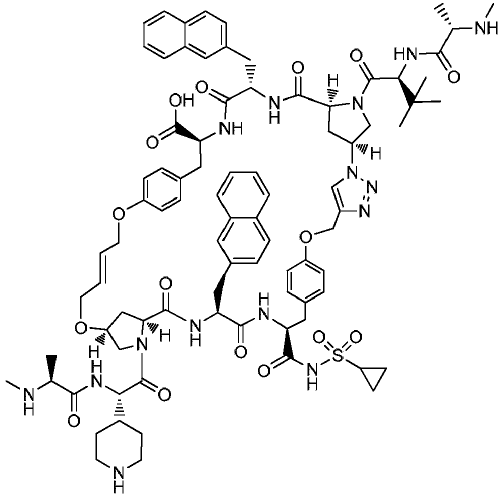 | 1624.92 | 813.5 M+2 |
| **111** | 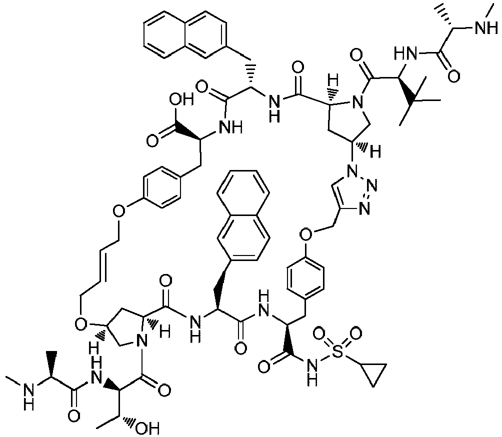 | 1585.84 | 794.0 M+2 |
| **112** | 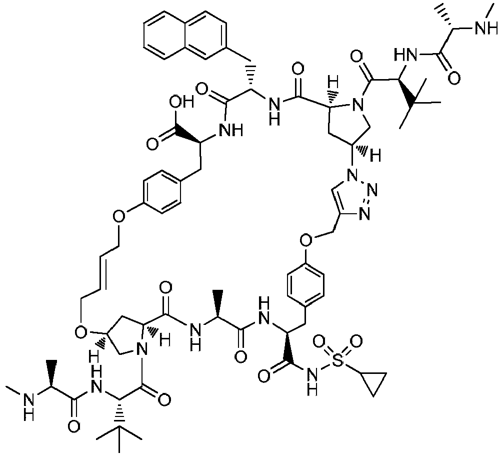 | 1624.92 | 814.5 M+2 |
| **113** | 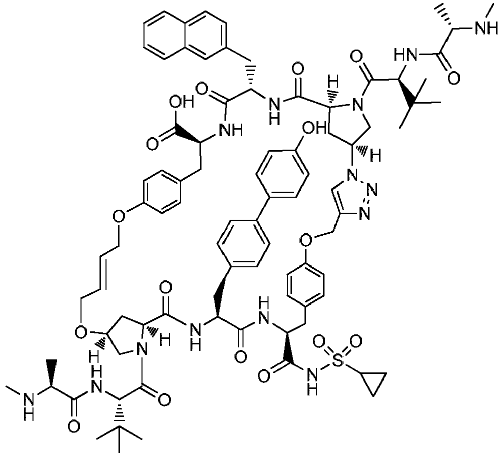 | 1639.93 | 822.0 M+2 |
| **114** | 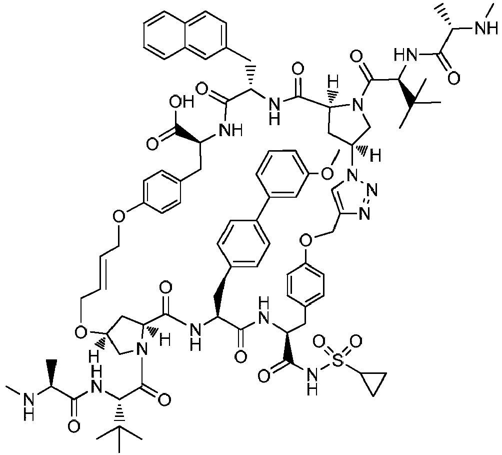 | 1653.96 | 828.7 M+2 |
| **115** | 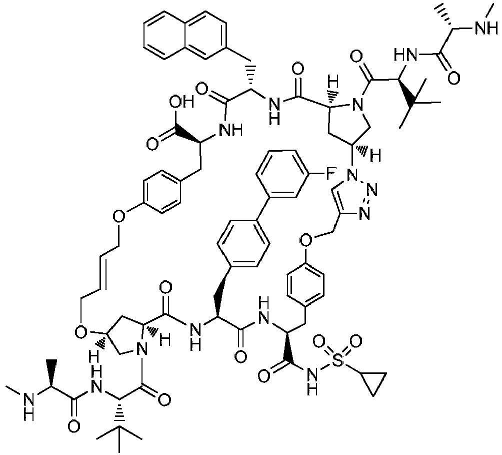 | 1641.92 | 822.5 M+2 |
| **116** | 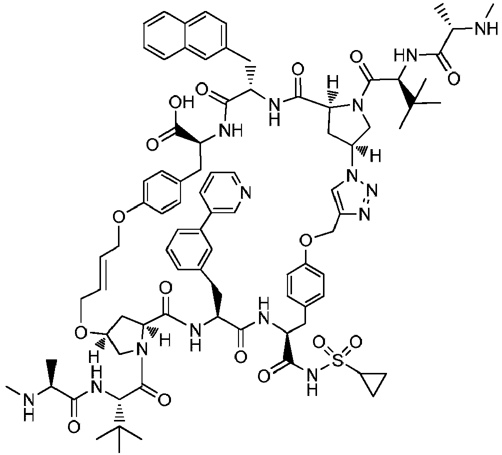 | 1624.92 | 814.0 M+2 |
| **117** | 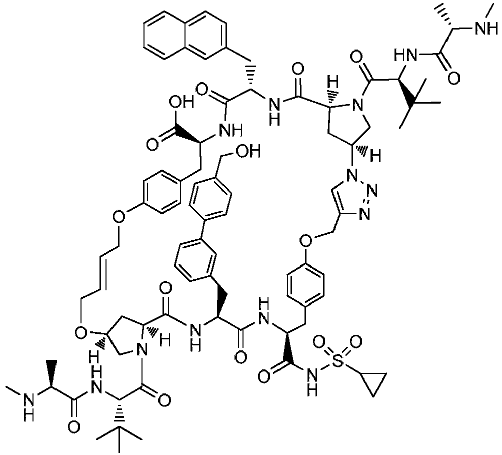 | 1653.96 | 828.5 M+2 |

### EXAMPLES 118

The following example was prepared according to the procedures described for the synthesis of Example **86.**

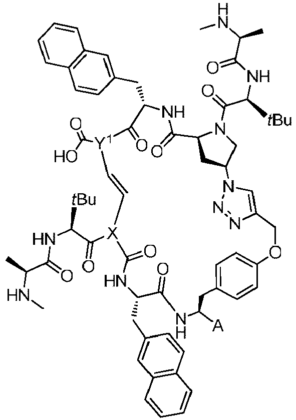

| **Ex No.** | **Structure** | **Predicted MS** | **Observed MS** |
|---|---|---|---|
| **118** | 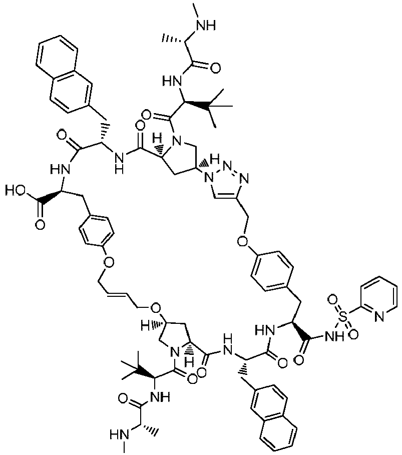 | 1634.91 | 819.08 M+2 |

### EXAMPLES 119 TO 127

The following examples were prepared according to the procedures described for the synthesis of Example **87.**

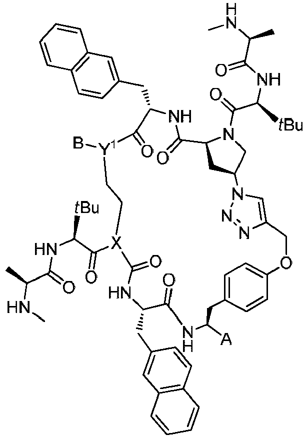

| **Ex No.** | **Structure** | **Predicted MS** | **Observed MS** |
|---|---|---|---|
| **119** | 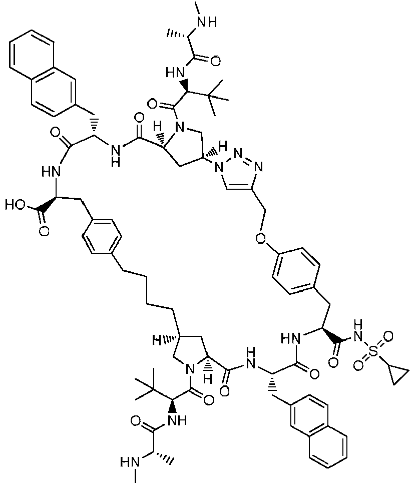 | 1567.91 | 785.08 M+2 |
| **120** | 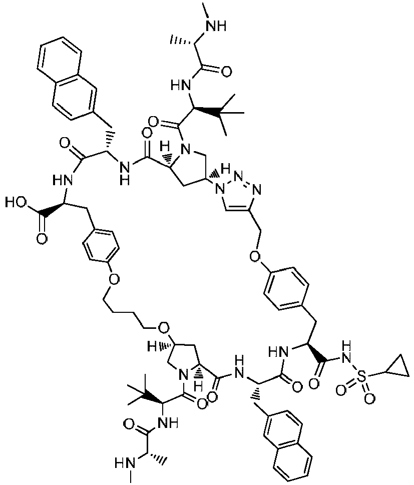 | 1599.91 | 801.08 M+2 |
| **121** | 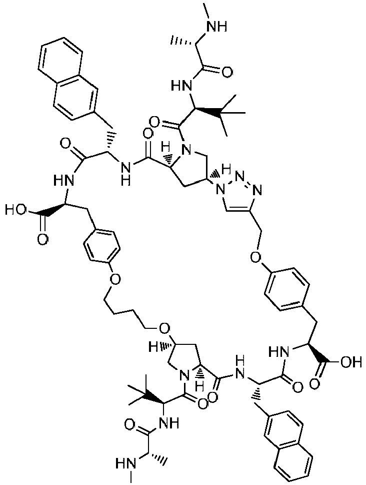 | 1496.76 | 749.4 M+2 |
| **122** | 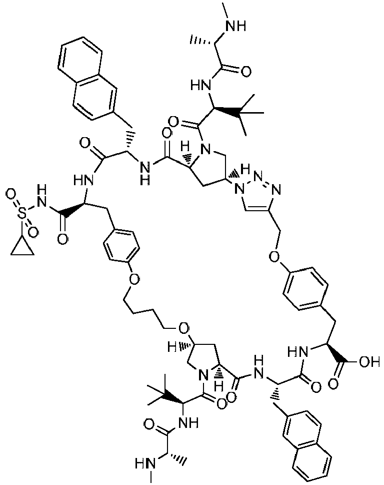 | 1599.91 | 801.0 M+2 |
| **123** | 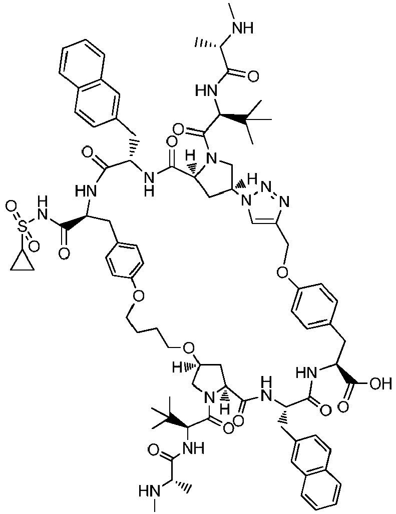 | 1599.91 | 801.0 M+2 |
| **124** | 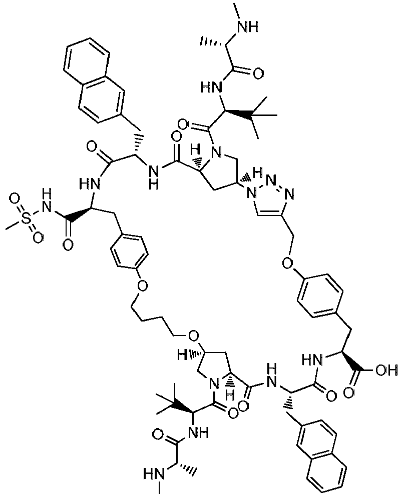 | 1573.87 | 787.3 M+2 |
| **125** | 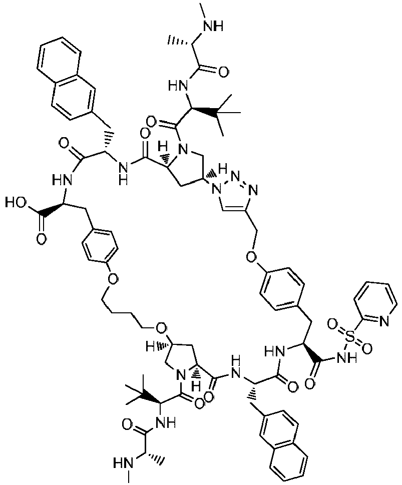 | 1636.93 | 1636.1 |
| **126** | 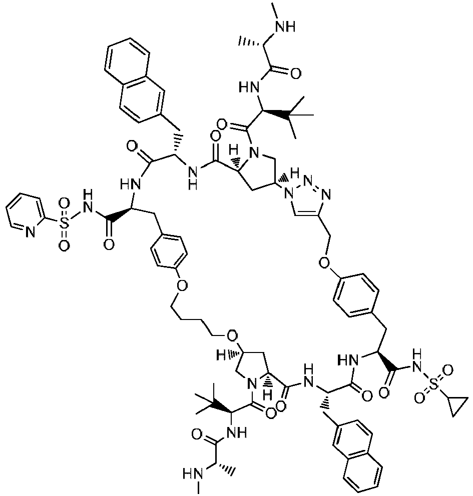 | 1740.08 | 870.6 M+2 |
| **127** | 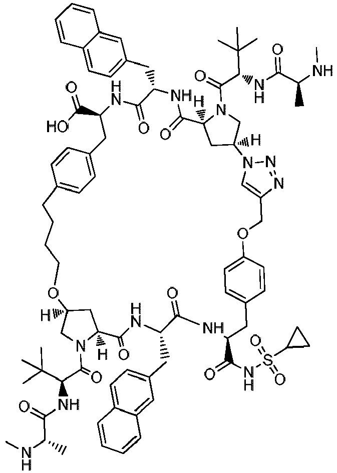 | 1583.91 | 792.6 M+2 |

### EXAMPLE 128

The following example was prepared according to the procedures described for the synthesis of Example **90.**

| **Ex No.** | **Structure** | **Predicted MS** | **Observed MS** |
|---|---|---|---|
| **128** | 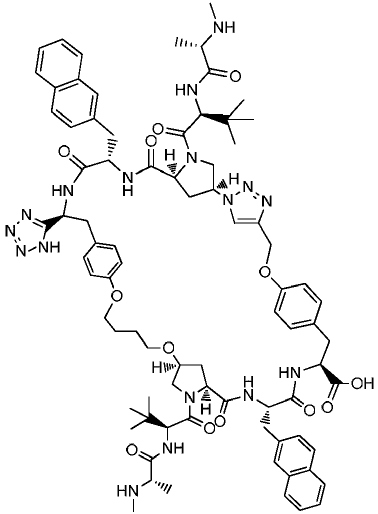 | 1520.79 | 761.1 M+2 |

### EVALUATION OF BIOLOGICAL ACTIVITY

Exemplary compounds were tested for inhibition of XIAP BIR3, XIAP BIR2 and XIAP BIR2-3 activity. Experimental procedures and results are provided below.

### A. XIAP-BIR3 SMAC Peptide Fluorescence Polarization Assay (FPA)

Assays were performed in black, flat-bottom, 384-well plates. The final assay volume was 50 µL prepared from additions of N-His-Tb-BIR3(241-356, XIAP), fluoresceinated modified SMAC peptide, and test compounds in assay buffer consisting of 20 mM Sodium Phosphate, 1 mM EDTA, 50 mM NaCl, and 0.05% Pluronic F68. The reaction was incubated at room temperature for 60 minutes and fluorescence polarization of the reaction was detected on the LJL Plate Reader. Inhibition data were calculated from mP values generated by the no protein control reactions for 100% inhibition and vehicle-only reactions for 0% inhibition. The final concentration of reagents in the assay was 130 nM N-His-Tb-BIR3(241-356, XIAP), 1.4 nM fluoresceinated modified SMAC peptide, and 1% DMSO. Dose response curves were generated to determine the concentration required for inhibiting 50% of polarization activity (IC₅₀). Compounds were dissolved at 10 mM in dimethylsulfoxide (DMSO) and evaluated at eleven concentrations. IC₅₀ values were derived by non-linear regression analysis.

### B. XIAP-BIR3 / SMAC Homogeneous Time Resolved Fluorescence (HTRF) Assay

Assays were performed in black, flat-bottom, 384-well plates. The final assay volume was 50 µL prepared from additions of His-BIR3 (241-356, XIAP), fluorescein labeled SMAC peptide, and test compounds in assay buffer consisting of 20 mM Sodium Phosphate, 1 mM EDTA, 50 mM NaCl, 50 µg/ml BSA, and 0.05% Pluronic F68. The reaction was incubated at room temperature for 60 minutes, following which 10 µl of mouse anti-6xHis-terbium labeled Fab (Medarex,Cis-bio) was added to the reaction (40 µl) for an additional 30 minute incubation. The HTRF signal, ratio of fluorescence intensities at emission wavelengths for fluorescein acceptor (520 nm) and terbium donor (615 nm), the 520/615 ratio, generated by the reaction was then measured on the Envision Plate Reader. Inhibition data were calculated from the 520/615 ratio generated by the no protein control reactions for 100% inhibition and vehicle-only reactions for 0% inhibition. The final concentration of reagents in the assay was 1 nM N-His -BIR3(241-356, XIAP), 5 nM fluorescein labeled SMAC peptide, 0.25 nM anti-His-Tb-Fab, and 0.1% DMSO. Dose response curves were generated to determine the concentration required for inhibiting 50% of the HTRF signal (IC₅₀). Compounds were dissolved at 3 mM in dimethylsulfoxide (DMSO) and evaluated at eleven serially diluted concentrations. IC₅₀ and Kᵢ values were derived by non-linear regression analysis.

### C. XIAP-BIR2 / SMAC Peptide AlphaScreen Assay

Assays were performed in white, flat-bottom, 384-well ProxiPlates (Perkin Elmer). The final assay volume was 10 µL prepared from additions of His-BIR2 (124-240/C202A/C213G), Biotinylated SMAC peptide, and test compounds in assay buffer consisting of 25 mM Hepes, 100 mM NaCl, 0.1% BSA, and 5 mM CaCl₂. The reaction was incubated at room temperature for 60 minutes. After 60 minutes, 2.5 µL of Alphascreen detection reagent (Perkin Elmer) was added to the reaction mixture and incubated at room temperature in the dark for 120 minutes. The Alphascreen signal generated by the reaction was detected on the Envision Plate Reader. Inhibition data were calculated from an Alphascreen signal generated by the no protein control reactions for 100% inhibition and vehicle-only reactions for 0% inhibition. The final concentration of reagents in the assay was 50 nM His-BIR2 (124-240/C202A/C213G), 50 nM

Biotinylated SMAC peptide, 4 µg/mL Alphascreen detection reagents, and 0.5% DMSO. Dose response curves were generated to determine the concentration required for inhibiting 50% of the activity (IC₅₀). Compounds were dissolved at 10 mM in dimethylsulfoxide (DMSO) and evaluated at eleven concentrations. IC₅₀ values were derived by non-linear regression analysis.

### D. XIAP-BIR2-3 dimeric SMAC Peptide Homogeneous Time Resolved Fluorescence (HTRF) Assay

Assays were performed in black, flat-bottom, 384-well plates. The final assay volume was 50 µL prepared from additions of His-BIR2-3 (125-356, C202A/C213G, XIAP), fluorescein labeled dimeric SMAC peptide, and test compounds in assay buffer consisting of 20 mM Sodium Phosphate, 1 mM EDTA, 50 mM NaCl, 50 µg/ml BSA, and 0.05% Pluronic F68. The reaction was incubated at room temperature for 60 minutes, following which 10 µl of mouse anti-6xHis-Tb IgG (Medarex,Cis-bio) was added to the reaction (40 µl) for an additional 30 minute incubation. The HTRF signal, ratio of fluorescence intensities at emission wavelengths for fluorescein acceptor (520 nm) and terbium donor (615 nm), the 520/615 ratio, generated by the reaction was then measured on the Envision Plate Reader. Inhibition data were calculated from the 520/615 ratio generated by the no protein control reactions for 100% inhibition and vehicle-only reactions for 0% inhibition. The final concentration of reagents in the assay was 0.5 nM N-His -BIR2-3(125-356, C202A/C213G, XIAP), 20 nM fluorescein labeled dimeric SMAC peptide, 0.25 nM anti-His-Tb-Fab, and 0.1% DMSO. Dose response curves were generated to determine the concentration required for inhibiting 50% of the HTRF signal (IC₅₀). Compounds were dissolved at 3 mM in dimethylsulfoxide (DMSO) and evaluated at eleven serially diluted concentrations. IC₅₀ and Kᵢ values were derived by non-linear regression analysis.

### Results:

Results of the XIAP BIR3, XIAP BIR2 and XIAP BIR2-3 assays are shown in the Table below. "NT" means that the compound was not tested in the assay.

**TABLE**

| **Examples Number** | **BIR3 FPA IC₅₀ (uM)** | **BIR3 HTRF IC₅₀ (uM)** | **BIR2 ALPHA IC₅₀ (uM)** | **BIR2-3 HTRF IC₅₀ (uM)** |
|---|---|---|---|---|
| **1** | NT | 0.0017 | NT | 0.0009 |
| **2** | NT | 0.0023 | NT | 0.0008 |
| **3** | NT | 0.0075 | NT | 0.0026 |
| **4** | NT | 0.0041 | NT | 0.0007 |
| **5** | 0.0500 | NT | 0.0715 | NT |
| **6** | NT | 0.0036 | NT | 0.0007 |
| **7** | NT | 0.0063 | NT | 0.0010 |
| **8** | 0.0496 | NT | 0.1904 | NT |
| **9** | NT | 0.0052 | NT | 0.0016 |
| **10** | NT | 0.0066 | NT | 0.0031 |
| **11** | NT | 0.0008 | NT | 0.0023 |
| **12** | NT | 0.0012 | NT | 0.0012 |
| **13** | NT | 0.0054 | NT | 0.0124 |
| **14** | NT | 0.0170 | NT | 0.0037 |
| **15** | NT | 0.0389 | NT | 0.0041 |
| **16** | NT | 0.0062 | NT | 0.0035 |
| **17** | NT | 0.0018 | NT | 0.0018 |
| **18** | NT | 0.0058 | NT | 0.0018 |
| **19** | NT | 0.0353 | NT | 0.0168 |
| **20** | NT | 0.0048 | NT | 0.0073 |
| **21** | NT | 0.0018 | NT | 0.0026 |
| **22** | NT | 0.0003 | NT | 0.0002 |
| **23** | NT | 0.0342 | NT | 0.0069 |
| **24** | NT | 0.0007 | NT | 0.0002 |
| **25** | NT | 0.0009 | NT | 0.0002 |
| **26** | NT | 0.0039 | NT | 0.0009 |
| **27** | NT | 0.0035 | NT | 0.0027 |
| **28** | NT | 0.0035 | NT | 0.0011 |
| **29** | NT | 0.0238 | NT | 0.0069 |
| **30** | NT | 0.0075 | NT | 0.0011 |
| **31** | NT | 0.0058 | NT | 0.0021 |
| **32** | 0.0361 | NT | 0.0162 | NT |
| **33** | 0.0316 | 0.0847 | 0.0216 | 0.0095 |
| **34** | 0.0401 | NT | 0.0336 | NT |
| **35** | 0.0476 | NT | 0.0491 | NT |
| **36** | 0.0490 | NT | 0.0388 | NT |
| **37** | 0.0330 | NT | 0.0582 | NT |
| **38** | 0.0421 | NT | 0.1163 | NT |
| **39** | 0.0313 | NT | 0.0411 | NT |
| **40** | 0.0263 | NT | 0.0442 | NT |
| **41** | 0.0467 | NT | 0.0457 | NT |
| **42** | 0.0440 | NT | 0.0746 | NT |
| **43** | 0.0741 | NT | 0.1475 | NT |
| **44** | NT | 0.1115 | NT | 0.0035 |
| **45** | NT | 0.1058 | NT | 0.0079 |
| **46** | NT | 0.0242 | NT | 0.0053 |
| **47** | NT | 0.0418 | NT | 0.0063 |
| **48** | NT | 0.0176 | NT | 0.0026 |
| **49** | NT | 0.0161 | NT | 0.0048 |
| **50** | NT | 0.0800 | NT | 0.0567 |
| **51** | NT | 0.0610 | NT | 0.0048 |
| **52** | NT | 0.0778 | NT | 0.0131 |
| **53** | NT | 0.0474 | NT | 0.0025 |
| **54** | NT | 0.0603 | NT | 0.0037 |
| **55** | NT | 0.2518 | NT | 0.1083 |
| **56** | NT | 0.0579 | NT | 0.0059 |
| **57** | NT | 0.2006 | NT | 0.0121 |
| **58** | NT | 0.7566 | NT | 0.1375 |
| **59** | NT | 0.1306 | NT | 0.0243 |
| **60** | NT | 0.0795 | NT | 0.0092 |
| **61** | NT | NT | NT | NT |
| **62** | NT | 0.0602 | NT | 0.0080 |
| **63** | NT | 0.0179 | NT | 0.0027 |
| **64** | NT | 0.0366 | NT | 0.0059 |
| **65** | NT | 0.0143 | NT | 0.0019 |
| **66** | NT | 0.0052 | NT | 0.0013 |
| **67** | NT | 0.0200 | NT | 0.0465 |
| **68** | NT | 0.0155 | NT | 0.0027 |
| **69** | NT | 0.0164 | NT | 0.0032 |
| **70** | NT | 0.0093 | NT | 0.0020 |
| **71** | NT | 0.0279 | NT | 0.0037 |
| **72** | NT | 0.0485 | NT | 0.0093 |
| **73** | NT | 0.0293 | NT | 0.4402 |
| **74** | NT | 0.0071 | NT | 0.0016 |
| **75** | NT | 0.0097 | NT | 0.0017 |
| **76** | NT | 0.2935 | NT | 0.0146 |
| **77** | NT | 0.2063 | NT | 0.0121 |
| **78** | NT | 0.0824 | NT | 0.0047 |
| **79** | NT | 0.0443 | NT | 0.0056 |
| **80** | NT | 0.3727 | NT | 0.0068 |
| **81** | NT | 0.0260 | NT | 0.0011 |
| **82** | NT | 1.1030 | NT | 2.0940 |
| **83** | NT | 0.0509 | NT | 0.0201 |
| **84** | NT | 0.0029 | NT | 0.0013 |
| **85** | NT | 0.0168 | NT | 0.0025 |
| **86** | NT | 0.0017 | NT | 0.0011 |
| **87** | NT | 0.0024 | NT | 0.0013 |
| **88** | NT | 0.0039 | NT | 0.0017 |
| **89** | NT | 0.0059 | NT | 0.0030 |
| **90** | NT | 0.0017 | NT | 0.0005 |
| **91** | NT | 0.0020 | NT | 0.0004 |
| **92** | NT | 0.0029 | NT | 0.0009 |
| **93** | NT | 0.0008 | NT | 0.0004 |
| **94** | NT | 0.0021 | NT | 0.0006 |
| **95** | NT | 0.0031 | NT | 0.0018 |
| **96** | NT | 0.0071 | NT | 0.0010 |
| **97** | NT | NT | NT | NT |
| **98** | NT | 0.0044 | NT | 0.0022 |
| **99** | NT | 0.0024 | NT | 0.0037 |
| **100** | NT | 0.0046 | NT | 0.0042 |
| **101** | NT | 0.0061 | NT | 0.001 |
| **102** | NT | 0.0100 | NT | 0.0350 |
| **103** | NT | 0.0672 | NT | 0.0029 |
| **104** | NT | 0.1054 | NT | 0.0087 |
| **105** | NT | 0.0497 | NT | 0.0132 |
| **106** | NT | 0.0179 | NT | 0.0017 |
| **107** | NT | 0.0261 | NT | 0.0017 |
| **108** | NT | 0.0903 | NT | 0.0078 |
| **109** | NT | 0.0443 | NT | 0.0044 |
| **110** | NT | 0.0100 | NT | 0.0015 |
| **111** | NT | 0.0341 | NT | 0.0026 |
| **112** | NT | 0.0158 | NT | 0.0148 |
| **113** | NT | 0.0135 | NT | 0.0071 |
| **114** | NT | NT | NT | NT |
| **115** | NT | 0.0181 | NT | 0.0111 |
| **116** | NT | 0.0308 | NT | 0.0201 |
| **117** | NT | 0.0094 | NT | 0.0113 |
| **118** | NT | 0.0063 | NT | 0.0018 |
| **119** | NT | 0.0028 | NT | 0.0034 |
| **120** | NT | 0.0420 | NT | 0.0080 |
| **121** | NT | 0.0116 | NT | 0.0079 |
| **122** | NT | 0.0129 | NT | 0.0113 |
| **123** | NT | 0.0078 | NT | 0.0054 |
| **124** | NT | 0.0194 | NT | 0.0136 |
| **125** | NT | 0.0037 | NT | 0.0027 |
| **126** | NT | NT | NT | NT |
| **127** | NT | 0.0075 | NT | 0.0071 |
| **128** | NT | 0.0055 | NT | 0.0033 |

## Claims

1. A compound of Formula (I) 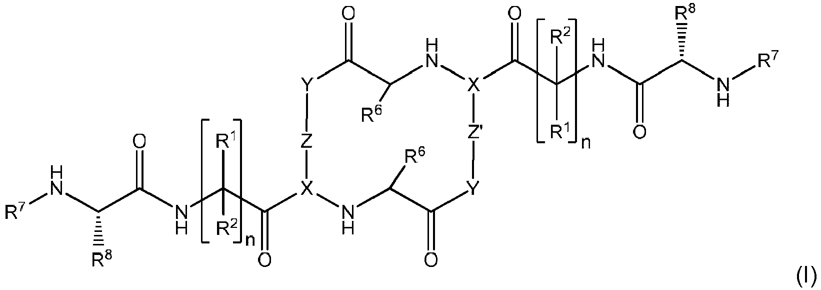 or a pharmaceutically acceptable salt thereof, wherein:
each n is independently 1 or 2;
each R¹ is independently hydrogen, optionally substituted C₁-C₄ alkyl, cycloalkyl, hydroxyalkyl, heterocyclyl or -(C₁-C₄ alkylene)-R⁴, wherein each R⁴ is independently hydrogen, -COOH, aryl, heteroaryl or cycloalkyl, and wherein at least one R¹ is other than hydrogen; and
each R² is hydrogen; or
R¹ and R² are taken together with the carbon atom to which they are commonly bound to form a cycloalkyl;
each R⁶ is independently -(C₁-C₄ alkylene)-R⁹, wherein each R⁹ is independently selected from hydrogen, aryl, heteroaryl and cycloalkyl; wherein any aryl, heteroaryl or cycloalkyl portion of R⁶ is optionally substituted with up to two substituents independently selected from halo, CF₃, OH, C₁-C₄ alkoxy, C₁-C₄ alkenyloxy, phenyl, phenyloxy, and phenylmethyloxy; and wherein one -CH₂- in the -(C₁-C₄ alkylene)- portion of R⁶ is optionally replaced with -O-;
each R⁷ is independently C₁-C₄ alkyl;
each R⁸ is independently C₁-C₄ alkyl;
each X is independently: 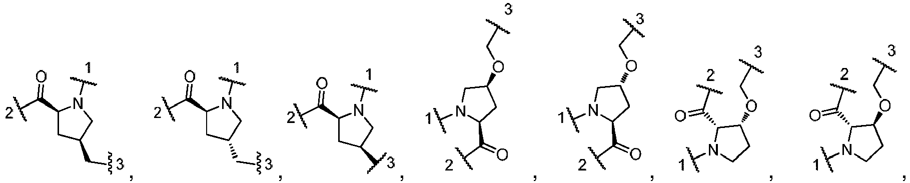 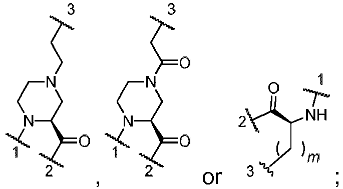
each of Z and Z' are independently: 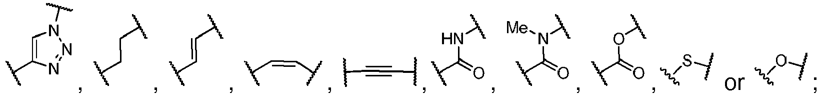 wherein each 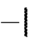 represents a point of attachment to the compound; however, Z and Z' cannot both be 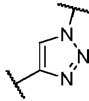 in any given compound;
each Y is independently: 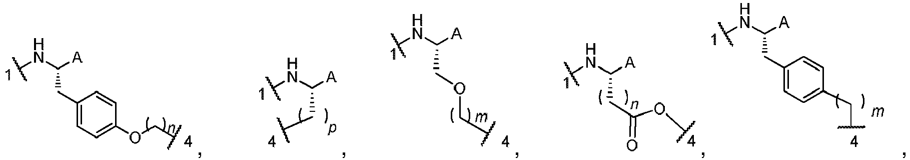 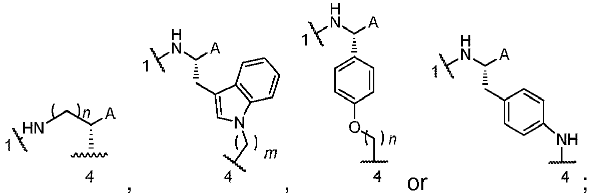 wherein: 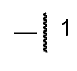 represents a point of attachment to a -C=O portion of the compound; 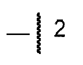 represents a point of attachment to a -NH portion of the compound; 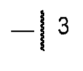 represents a first point of attachment to Z; 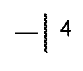 represents a second point of attachment to Z;
m = 0-3; n= 1-3, p = 0-4; and
A is -C(O)R³ or 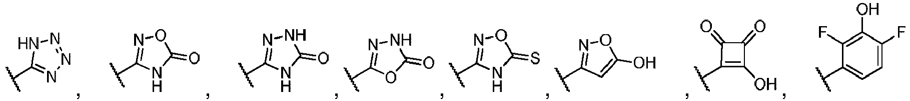
(including the various tautomeric forms);
R³ is OH, NHCN, NHSO₂R¹⁰, NHOR¹¹ or N(R¹²)(R¹³);
R¹⁰ and R¹¹ are hydrogen, optionally substituted: -C₁-C₄ alkyl, cycloalkyl, aryl, heteroaryl, heterocyclyl or heterocycloalkyl;
each of R¹² and R¹³ are independently selected from hydrogen, -C₁-C₄ alkyl, -(C₁-C₄ alkylene)-NH-(C₁-C₄ alkyl), and -(C₁-C₄ alkylene)-O-(C₁-C₄ hydroxyalkyl), or R¹² and R¹³ are taken together with the nitrogen atom to which they are commonly bound to form a saturated heterocyclyl optionally comprising one additional heteroatom selected from N, O and S, and wherein the saturated heterocycle is optionally substituted with methyl.

2. The compound according to Claim 1 wherein
each R⁶ is independently -(C₁-C₄ alkylene)-R⁹, wherein each R⁹ is independently selected from hydrogen, aryl and heteroaryl;
each R⁷ is independently selected from hydrogen and methyl;
each R⁸ is independently selected from methyl and ethyl;
each X is independently 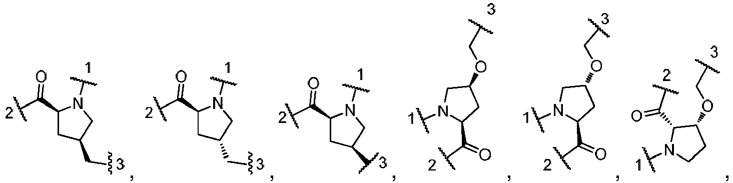 or 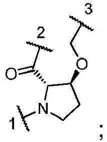 each Y is independently 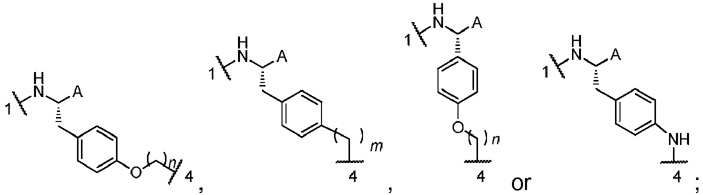 A is -C(O)R³; and
R³ is R³ is OH or NHSO₂R¹⁰.

3. The compound according to Claim 2 wherein each R¹ is independently t-butyl;
each R² is independently hydrogen;
each R⁶ is independently naphthalenylmethyl;
each R⁷ is independently methyl;
each R⁸ is independently methyl;
each X is independently 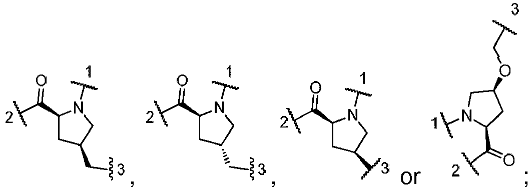 each Y is independently 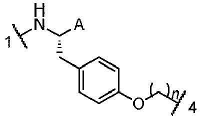 each of Z and Z' are independently 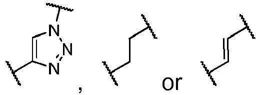 wherein each 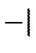 represents a point of attachment to the compound; however, Z and Z' cannot both be 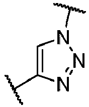 in any given compound;
A is -C(O)R³ or tetrazole;
R³ is OH or NHSO₂R¹⁰, where R¹⁰ is C₁-C₄alkyl or cycloalkyl.

4. The compound according to Claim 3 wherein R¹⁰ is methyl or cyclopropyl.

5. The compound which is 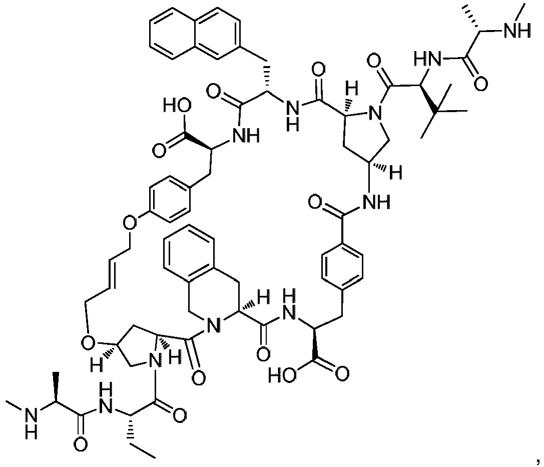 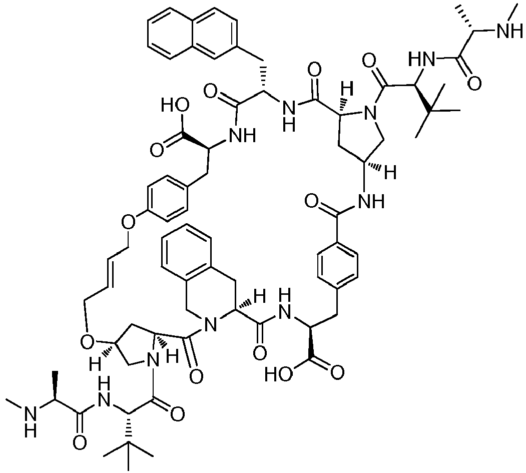 or 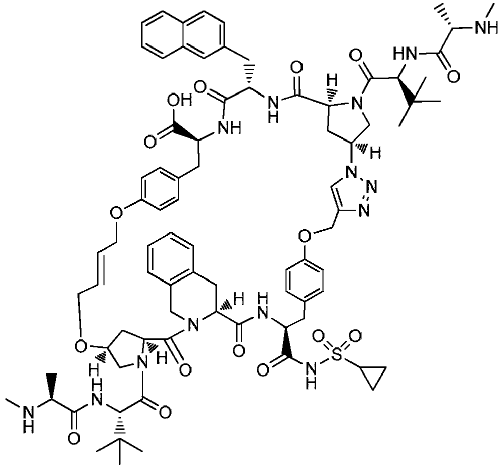 or a pharmaceutically acceptable salt thereof.

6. A pharmaceutical composition comprising a compound of any one of Claims 1 to 5 and a pharmaceutically acceptable carrier.

7. A compound according to any one of Claims 1 to 5 or a pharmaceutically acceptable salt thereof for use in medicine.

8. A compound according to any one of Claims 1 to 5 or a pharmaceutically acceptable salt thereof for use in the treatment or prevention of a proliferative disorder.

9. The compound for use according to Claim 8 wherein the proliferative disorder is cancer.

10. The compound for use according to Claim 9 wherein the treatment further comprises administering to the patient a therapeutically effective amount of a chemotherapeutic agent prior to, simultaneously with or after administration of the compound of any one of Claims 1 to 5 or a pharmaceutically acceptable salt thereof.

11. An ex *vivo* method for inducing apoptosis in a cell comprising contacting the cell with a compound or pharmaceutically acceptable salt thereof according to any one of Claims 1 to 5.

12. The method according to Claim 11 wherein the cell is a cancer cell.

## Patentansprüche

1. Verbindung mit der Formel (I) 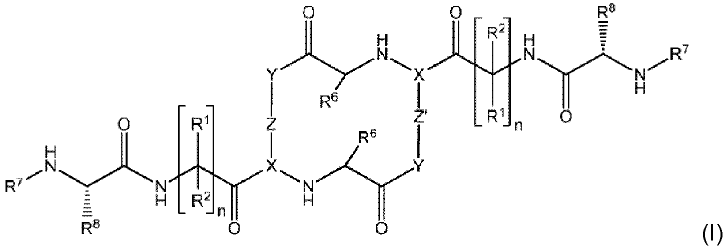 oder ein pharmazeutisch unbedenkliches Salz davon, wobei:
jedes n unabhängig 1 oder 2 ist;
jeder R¹ unabhängig Wasserstoff ist, optional substituiert mit C₁-C₄-Alkyl, Cycloalkyl, Hydroxyalkyl, Heterocyclyl oder -(C₁-C₄-Alkylen)-R⁴, wobei jeder R⁴ unabhängig Wasserstoff, -COOH, Aryl, Heteroaryl oder Cycloalkyl ist und wobei wenigstens ein R¹ von Wasserstoff verschieden ist; und
jeder R² Wasserstoff ist; oder
R¹ und R² gemeinsam mit dem Kohlenstoffatom, an das sie beide gebunden sind, ein Cycloalkyl ausbilden;
jeder R⁶ unabhängig -(C₁-C₄-Alkylen)-R⁹ ist, wobei jeder R⁹ unabhängig ausgewählt ist aus Wasserstoff, Aryl, Heteroaryl und Cycloalkyl; wobei jeder Aryl-, Heteroaryl- oder
Cycloalkylabschnitt von R⁶ optional substituiert ist mit bis zu zwei Substituenten, unabhängig ausgewählt aus Halogen, CF₃, OH, C₁-C₄-Alkoxy, C₁-C₄-Alkenyloxy, Phenyl, Phenyloxy und
Phenylmethyloxy; und wobei ein -CH₂- in dem Abschnitt -(C₁-C₄-Alkylen)- von R⁶ optional durch -O- ersetzt ist;
jeder R⁷ unabhängig C₁-C₄-Alkyl ist;
jeder R⁸ unabhängig C₁-C₄-Alkyl ist;
jedes X unabhängig Folgendes ist: 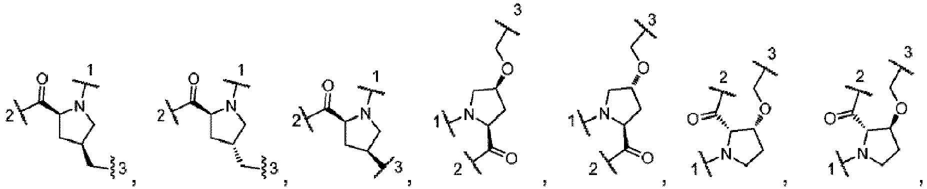 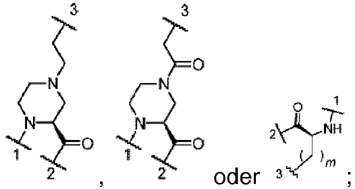
jedes aus Z und Z' unabhängig Folgendes ist: 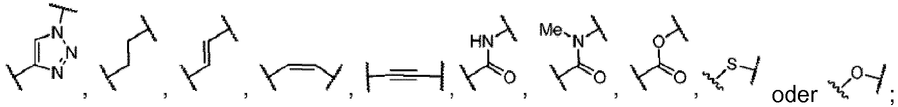 wobei jedes 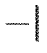 eine Bindungsstelle an der Verbindung darstellt; wobei allerdings Z und Z' in jeder beliebigen Verbindung nicht beide 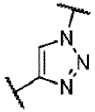 sein können; 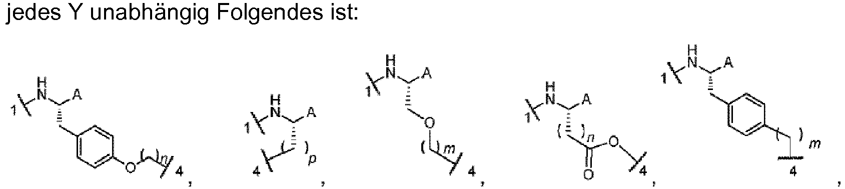 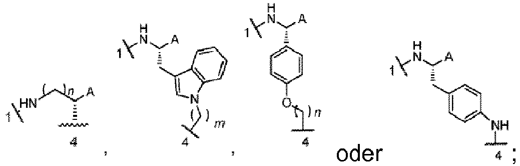 wobei: 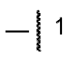 eine Bindungsstelle an einen Abschnitt -C=O der Verbindung darstellt; 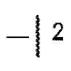 eine Bindungsstelle an einen Abschnitt -NH der Verbindung darstellt; 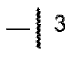 eine erste Bindungsstelle an Z darstellt; 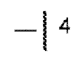 eine zweite Bindungsstelle an Z darstellt;
m = 0-3; n= 1-3, p = 0-4; und
A -C(O)R³ oder Folgendes ist: 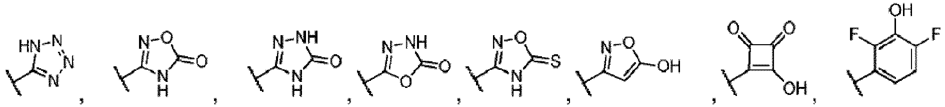
(einschließlich der verschiedenen tautomeren Formen);
R³ O^{H}, NHCN, NHSO₂R¹⁰, NHOR¹¹ oder N(R¹²)(R¹³) ist;
R¹⁰ und R¹¹ Wasserstoff sind, optional substituiert: -C₁-C₄-Alkyl, Cycloalkyl, Aryl, Heteroaryl, Heterocyclyl oder Heterocycloalkyl;
jeder aus R¹² und R¹³ unabhängig ausgewählt ist aus Wasserstoff, -C₁-C₄-Alkyl, -(C₁-C₄-Alkylen)-NH-(C₁-C₄-Alkyl) und -(C₁-C₄-Alkylen)-O-(C₁-C₄-Hydroxyalkyl) oder R¹² und R¹³ gemeinsam mit dem Stickstoffatom, an das sie beide gebunden sind, ein gesättigtes Heterocyclyl ausbilden, das optional ein zusätzliches Heteroatom umfasst, ausgewählt aus N, O und S, und wobei der gesättigte Heterocyclus optional mit Methyl substituiert ist.

2. Verbindung nach Anspruch 1, wobei
jeder R⁶ unabhängig -(C₁-C₄-Alkylen)-R⁹ ist, wobei jeder R⁹ unabhängig ausgewählt ist aus Wasserstoff, Aryl und Heteroaryl;
jeder R⁷ unabhängig ausgewählt ist aus Wasserstoff und Methyl;
jeder R⁸ unabhängig ausgewählt ist aus Methyl und Ethyl;
jedes X unabhängig Folgendes ist: 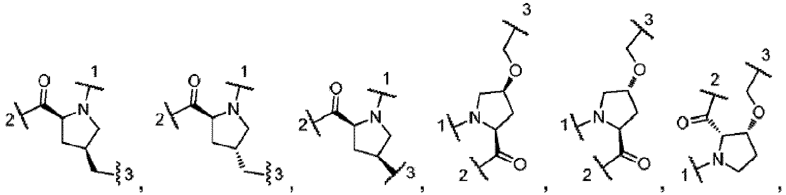 oder 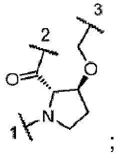 jedes Y unabhängig Folgendes ist: 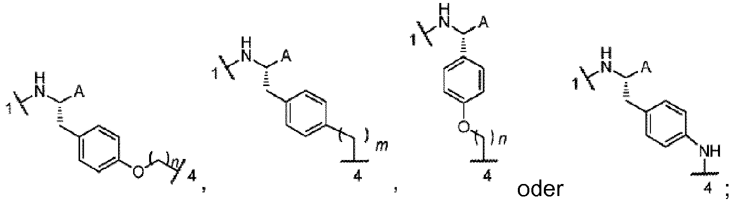 A -C(O)R³ ist; und
R³ OH oder NHSO₂R¹⁰ ist.

3. Verbindung nach Anspruch 2, wobei jeder R¹ unabhängig t-Butyl ist;
jeder R² unabhängig Wasserstoff ist;
jeder R⁶ unabhängig Naphthalenylmethyl ist; jeder R⁷ unabhängig Methyl ist;
jeder R⁸ unabhängig Methyl ist;
jedes X unabhängig Folgendes ist: 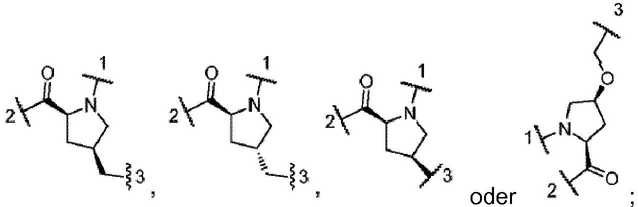 jedes Y unabhängig Folgendes ist: 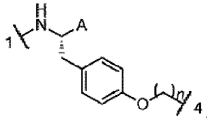 jedes aus Z und Z' unabhängig Folgendes ist: 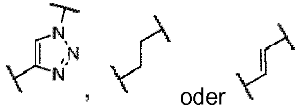 wobei jedes 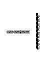 eine Bindungsstelle an der Verbindung darstellt; wobei allerdings Z und Z' in jeder beliebigen Verbindung nicht beide 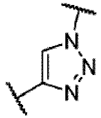 sein können;
A -C(O)R³ oder Tetrazol ist;
R³ OH oder NHSO₂R¹⁰ ist, wobei R¹⁰ C₁-C₄-Alkyl oder-Cycloalkyl ist.

4. Verbindung nach Anspruch 3, wobei R¹⁰ Methyl oder Cyclopropyl ist.

5. Verbindung, die Folgendes ist: 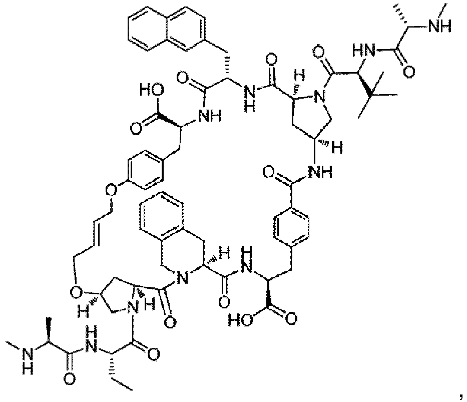 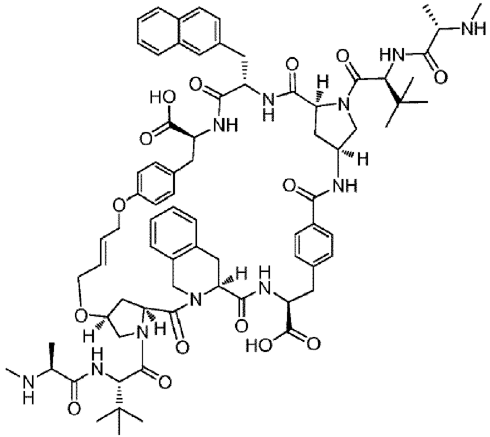 oder 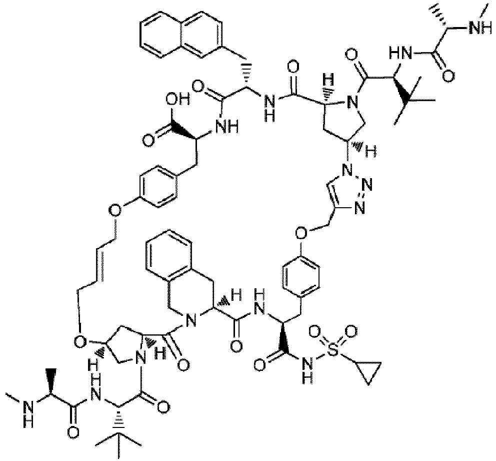 oder ein pharmazeutisch unbedenkliches Salz davon.

6. Pharmazeutische Zusammensetzung, umfassend eine Verbindung nach einem der Ansprüche 1 bis 5 und einen pharmazeutisch unbedenklichen Träger.

7. Verbindung nach einem der Ansprüche 1 bis 5 oder ein pharmazeutisch unbedenkliches Salz davon für den Gebrauch in der Medizin.

8. Verbindung nach einem der Ansprüche 1 bis 5 oder ein pharmazeutisch unbedenkliches Salz davon für den Gebrauch beim Behandeln oder Vorbeigen einer proliferativen Erkrankung.

9. Verbindung für den Gebrauch nach Anspruch 8, wobei die proliferative Erkrankung Krebs ist.

10. Verbindung für den Gebrauch nach Anspruch 9, wobei das Behandeln ferner das Verabreichen einer therapeutisch wirksamen Menge eines Chemotherapiemittels vor, gleichzeitig mit oder nach dem Verabreichen der Verbindung nach einem der Ansprüche 1 bis 5 oder eines pharmazeutisch unbedenklichen Salzes davon an den Patienten umfasst.

11. Ex-vivo-Verfahren zum Einleiten von Apoptose in einer Zelle, umfassend das Inberührungbringen der Zelle mit einer Verbindung oder einem pharmazeutisch unbedenklichen Salz davon nach einem der Ansprüche 1 bis 5.

12. Verfahren nach Anspruch 11, wobei die Zelle eine Krebszelle ist.

## Revendications

1. Composé de Formule (I) : 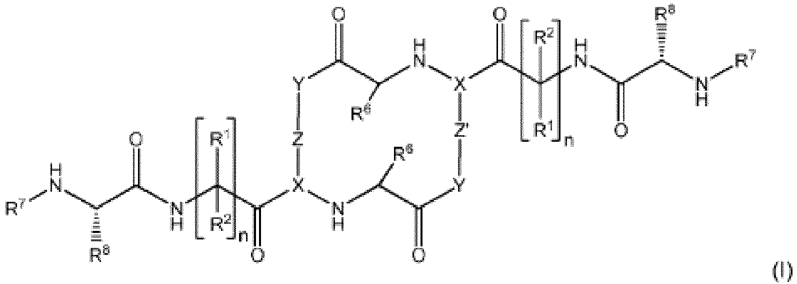 ou sel pharmaceutiquement acceptable de celui-ci, dans lequel :
chaque n est indépendamment 1 ou 2 ;
chaque R¹ est indépendamment de l'hydrogène, un alkyle en C₁-C₄ facultativement substitué, un cycloalkyle, un hydroxyalkyle, un hétérocyclyle ou un -(alkylène en C₁-C₄)-R⁴,
où chaque R⁴ est indépendamment de l'hydrogène, -COOH, un aryle, un hétéroaryle ou un cycloalkyle, et où au moins un R¹ est autre que de l'hydrogène ; et
chaque R² est de l'hydrogène ; ou
R¹ et R² sont pris ensemble avec l'atome de carbone auquel ils sont généralement liés pour former un cycloalkyle ;
chaque R⁶ est indépendamment un -(alkylène en C₁-C₄)-R⁹, où chaque R⁹ est indépendamment choisi parmi l'hydrogène, l'aryle, l'hétéroaryle ou le cycloalkyle ; où un quelconque fragment aryle, hétéroaryle ou cycloalkyle de R⁶ est facultativement substitué par jusqu'à deux substituants indépendamment choisis parmi l'halo, CF₃, OH, l'alcoxy en C₁-C₄, l'alcényloxy en C₁-C₄, le phényle, le phényloxy et le phénylméthyloxy ; et où un -CH₂-dans le fragment -(alkylène en C₁-C₄)- de R⁶ est facultativement remplacé par -O- ;
chaque R⁷ est indépendamment un alkyle en C₁-C₄ ;
chaque R⁸ est indépendamment un alkyle en C₁-C₄ ;
chaque X est indépendamment : 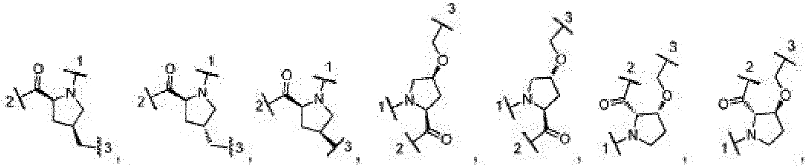 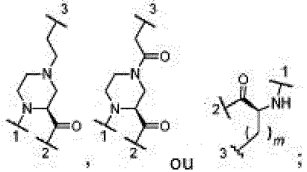
chacun de Z et Z' est indépendamment : 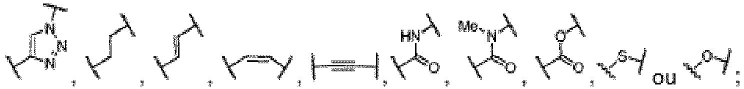 où chaque 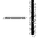 représente un point de fixation au composé ; cependant, Z et Z' ne peuvent pas être tous deux 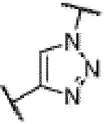 dans un quelconque composé donné ; chaque Y est indépendamment : 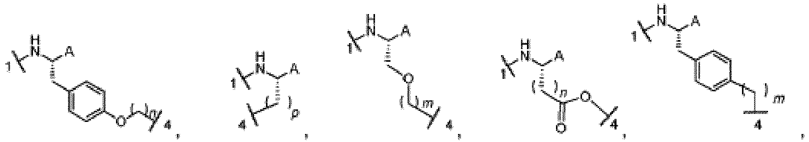 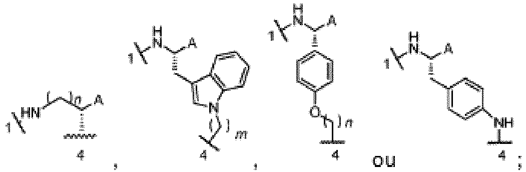 où : 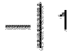 représente un point de fixation à un fragment -C=O du composé ; 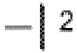 représente un point de fixation à un fragment -NH du composé ; 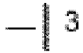 représente un premier point de fixation à Z ; 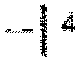 représente un deuxième point de fixation à Z ;
m = 0 à 3 ; n = 1 à 3, p = 0 à 4 ; et
A est -C(O)R³ ou 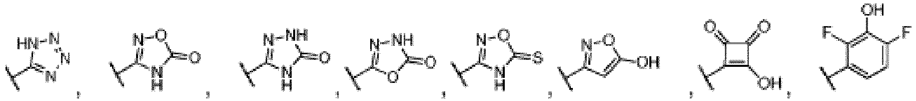
(y compris les différentes formes tautomères) ;
R³ est OH, NHCN, NHSO₂R¹⁰, NHOR¹¹ ou N(R¹²)(R¹³) ;
R¹⁰ et R¹¹ sont de l'hydrogène, un alkyle en C₁-C₄, un cycloalkyle, un aryle, un hétéroaryle, un hétérocyclyle ou un hétérocycloalkyle facultativement substitué ;
chacun de R¹² et R¹³ est indépendamment choisi parmi l'hydrogène, l'alkyle en C₁-C₄, le - (alkylène en C₁-C₄)-NH-(alkyle en C₁-C₄) et le -(alkylène en C₁-C₄)-O-(hydroxyalkyle en C₁-C₄), ou R¹² et R¹³ sont pris ensemble avec l'atome d'azote auquel ils sont généralement liés pour former un hétérocyclyle saturé comprenant facultativement un hétéroatome supplémentaire choisi parmi N, O et S, et où l'hétérocycle saturé est facultativement substitué par du méthyle.

2. Composé selon la revendication 1, dans lequel
chaque R⁶ est indépendamment un -(alkylène en C₁-C₄)-R⁹, où chaque R⁹ est indépendamment choisi parmi l'hydrogène, l'aryle et l'hétéroaryle ;
chaque R⁷ est indépendamment choisi parmi l'hydrogène et le méthyle ;
chaque R⁸ est indépendamment choisi parmi le méthyle et l'éthyle ;
chaque X est indépendamment 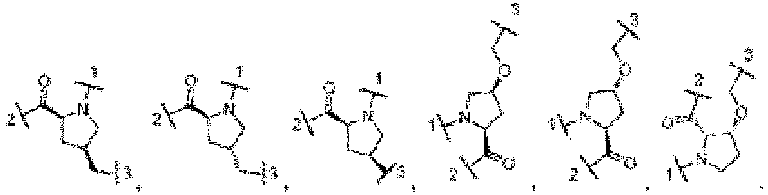 ou 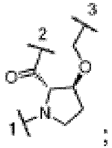 chaque Y est indépendamment 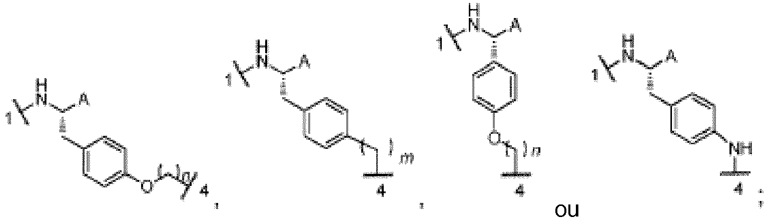 A est -C(O)R³ ; et
R³ est OH ou NHSO₂R¹⁰.

3. Composé selon la revendication 2, dans lequel chaque R¹ est indépendamment du t-butyle ;
chaque R² est indépendamment de l'hydrogène ; chaque R⁶ est indépendamment du napthalénylméthyle ; chaque R⁷ est indépendamment du méthyle ;
chaque R⁸ est indépendamment du méthyle ;
chaque X est indépendamment 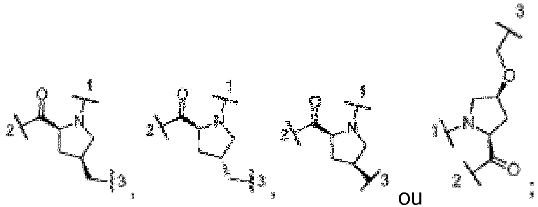 chaque Y est indépendamment 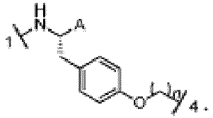 chacun de Z et Z' est indépendamment 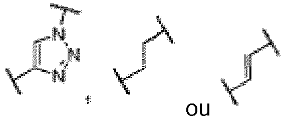 où chaque 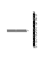 représente un point de fixation au composé ; cependant, Z et Z' ne peuvent pas être tous deux 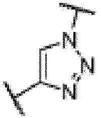 dans un quelconque composé donné ;
A est -C(O)R³ ou du tétrazole ;
R³ est OH ou NHSO₂R¹⁰, où R¹⁰ est un alkyle en C₁-C₄ ou un cycloalkyle.

4. Composé selon la revendication 3, dans lequel R¹⁰ est du méthyle ou du cyclopropyle.

5. Composé qui est 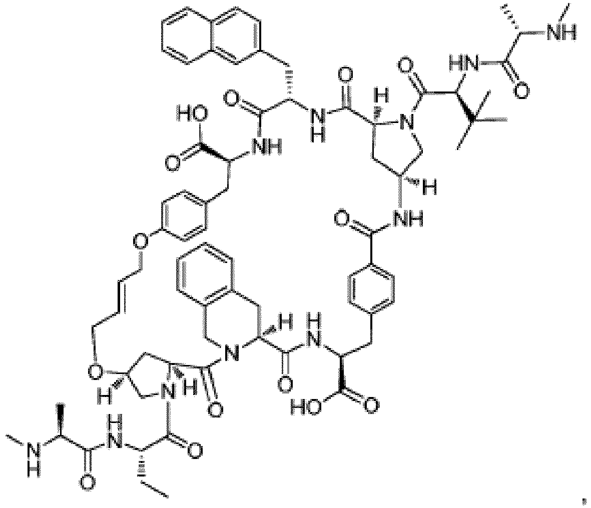 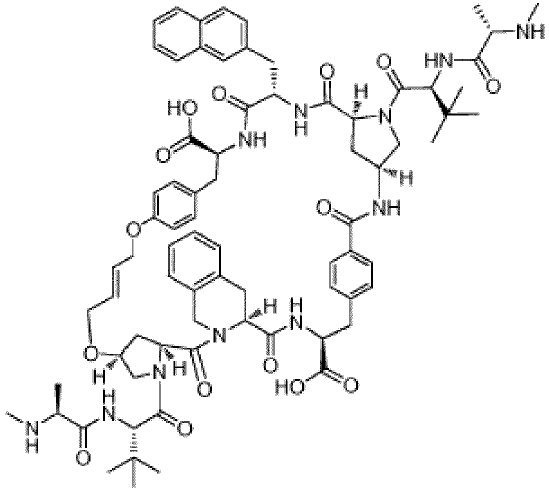 ou 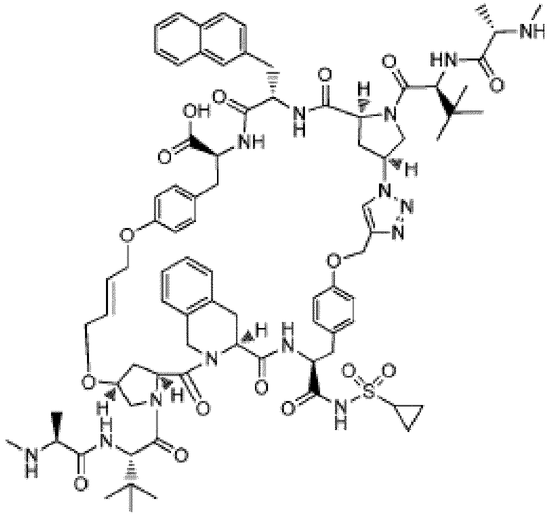 ou sel pharmaceutiquement acceptable de celui-ci.

6. Composition pharmaceutique comprenant un composé selon l'une quelconque des revendications 1 à 5 et un vecteur pharmaceutiquement acceptable.

7. Composé selon l'une quelconque des revendications 1 à 5 ou sel pharmaceutiquement acceptable de celui-ci pour une utilisation en médecine.

8. Composé selon l'une quelconque des revendications 1 à 5 ou sel pharmaceutiquement acceptable de celui-ci pour une utilisation dans le traitement ou la prévention d'un trouble prolifératif.

9. Composé pour une utilisation selon la revendication 8, dans lequel le trouble prolifératif est le cancer.

10. Composé pour une utilisation selon la revendication 9, dans lequel le traitement comprend en outre l'administration au patient d'une quantité thérapeutiquement efficace d'un agent chimiothérapeutique avant, pendant ou après l'administration du composé selon l'une quelconque des revendications 1 à 5 ou d'un sel pharmaceutiquement acceptable de celui-ci.

11. Procédé *ex vivo* d'induction de l'apoptose dans une cellule comprenant la mise en contact de la cellule avec un composé ou un sel pharmaceutiquement acceptable de celui-ci selon l'une quelconque des revendications 1 à 5.

12. Procédé selon la revendication 11, dans lequel la cellule est une cellule cancéreuse.
